# EUROPEAN PATENT SPECIFICATION

(11) **EP 2 563 337 B1**
(45) Date of publication and mention of the grant of the patent: **26.07.2017**
(21) Application number: 11719768.1
(22) Date of filing: 21.04.2011
(51) Int. Cl.: A61K 9/127, A61K 31/704, A61K 9/00

(54) **CARRIER AND TARGETING SYSTEM COMPRISING A SIOSOMAL COMPOSITION FOR INTRACELLULAR DELIVERY AND TARGETING OF ACTIVE SUBSTANCE**
Siosomale Formulierungen für intrazelluläre Abgabe und Targeting therapeutischer Wirkstoffe
Formulation siosomal pour l'administration intracellulaire et le ciblage des agents thérapeutiques

(30) Priority: 27.04.2010 US 328403 P; 27.04.2010 EP 10075170
(43) Date of publication of application: 06.03.2013
(73) Proprietor: Salama, Zoser B., 88214 Ravensburg (DE)
(72) Inventor: Salama, Zoser B., 88214 Ravensburg (DE)
(74) Representative: Hertin und Partner Rechts- und Patentanwälte PartG mbB
(86) International application number: PCT/EP2011/002166
(87) International publication number: WO 2011/134675

(56) References cited:
- EP-A1- 0 483 465
- WO-A1-2007/121947
- WO-A2-2008/078102
- WO-A2-2010/018003
- DE-A1-102005 053 011
- KUNATH U ET AL: "DI(ACYLOXY)DIALKYL- UND DI(ACYLOXY)DIPHENYLSILANE - NEU-ARTIGE VESIKELBILDNER. \TEIL 43: EINSCHLUSS VON INSULIN IN SIOSOMEN", DIE PHARMAZIE, GOVI VERLAG PHARMAZEUTISCHER VERLAG GMBH, ESCHBORN, DE, vol. 47, no. 9, 1 September 1992 (1992-09-01), pages 713-714, XP000307030, ISSN: 0031-7144
- RICHTER H ET AL: "DI(ACYLOXY)DIALKYL- UND DI(ACYLOXY)DIPHENYLSILANE - NEUARTIGE VESIKELBILDNER. \TEIL 31: EINSCHLUSS VON DITHRANOL IN SIOSOMEN AUS DI(ACYLOXY)-DIALKYL- UND DI(ACYLOXY)DIPHENYLSILANEN", DIE PHARMAZIE, GOVI VERLAG PHARMAZEUTISCHER VERLAG GMBH, ESCHBORN, DE, vol. 47, no. 6, 1 June 1992 (1992-06-01), pages 464-465, XP000353916, ISSN: 0031-7144

## Description

### Field of Invention

The invention relates to a carrier and targeting system comprising a siosomal composition for the intracellular delivery and release of a siosome- and/or siosome-liposome-entrapped or encapsulated active substance or therapeutic agent. The invention further relates to the targeted release and delivery of doxorubicin, cis-platin, carbo-platin and/or oxali-platin, docetaxel and paclitaxel using said carrier and targeting system. The invention relates to the use of cationic lipids, which are covalently linked to the silicone central atom of the silanes, sugar silanes and/or amino-sugar silanes, or to the substituents thereof. The invention is further related to the use of PEGylated cationic organosilicon compounds, which are covalently linked to the silicon central atom of the Silanes, sugar Silanes and/or amino sugar Silanes, or to the substituents thereof. The invention also relates to a method for preparing such carrier and targeting systems and methods for their administration, particularly in the treatment of cancer.

### Background of the invention

It is well recognised in the medical field that the most effective procedures for treating localised diseases relates to directing a pharmaceutical agent or active substance to the affected area, thereby avoiding undesirable toxic effects of systemic treatment. Techniques and methods currently used to deliver active substances to specific target sites within the body involve the utilisation of time-release delivery systems.

Typically, the encapsulation of an active substance in a vesicle can alter the pattern of biodistribution and the pharmacokinitecs of the active substance. In certain cases such as liposomal encapsulation, it has been found that toxicity due to side effects of the active substance can be lowered. In particular, the so called "long circulating liposomal formulations", which avoid uptake by the organs of the mononuclear phagocyte system, primarily in the liver and spleen, have been extensively studied.

Such long circulating liposomes may include a surface coat of flexible water soluble chains that act to prevent interaction between the liposome and plasma components which play a role in liposome uptake. Otherwise, such liposomes can be produced without this coating, whereby saturated long chain phospholipid and cholesterol compounds are used instead.

Doxorubicin (DXR) is a potent chemoactive substance effective against a broad spectrum of neoplasms. However, clinical use of the drug in free form or as doxorubicin hydrochloride liposomal, Doxil ® (formulated in Stealth ® liposomes composed of N-(carbomoyl-methoxypolyethylene glycol 2000)-1,2-distearoyl-sn-glycero-3-phosphoethanolamine sodium [MPEG-DSPE], hydrogenated soy phosphatidyl-choline [HSPC], and cholesterol) is limited by serious side effects and acute toxicity including malaise, nausea, vomiting, myelosuppression, and severe alopecia. In addition, cumulative and irreversible cardiac damage occurs after repeated administration, which seriously limits the use of the drug, although the DXR in liposomes achieved increased therapeutic action and reduced toxicity.

The degradation of the lipid and active substances which can occur on storage will have an important impact on the efficacy of the formulation. Phospholipid degradation in liposomes for example can take the form of hydrolytic release of fatty acyl chain groups and lipid peroxidative damage, particularly at unsaturated bound regions and lipid acyl chain moieties. In addition, drugs such as DXR are themselves capable of initiating oxidative reactions, and in the presence of lipids, appear to contribute to free radical/oxidative reactions, and also undergo rapid chemical changes on storage in liposomes.

The lipid concentration in the DXR/liposomes intended for intravenous administration in cancer chemotherapy is very high, of at least 100 mM liposome lipid in addition to other additives as cryoprotectants (1-10%).

Despite some recent success using liposomes as delivery agents for active substances, whereby the toxicity due to side effects of the active substance can be lowered in some cases, the lipids and liposomes themselves also exhibit significant toxicity, so that the end effect of the administered agent often remains toxic.

The exact mechanism of action of doxorubicin is complex and still somewhat unclear, although it is known to interact with DNA by intercalation and inhibition of macromolecular biosynthesis. This inhibits the progression of the enzyme topoisomerase II, which unwinds the DNA for transcription.

Doxorubicin stabilises the topoisomerase II complex after it has broken the DNA chain for replication, preventing the DNA double helix from being resealed and thereby stopping the process of replication. The planar aromatic chromophone position of the molecule intercalates between two base pairs of the DNA, while the six-membered sugar sits in the minor groove and interacts with flanking base pairs immediately adjacent to the intercalation site, as evidenced by several crystal structure studies. Doxorubicin cardiotoxicity is characterized by a dose-dependent decline in mitochondrial oxidative phosphorylation. Reactive oxygen species, which are generated by the interaction of doxorubicin with iron, can then damage the myocytes (heart cells), causing myofibrillar loss and cytoplasmic vacuolisation.

The drug is administered intravenously in the form of hydrochloride salt, as a lysosomal doxorubicin for injection and PEGylated lysosomal doxorubicin (Doxil®). All formulations of doxorubicin hydrochloride have shown serious adverse effects. A summary of the adverse effects is provided below:

### 1. Hematologic effects

- Leukopenia (principally granulocytopenia) is the predominant manifestation of hematologic toxicity, the severity of which depends on the dose of the drug and on the regenerative capacity of the bone marrow.
- The principal dose-limiting toxicity of PEG-stabilised liposomal doxorubicin in patients with AIDS-related Kaposi's sarcoma has been myelosuppression, commonly manifested by leukopenia and neutropenia; anaemia and thrombocytopenia also occur frequently.

### 2. Cardiac effects

These include congestive heart failure, dilated cardiomyopathy, and death. Doxorubicin cardiotoxicity is characterized by a dose-dependent decline in mitochondrial oxidative phosphorylation. Reactive oxygen species, generated by the interaction of doxorubicin with iron, can then damage the myocytes (heart cells), causing myofibrillar loss and cytoplasmic vacuolisation.

### 3. Gastro-intestinal effects (GI effects)

Stomatitis and esophagitis (mucositis) may occur in patients receiving doxorubicin. GI toxicity (evidenced frequently by nausea and vomiting and occasionally by anorexia and diarrhoea) may occur, usually on the day of drug administration.

### 4. Dermatologic effects

Complete alopecia almost always accompanies doxorubicin therapy.

### 5. Nervous system effects

### Peripheral neurotoxicity.

Small interfering RNA (siRNA), also called silencing RNA or short interfering RNA, was first implicated in post-transcriptional gene silencing (PTGS) in plants (Hamilton et al., 1999).

The siRNA which contains 21-25 base pairs, plays an important role in silencing an RNA pathway. It describes the specific knock-down of target gene (target sequences) of interest by an artificial mechanism. The siRNA delivery (or transfection) reagent complexes are often specifically referred to as lipoplexes, dedriplexes and polyplexes, depending on whether the vector used is a cationic lipid, dendrimer, or polymer, respectively. Alternatively, these cationic lipids and polymers can form nanoparticles, in which the nucleic acid is entrapped within the body of the particle and not simply adsorbed onto the surface, usually via ionic interaction, as is the case with complexes. However, in the literature, the term nanoparticle is often interchangeably used for complexes, possibly to better reflect the nomenclature being used within the ongoing nanotechnology/nanomedicine field. Although the exact mechanism of delivery varies, these complexes and nanoparticles can, to varying degrees, provide siRNA stability within blood serum, avoid sequestration within the RES, and eventually reach target cells.

They are taken into the cell by some form of vesicular transport (endocytosis and/or macropinocytosis). Complexes and nanoparticles generally have to be less than 100 nm to avoid renal excretion and be taken up by the target cells.

The encapsulation, entrapment efficiency and the intracellular delivery and targeting of siRNA, DNA, RNA and other nucleosides are very low and limited for a variety of the cited reasons, such as that vesicles, after systemic administration to the blood stream, are rapidly removed from circulation by the reticulo-endothelial system. Another reason is the inherent difficulty in delivering a molecule, in particular a large or a charged molecule, into the cellular cytoplasm and/or the nucleus.

It has been difficult to deliver the nucleic acid to the required areas of the body safely and in sufficient quantities, especially in a form stable enough for either expression of the protein for which it codes or silencing of its target, depending on the intended function of the transfected nucleic acid. Viruses have been used as delivery vectors but have fallen out of favour somewhat because of toxicity issues, and attention has turned to non-viral alternatives. Positively charged (cationic) synthetic molecules will readily bind to negatively charged DNA molecules and have been used for DNA delivery, but these cationic molecules are often toxic to cells. WO2010/018003 describes a carrier system for biological agents containing organosilicon compounds covalently attached to a non-charged lipids. In view of the foregoing, there exists a need in the art for a method for targeted intracellular drug delivery that overcomes the disadvantages of the currently available methods. Specifically, a delivery systems is required that is stable in the circulation, following intravenous administration, allowing retention of encapsulated or associated drug or active substances.

### SUMMARY OF THE INVENTION

The technical problem underlying the present invention is to provide a novel carrier and targeting system for the intracellular delivery and release of a siosome and/or siosome-liposome entrapped agent/active substance that overcomes the disadvantages of the prior art.

This problem is solved by the features of the independent claims. Preferred embodiments of the present invention are provided by the dependent claims.

The object of the invention is therefore to provide a carrier and targeting system for the intracellular delivery and release of a siosome- and/or siosome-liposome-entrapped active substance, comprising a composition of active substance, organosilicon, sugar organosilicon, amino-sugar organosilicon, alkyl, alkoxy or acyloxy organosilicon, peptide organosilicon, amino acid(s) organosilicon, lipids and/or their derivatives.

The invention therefore relates to Carrier and targeting system for the intracellular delivery and release of a siosome- and/or siosome-liposome-entrapped active substance, comprising a composition, wherein said composition comprises:
- organosilicon, sugar organosilicon, amino-sugar organosilicon, alkyl, alkoxy or acyloxy organosilicon, peptide organosilicon, nucleotide organosilicon and/or amino acid(s) organosilicon,
- active substance, and
- lipids,
whereby
the organosilicon, sugar organosilicon, or amino-sugar organosilicon compounds are characterized by the general formula (I): 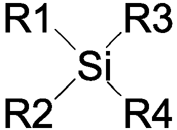 whereby R₁ and R₂ can be the same or different, and where R₃ and R₄ can be the same or different, whereby
R₁, R₂ = Monosaccharide, disaccharide, oligosaccharide, nucleotide, alkyl or aryl, aromatic or aliphatic heterocycles, and
R₃, R₄ = Polynucleotide, peptide, aliphatic chains, proteins, siRNA, RNA, DNA, ligand, vector, fatty acid, acyl group,
characterized in that
one or more cationic lipids are covalently attached to the silicon and/or to chemical groups and/or residues of R₁, R₂, R₃ and R₄ of compounds according to the general formula (I).

In a further preferred embodiment the carrier and targeting system of the present invention is characterized in that one or more cationic lipids are covalently attached to the silicon and/or to the chemical groups and/or residues of R₁, R₂, R₃ and R₄ of the general formula (I), whereby
- R₁ =: Monosaccharide, di-saccharide, oligosaccharide, derivatives;
- R₂ =: Monosaccharide, di-saccharide, oligosaccharide, amino acid, peptide, nucleoside, nucleotides, alkyl, aryl residues, aromatic or aliphatic heterocycles;
- R₃, R₄ =: 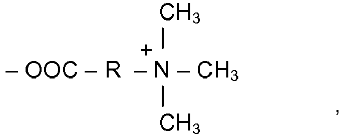 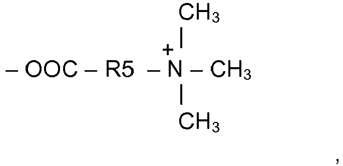 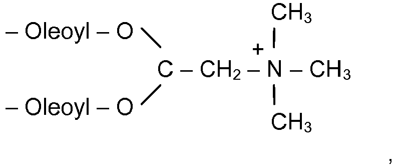
or a peptide residue of the formula: 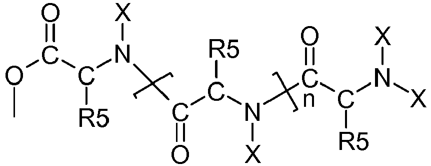 wherein R represents an unbranched or branched alkyl, alkenyl or alkinyl residue with 5 to 29 C atoms which can be substituted by one to three halogen atoms, alkoxy residues with 1 to 18 C atoms or amino groups, the residues R5, which can be the same or different, represent the residue remaining after the removal of the group 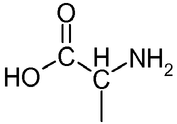 from an amino acid, the residues X, which may be the same or different, represent a hydrogen atom or an amino-protective group usually occurring in peptide chemistry and n represents an integer from 1 to 12,or wherein R₃ and R₄ which can be the same or different, each represent an aryl group, such as a phenyl group, or the residue remaining after the removal of a hydrogen atom from a monosaccharide, disaccharide, amino sugar or a hydroxyl carbon acid, or alkoxy residues with 1-5 C atoms, or acyl residues of an amino acid with a free or protected amino group, or a dipeptide, tripeptide or tetrapeptide residue of an amino acid with free or protected amino groups, or they have the meaning R₁ and R₂.

In a further preferred embodiment the carrier and targeting system of the present invention is characterized in that the encapsulated and/or entrapped active substance is an anti-cancer agent, such as doxorubicin, cis-platin, carbo-platin, dacetaxel, Paclitaxel and/or oxali-platin.

In a further preferred embodiment the carrier and targeting system of the present invention is characterized in that the encapsulated and/or entrapped active substance is genetic material, preferably a nucleic acid such as DNA, RNA, siRNA and/or an anti-viral and/or anti-bacterial agent.

In a further preferred embodiment the carrier and targeting system of the present invention is characterized in that polyethylene-glycol is covalently attached to the silicone and/or one or more of the groups R₁, R₂, R₃ and R₄ of the general formula (I).

In a further preferred embodiment the carrier and targeting system of the present invention is characterized in that the covalent attachment of polyethylene-glycol, polymers, polymer derivatives can be carried out by means of a cleavable spacer linker.

In a further preferred embodiment the carrier and targeting system of the present invention is characterized in that the composition is lyophilized.

In a further preferred embodiment the carrier and targeting system of the present invention is characterized in that the composition comprises a cryoprotectant.

In a further preferred embodiment the carrier and targeting system of the present invention is characterized in that the cryoprotectant is a disaccharide selected from the group consisting of sucrose, maltose, trehalose, and/or lactose.

In a further preferred embodiment the carrier and targeting system of the present invention is characterized in that the cryoprotectant is a disaccharide having a concentration of 1-40%.

In a further preferred embodiment the carrier and targeting system of the present invention comprises additionally of liposomes, unsaturated lipids, a vesicle-forming lipid, a hydrophilic polymer and/or liposomes.

In a further preferred embodiment the carrier and targeting system of the present invention is characterized in that the vesicle-forming lipids exhibit two hydrocarbon chains, typically acyl chains, and a polar head group.

In a further preferred embodiment the carrier and targeting system of the present invention is characterized in that the phospholipids are selected from the group comprising phosphatidylcholine (PC), phosphatidylethanolamine (PE), phosphatidic acid (PA), Phosphatidyl-inositol (PI), and sphingomyelin (SM).

In a further preferred embodiment the carrier and targeting system of the present invention is characterized in that the two hydrocarbon chains are between 8-26 carbon atoms in length.

In a further preferred embodiment the carrier and targeting system of the present invention, it is intended that the carrier system comprises additionally of glycolipids such as cerebiosides and gangliosides.

In a further preferred embodiment the carrier and targeting system of the present invention is characterized in that the carrier and targeting system is obtainable by:
a) mixing one or more organosilicon, sugar organosilicon, amino-sugar organosilicon, alkyl, alkoxy or acyloxy organosilicon, peptide organosilicon, amino acid(s) organosilicon, lipids and/or their derivatives, salts and/or the vesicles formed from them, with one or more polymers, such as PEG, one or more salts, such as Zn²⁺, Ca²⁺, Mg²⁺, Na⁺, buffers, with one or more active substances, such as doxorubicin, cis-oxoplatin, oxali-platin, nucleic acids, such as DNA, RNA, siRNA, nucleosides at selected pH, salt concentration and temperature
   characterized in that one or more charged cationic lipids are covalently attached to the silicon and/or to chemical groups and/or residues of R1, R2, R3 and R4 of compounds according to the general formula (I);
b) homogenisation, sonication and/or extrusion of the mixture,
c) with or without separation of the free active substance,
d) with or without sterile filtration of the mixture,
e) with our without lyophilisation,
f) with or without reconstitution to form a siosome and/or a combined siosome-liposome complex concentrate.

A further aspect of the invention is a method for preparing the carrier and targeting system of the present invention, characterized by
a) mixing one or more organosilicon, sugar organosilicon, amino-sugar organosilicon, alkyl, alkoxy or acyloxy organosilicon, peptide organosilicon, amino acid(s) organosilicon, lipids and/or their derivatives, salts and/or the vesicles formed from them, with one or more polymers, such as PEG, one or more salts, such as Zn²⁺, Ca²⁺, Mg²⁺, Na⁺, buffers, with one or more active substances, such as doxorubicin, cis-oxoplatin, oxali-platin, nucleic acids, such as DNA, RNA, siRNA, nucleosides at selected pH, salt concentration and temperature
   characterized in that one or more charged cationic lipids are covalently attached to the silicon and/or to chemical groups and/or residues of R1, R2, R3 and R4 of compounds according to the general formula (I);
b) homogenisation, sonication and/or extrusion of the mixture, followed by
c) separation of the free active substance,
d) sterile filtration of the mixture,
e) lyophilisation,
f) reconstitution to form a siosome and/or a combined siosome-liposome complex concentrate.

In a further preferred embodiment of the present invention the method for producing the carrier and targeting system is characterized in that the active substance, preferably nucleic acids, DNA, RNA and/or siRNA, can be loaded with cations prior to preparation of the siosome carrier systems.

Also disclosed is a method for preparing the carrier and targeting system, wherein the cationic silanes and/or siosomes can be loaded with anionic salts.

Also disclosed is a method for preparing the carrier and targeting system, wherein the anionic silanes and/or siosomes can be loaded with cationic salts.

In a further preferred embodiment of the present invention the method for preparing the carrier and targeting system is characterized in that the mixing is carried out in a buffer solution that comprises TRIS, HEPES; MOPS, MES and/or other commonly used biological buffers.

In a further preferred embodiment of the present invention the method for preparing the carrier and targeting system is characterized in that the method includes one or more additives selected from the group comprising of detergent solution, adjuvant solution, salt solution, PEG-conjugates and/or cryoprotectants.

In a further preferred embodiment of the present invention the method for preparing the carrier and targeting system is characterized in that the silanes, sugar silanes, amino-sugar Silanes, lipids and/or their derivatives and/or the active substances such as doxorubicin, is characterized in that the method includes one or more solubilizer and the use of all techniques of solubility enhancement such as physical and/or chemical modifications.

A further aspect of the present invention relates to a method for administering the carrier and targeting system, siosome- and/or siosome-liposome-complex via oral, rectal, vaginal, topical, nasal, intradermal, or parenteral administration.

In a preferred embodiment the method for administering the carrier and targeting system is characterized in that the parenteral administration method is selected from the group comprising subcutaneous, intravenous, intramuscular and infusion.

A further aspect of the present invention relates to a method for treating cancer, such as solid and haematological malignancies, in subjects, comprising the administration of the carrier and targeting system of the present invention.

A further aspect of the present invention relates to a method for increasing intracellular concentration of a siosome-entrapped active substance, preferably doxorubicin, cis-platin and/or siRNA, characterized by administering the carrier and targeting system of the present invention to a patient or a target cell.

In a preferred embodiment the method for increasing intracellular concentration of a siosome-entrapped active substance is characterized in that the intracellular concentration of the active substance is increased by specific binding of a ligand to a target cell and/or internalisation of the siosome- and/or siosome-liposome-complex by the target cell. In a further preferred embodiment the method is characterized in that the intracellular cytotoxicity of a tumor cell or other target cell is increased by administration of the carrier and targeting system according to the present invention. The present invention therefore provides siosomal compositions, carrier systems and methods using systems for intracellular delivery and triggered release by siosome-entrapped agents or active substances from the enzyme-rich acidic environment of the lysosomes (pH-sensitive, triggered release siosomes).

A further aspect of the present invention is the use of the carrier and targeting system as described herein as a medicament.

A further aspect of the present invention is the use of the carrier and targeting system as described herein as a medicament for the treatment of cancer.

A further aspect of the present invention relates to a pharmaceutical agent comprising the carrier and targeting system as described herein and a pharmaceutically acceptable carrier and/or pharmaceutically acceptable salt.

A further aspect of the invention also relates to a method of transfecting a cell with an anti-cancer active substance or therapeutic agent such as doxorubicin, cis-platin, carbo-platin, oxali-platin, bleomycin, a nucleic acid, such as DNA, RNA, siRNA, an anti-viral active substance and/or an anti-bacterial agent, using as the transfection vector a carrier and targeting system according to the present invention comprising at least one sugar organosilicon compound and/or derivatives thereof as described herein, preferably in addition to PEG and/or a cryoprotectant.

### DETAILED DESCRIPTION OF THE INVENTION

This delivery system would be capable of accumulating at a target organ, tissue or cell via active targeting (for example by incorporating an antibody or saccharide, nucleoside, peptide, hormone, ligand on the surface of the siosomal vesicle).

Following accumulation at the target site, the siosomal carrier would become capable of fusing without the need for any external stimulus, and would subsequently release any encapsulated or associated drug or active substance in the vicinity of the target cell, or fuse with the target cell plasma membrane, introducing the drug or active substance into the cell cytoplasm. In certain instances, fusion of the carrier with the plasma membrane would be preferred because this would provide more specific drug delivery and hence minimise any adverse effects on normal healthy cells or tissues.

In addition, in the case of active substances such as DNA, RNA, siRNA, proteins, peptides etc., which are generally not permeable to the cell membrane, such a fusogenic carrier would provide a mechanism whereby the active substance could be delivered to its required intracellular site of action. Further, by avoiding the endocytic pathway, the active substance would not be exposed to acidic conditions and/or degradative enzymes that could be destructive to the said active substance. Quite surprisingly, the present invention addresses this need by providing such a method.

One aspect of the present invention is the triggered release of the encapsulated or bound active substances upon entry to a cell. The intracellular delivery can be released in a triggered manner, whereby the enzyme-rich acidic environment of the lysosomes leads to opening of the siosome and release of the active substance. pH-sensitive lipids are intended for this purpose. Once administered, the carrier system is taken up by the cell by either endocytosis, phagocytosis or pinocytosis. This may occur via sugar interactions with the cell surface to enter the cell via receptor mediated endocytosis. In the form of endocytosis the cytoplasm membrane folds inward to form coated pits (Endosome). These vesicles are subsequently carried into the cell cytosol and are fused with the lysosome. The lysosome then digests the carrier system (at a pH of approximately pH 4.8) to release the active substance into the cell cytosol.

The procedures according to the present application produce a carrier system complex that is unexpectedly stable in comparison to the prior art siosome encapsulation, entrapment and/or complex formation, that is, the carrier system of the present invention is easier to produce, has a higher encapsulation efficiency and exhibits a higher intracellular concentration of active agent, and leads to more specific cell targeting of the active substances and also exhibits less toxicity.

Furthermore, it is a preferred embodiment of the present invention that the method of preparation of the carrier system can be carried out by the entrapment of the siosomal nanoparticles in the lipid bilayer of the siosomes. The selection of the optimal sugar silanes/derivatives and/or siosomes for the entrapment of the small drugs (pharmaceutical agents, photo-sensitizer, and magnetic agents for magnetic hyperthermia) will depend on the required release profile of the drugs and/or agents encapsulated in the larger Siosomes.

The selection of the optimal sugar silanes for the encapsulation of the small drug will depend on the organs and tissue of interest.

The silanes/sugar silanes according to the present invention offer enormous possibilities with different molecular structures, size, shape and charge.

The Siosomes® prepared from the silanes/sugar silanes using the procedures according to the present invention will offer very specific cell/tissue targeting based on the selection of the sugar molecules on the surface of the siosomes®. It is known that the sugar molecules are responsible for the cell recognition and communication.

According to the present invention, the objective of the combined carrier system could be for example: to target the carrier system to accumulate in the tissues of interest e. g. under the influence/effect of magnet field and/or temperature, the release of the siosomal nanoparticle will take place followed by the release of the small molecules (drugs/agents) active agents and the release of the active agent(s) from the larger siosomes in different release and efficacy profiles.

In addition, the objective of the combined carrier system according to the present invention is to release the agent from the siosomal nanoparticle to trigger and/or enhance the cell penetration and targeting of the agent and/or biological agent encapsulated/entrapped in the larger Siosomes.

In a number of studies with Doxorubicin, it has been suggested that the toxicity, including chronic cardiotoxicity can be reduced by lowering peak plasma levels.

Siosomes® with the encapsulated drug have shown unexpected less plasma protein and tissue binding than the free drug. For example, Cisplatin has very high plasma protein binding of 90%. This means the effective free Cisplatin fraction is only 10% of the total therapeutic dose. The unexpected lower protein binding using the encapsulated drug means that the total therapeutic dose and the toxic effects could be reduced drastically due to the unexpected increase of the effective free drug fraction. Doxorubicin has very extensive tissue binding. This has been unexpected decreased using Siosomes® for its encapsulation.

The use of Siosomes®have shown unexpected the ability to avoid the drug distribution to certain organs and provides a way to prevent organ specific toxicities such as Doxorubicin cardio-toxicity. In addition the Dose-Limiting-Toxicity (DLT) for encapsulated Siosomes is unexpected lower than that of the free un-encapsulated drug, and the delivery of the encapsulated drug will be targeted to cellular compartments different than that accessed by free drug.

According to the procedures of this invention, it has been shown that the encapsulated and/or entrapped molcules of the chemostatic agents such as the anticancer drugs Cisplatin, Carboplatin, Oxaliplatin, Doxorubicin and Docetaxel will not be chemically active in the target tissues until their release from the Siosomes.

For example Cisplatin binds DNA fast and directly forming adducts and in case that are not removed the DNA replication and transcription will be blocked. Using the Siosomes® for the encapsulation of the Cisplatin according to this invention, in this case the chemical reaction will take place gradually upon the release of the cisplatin molecules from the Siosomes. For example this will allow the kidney to clear the drug and avoid or reduce the nephrotoxicity.

The procedures according to this invention for the preparation of the Siosomes with the combination of the short acting, with a predetermined free anticancer agent, and of the long acting with predetermined part of the anticancer agent encapsulated in Siosomes ®have shown unexpected that in this case the determined "Therapeutic Dose" of the combination is lower than the "Therapeutic Dose" of the standard parenteral anticancer infusion formulation. This is due to the lower protein binding and the different PK/PD profiles of the Siosomes combination.

It was unexpected that the bioavailability, pharmacokinetic and tissue distribution of the Siosomes®-Chemostatic-Complex of the Siosomes combination have shown different release and efficacy profiles than the standard and commercially available parenteral anticancer drugs.

It is known, that the toxicity is dependent on the systemic, tissue and organ drug concentration and drug clearance and bioaccumulation in organs such as liver, spleen, kidney, and lung tissues.

Therefore, short acting drugs (immediate systemic release formulations) as standard parenteral Cisplatin , Carboplatin, Oxaliplatin, Doxorubicin and Docetaxel infusions cause in the "determined therapeutic dose" an overdose of systemic and tissue drug/metabolites concentrations with selective acute and chronic drug toxicity due to the high "absolute systemic bioavailabilty" of the drug.

Unexpectedly the use of the procedures according to this invention for the preparation of the Siosomes®-Chemostatic-Complex formulations have shown a number of advantages in comparison to the commercially available formulations and improved systemic and tissues/organs clearance of the chemostatics drugs such as lowering the peak plasma level (toxic blood concentration) and improved systemic and tissues/organs clearance of the chemostatics drugs to avoid and reduce the acute and chronic toxicity.

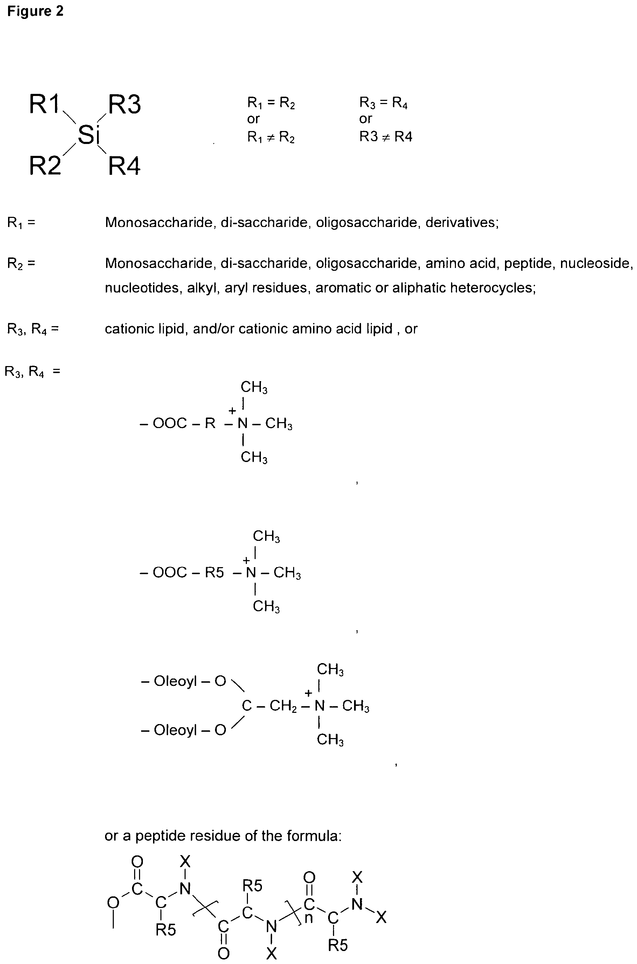

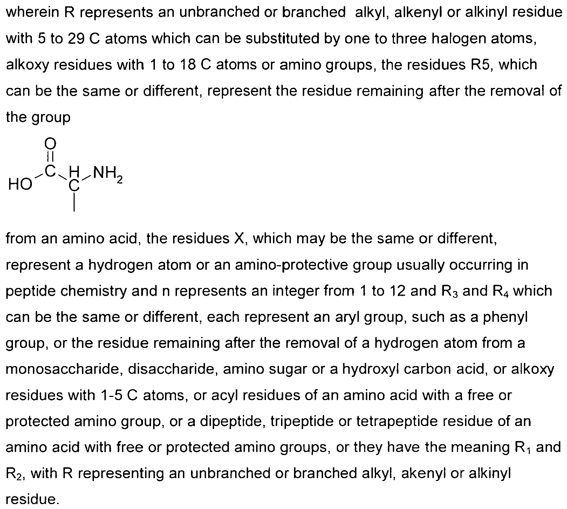

Unexpectedly, these compounds and derivatives of the general formula (I) form surprisingly stable carrier molecules and differ significantly from the siosomes and liposomes described in the prior art. Such vesicles are structurally distinct from the carrier systems of the prior art. Furthermore, it is a preferred embodiment of the invention to provide specific carrier systems for the intracellular "transfection" delivery of active substances as chemostatics, anti-cancer agents, such as doxorubicin, cis-platin, siRNA and nucleic acids, such as RNA, DNA, antiviral and antibacterial agents and active substances for CNS disorders, whereby one or more of the cationic lipids are covalently attached to the silicon and/or to the chemical groups and/or residues of R₁, R₂, R₃ and R₄ as described in Figure 2. These compounds according to the present invention are used as cationic sugar silanes and/or cationic sugar siosomes for the encapsulation, entrapment and/or complex formation of active substances. Unexpectedly, these compounds form stable carriers with high encapsulation efficiency and intracellular delivery of the active substances and specifically the nucleic acids, such as siRNA and anticancer, antiviral, antibacterial agents and therapeutics for the treatment of CNS disorders.

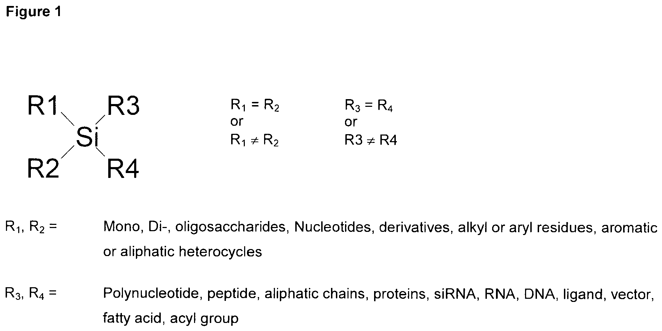

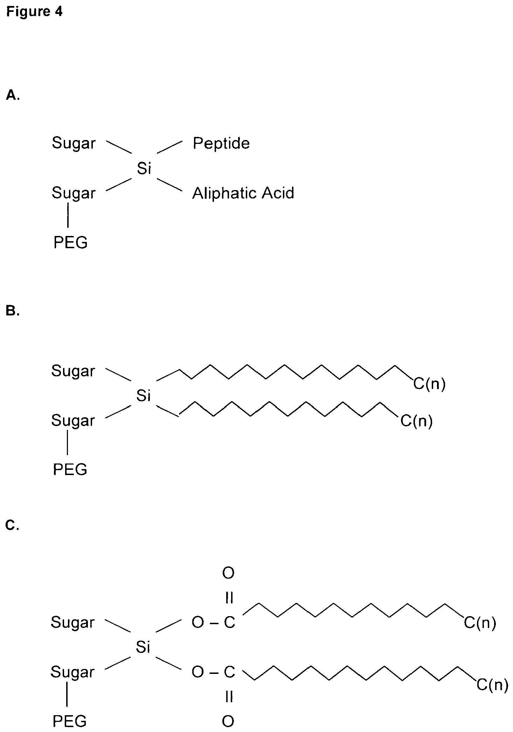

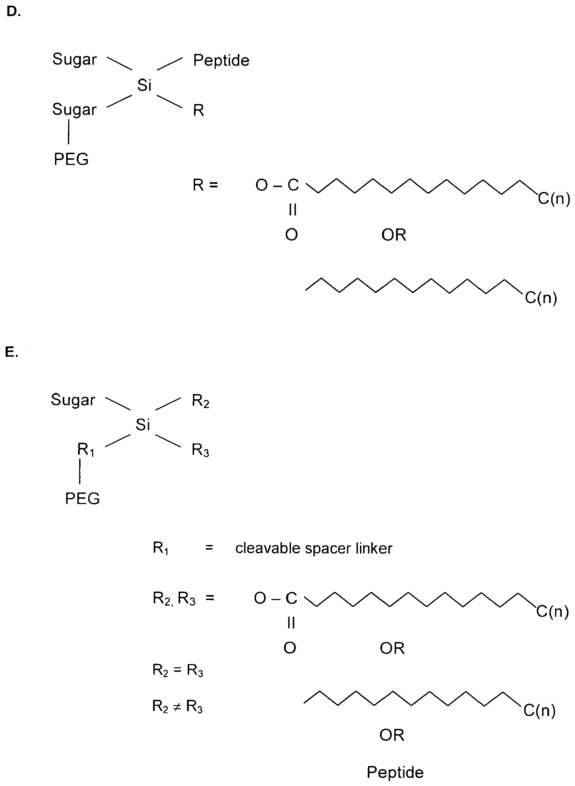

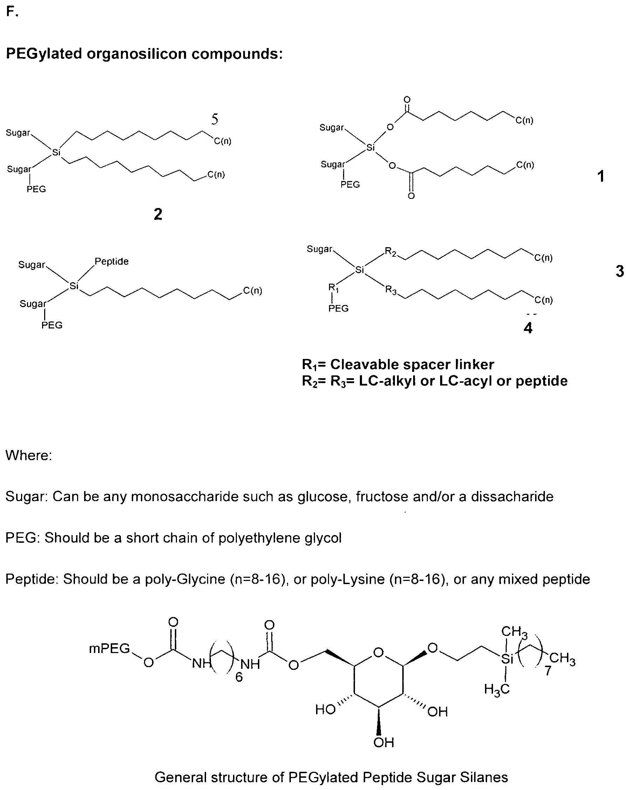

In yet another embodiment, the present invention provides a carrier and delivery system for active substances, whereby polyethylene-glycols/derivatives of varying molecular weights can be used to be covalently attached to the silicone with or without spacer and/or chemical group or covalently attached to one or more of the groups R₁, R₂, R₃ and R₄ of the general formula according to figure 1. Examples of PEGylated silane molecules of the present invention are found in Figure 4. According to the present application, the covalent attachment can be carried out by means of a cleavable spacer linker.

Furthermore, it is a preferred embodiment of the present invention that the method of preparation of the carrier system can be carried out by mixing the active substances with the polyethylene-glycols of varying molecular weights at selected pH and temperature with an appropriate solvent. The active substance-PEG mixture can be used for the preparation of the carrier and delivery system according to the procedures described in this invention.

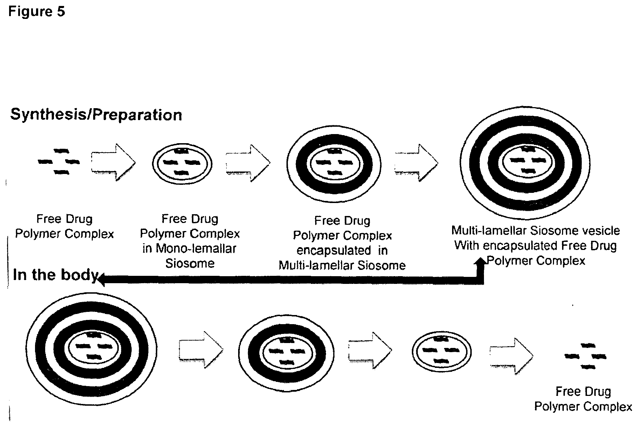

Surprisingly, preparation of a carrier system using these procedures produces stable vesicles with high encapsulation and entrapment efficiency and delayed release of the active substances in the target cells (Figures 5 - 8).

In the method according to the invention, siosomes as carrier systems made from non-toxic lipids, peptides, carbohydrates, polymers, nucleosides and/or other ingredients are used, wherefore they are applicable for in vivo use in different therapeutics.

Furthermore, it is a preferred embodiment of the present invention that the active substance, such as nucleic acids, DNA, RNA, siRNA can be loaded with cations prior to the preparations of the siosomes carrier systems according to the procedures described in the examples of this invention (Figures 5 - 8).

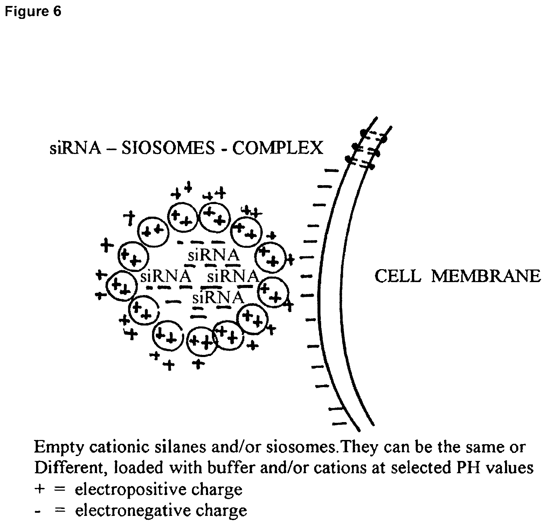

Furthermore, it is a preferred embodiment of the present invention that the cationic silanes and/or siosomes can be loaded with anionic salts for the preparation of the siosome carrier and targeting systems according to the procedures described in the examples of the present invention (Figures 6).

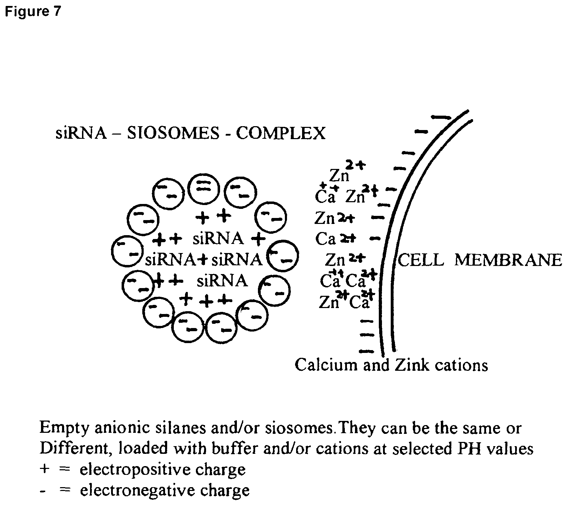

Also disclosed herein is that the anionic silanes and/or siosomes can be loaded with cationic salts for the preparation of the siosome carriers and intracellular targeting systems according to the procedures described in the examples of the present invention (Figures 7).

Furthermore, it is a preferred embodiment of the present invention, that the siosomes carrier and targeting system can be comprised from one or more silanes according to the procedures described in the examples of the present invention.

It is a further preferred embodiment of the invention that the carrier and targeting systems can be prepared using silanes described in the present invention and lipids used for the preparation of the liposomes, such as cationic lipids, known to person skilled in the art.

Unexpectedly, the above mentioned methods of preparations of the active substances, cationic silanes and/or siosomes and the mixed siosomes and lipo-sio-carrier systems are structurally distinct from the carrier systems of the prior art and exhibit adjustable encapsulation efficiency, release profile and transfection properties and efficiencies for the active substances.

It is a preferred embodiment of the present invention, that buffer solution containing TRIS, HEPES; MOPS, MES or other commonly used biological buffers will be used for the preparation of the siosome carrier and targeting system. The use of such buffer surprisingly enhances the efficiency, strength and stability of the active substance-siosome complex.

A preferred embodiment of the present invention is that the method for preparation of the carrier system includes one or more of the following additives, including detergent solution, adjuvant solution, salt solution, PEG-conjugates, cryoprotectants, solubilizers solution and/or other vesicles and ingredients.

In the method according to the invention, siosomes and/or siosomes-liposomes-complex (sio-lipo) may be administered as oral, rectal, vaginal, topical, nasal, intradermal, or parenteral, the term parenteral including subcutaneous, intravenous, intramuscular and infusion. This particular mode of administration will depend, of course, upon a particular drug selected, the severity of the condition being treated and the dosage required for the therapeutic efficacy.

The preferred organosilicon, sugar organosilicon, and amino sugar organosilicon compounds are listed in table 1.

**Table 1. Preferred organosilicon, sugar organosilicon, and amino sugar organosilicon compounds for carrier system production.**

| No. | Structure and Name | Molecular Formula | MW (Da) |
|---|---|---|---|
| Sil 1 | 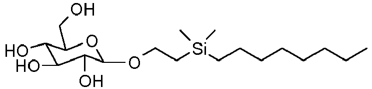 | | |
| | 2-(Dimethyloctylsilyl)ethyl-β-D-glucopyranosid | C18H38O6Si | 378.59 |
| Sil 2 | 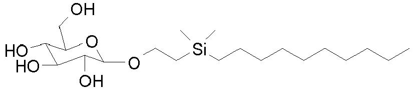 | | |
| | 2-(Dimethyldecylsilyl)ethyl-β-D-glucopyranosid | C20H42O6Si | 406.64 |
| Sil 3 | 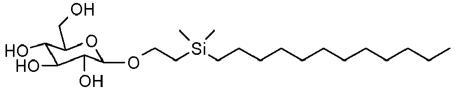 | | |
| | 2-(Dimethyldodecylsilyl)ethyl-β-D-glucopyranosid | C22H46O6Si | 434.69 |
| Sil 4 | 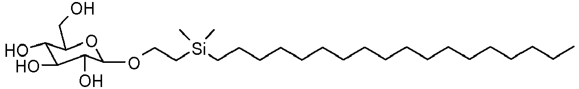 | | |
| | 2-(Dimethyloctadecylsilyl)ethyl-β-D-glucopyranosid | C28H58O6Si | 518.86 |
| Sil 5 | 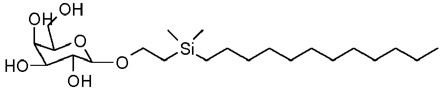 | | |
| | 2-(Dimethyldodecylsilyl)ethyl-β-D-galactopyranosid | C22H46O6Si | 434.69 |
| Sil 6 | 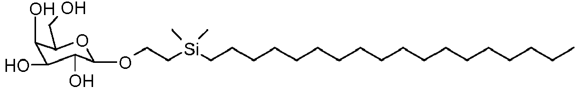 | | |
| | 2-(Dimethyloctadecylsilyl)ethyl-β-D-galactopyranosid | C28H58O6Si | 518.86 |
| SIL 7 | 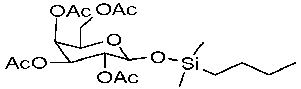 | | |
| | 1-O-(Butyldimethylsilyl)-2,3,4,6-tetra-O-acetyl-galactose | C20H34O10Si | 462.57 |
| Sil 7.1 | 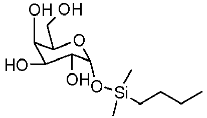 | | |
| | Butyldimethylsilyl-α-D-galactopyranosid | C12H26O6Si | 294.42 |
| SIL 8 | 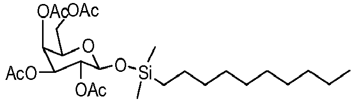 | | |
| | 1-O-(Decyldimethylsilyl)-2,3,4,6-tetra-O-acetyl-galactose | C26H46O10Si | 546.76 |
| Sil 8.1 | 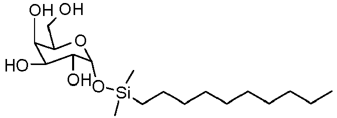 | | |
| | Decyldimethylsilyl-α-D-galactopyranosid | C18H35O6Si | 378.59 |
| SIL 9 | 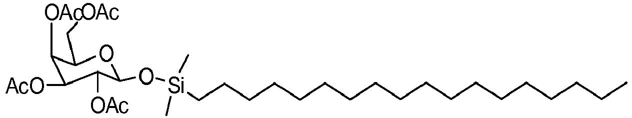 | | |
| | 1-O-(Octadecyldimethylsilyl)-2,3,4,6-O-tetraacetyl-galactose | C34H62O10Si | 658.95 |
| Sil 9.1 | 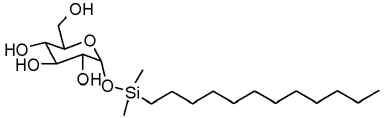 | | |
| | Dodecyldimethylsilyl-α-D-glucopyranosid | C20H42O6Si | 406.64 |
| SIL 10 | 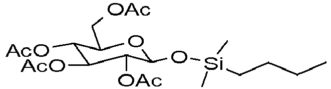 | | |
| | 1-O-(Butyldimethylsilyl)-2,3,4,6-tetra-O-acetyl-glucose | C20H34O10Si | 462.57 |
| Sil 10.1 | 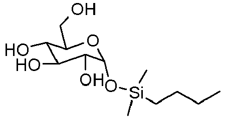 | | |
| | Butyldimethylsilyl-α-D-glucopyranosid | C12H26O6Si | 294.42 |
| SIL 11 | 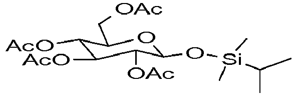 | | |
| | 1-O-(Isopropyldimethylsilyl)-2,3,4,6-O-tetraacetyl-glucose | C19H32O10Si | 448.55 |
| Sil 11.1 | 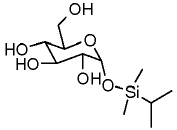 | | |
| | Dimethyl-isopropylsilyl-α-D-glucopyranosid | C11H24O6Si | 280.40 |
| SIL 12 | 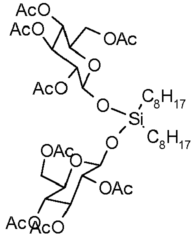 | | |
| | 1-O-Dioctylsilyl-di(2,3,4,6-O-tetraacetyl-β-D-glucopyranosid) | C44H72O20Si | 949.14 |
| SIL 13 | 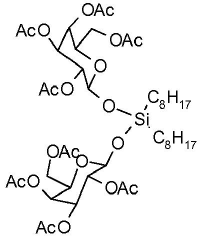 | | |
| | 1-O-Dioctylsilyl-di(2,3,4,6-O-tetraacetyl-β-D-galactopyranosid) | C44H72O20Si | 949.14 |
| SIL 14 | 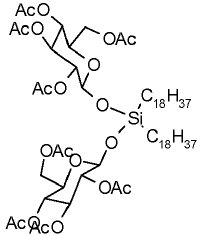 | | |
| | 1-O-Dioctadecylsilyl-di(2,3,4,6-O-tetraacetyl-β-D-glucopyranosid) | C64H112O20Si | 1229.68 |
| SIL 15 | 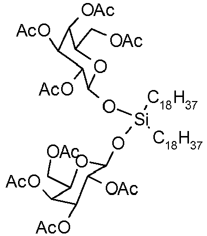 | | |
| | 1-O-Dioctadecylsilyl-di(2,3,4,6-O-tetraacetyl-β-D-galactopyranosid) | C64H112O20Si | 1229.68 |
| SIL 16 | 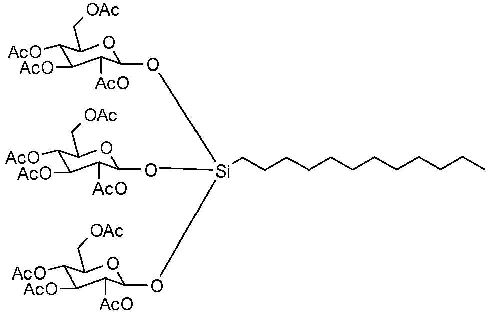 | | |
| | Dodecylsilyl-tris(2,3,4,6-O-tetraacetyl-β-D-glucopyranosid) | C54H82O30Si | 1239.32 |
| SIL 17 | 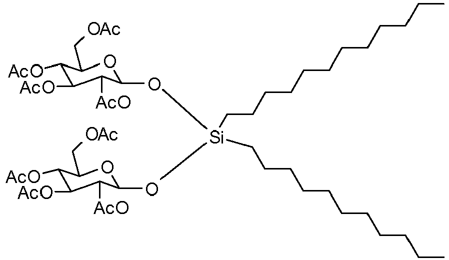 | | |
| | Didoceclylsilyl-di(2,3,4,6-O-tetraacetyl-β-D-glucopyranosid) | C52H88O20Si | 1061.36 |
| SIL 18 | 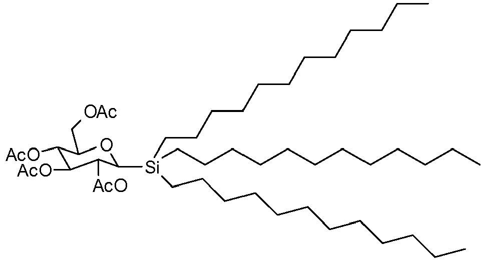 | | |
| | Tridodecylsilyl-(2,3,4,6-O-tetraacetyl-β-D-glucopyranosid) | C50H94O10Si | 883.39 |
| SIL 19 | 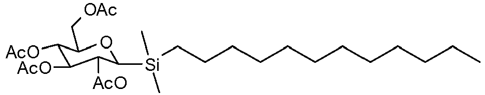 | | |
| | 1-O-Dimethyl(dodecyl)silyl-(2,3,4,6-O-tetraacetyl-β-D-glucopyranosid) | C28H50O10Si | 574.79 |
| SIL 20 | 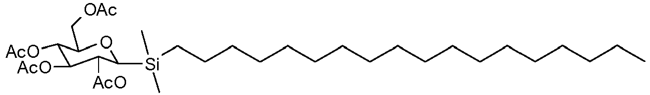 | | |
| | 1-O-Dimethyl(octadecyl)silyl-(2,3,4,6-O-tetraacetyl-β-D-glucopyranosid) | C34H62O10Si | 658.95 |
| SIL 21 | 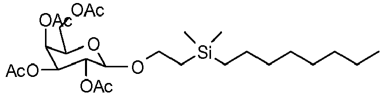 | | |
| | 2-(Dimethyloctylsilyl)ethyl-2,3,4,6-O-tetraacetyl-β-D-galactopyranosid | C26H46O10Si | 546.74 |
| Sil 21.1 | 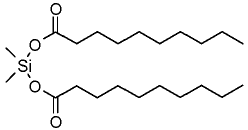 | | |
| | Di(decanoyloxy)dimethylsilan | C22H44O4Si | 400.68 |
| SIL 22 | 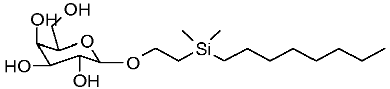 | | |
| | 2-(Dimethyloctylsilyl)ethyl-β-D-galactopyranosid | C18H38O6Si | 378.59 |
| Sil 22.1 | 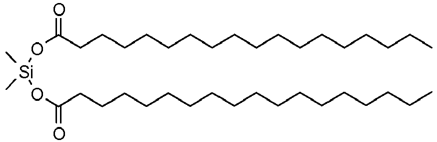 | | |
| | Di(octadecanoyloxy)dimethylsilan | C38H76O4Si | 625.09 |
| SIL 23 | 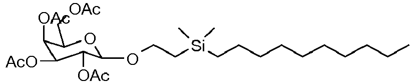 | | |
| | 2-(Dimethyldecylsilyl)ethyl-2,3,4,6-O-tetraacetyl-β-D-galactopyranosid | C28H50O10Si | 574.79 |
| Sil 23.1 | 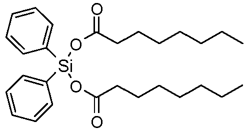 | | |
| | Di(octanoyloxy)diphenylsilan | C28H40O4Si | 468.70 |
| SIL 24 | 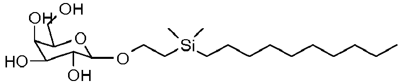 | | |
| | 2-(Dimethyldecylsilyl)ethyl-β-D-galactopyranosid | C20H42O6Si | 406.64 |
| Sil 24.1 | 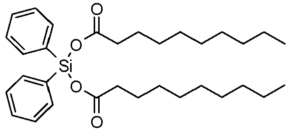 | | |
| | Di(decanoyloxy)diphenylsilan | C32H48O4Si | 524.82 |
| SIL 25 | 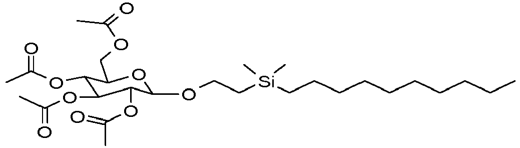 | | |
| | 2-(Dimethyldecylsilyl)ethyl-2,3,4,6-O-tetraacetyl-β-D-glucopyranosid | C28H50O10Si | 574.79 |
| Sil 25.1 | 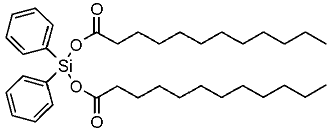 | | |
| | Di(dodecanoyloxy)diphenylsilan | C36H56O4Si | 580.93 |
| SIL 26 | 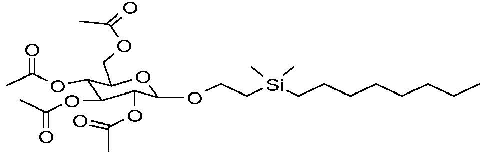 | | |
| | 2-(Dimethyloctylsilyl)ethyl-2,3,4,6-O-tetraacetyl-β-D-glucopyranosid | C26H46O10Si | 546.74 |
| Sil 26.1 | 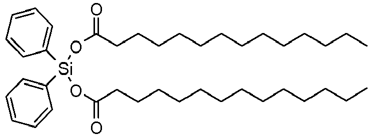 | | |
| | Di(tetradecanoyloxy)diphenylsilan | C40H64O4Si | 637.04 |
| SIL 27 | 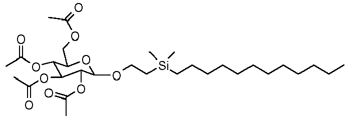 | | |
| | 2-(Dimethyldodecylsilyl)ethyl-2,3,4,6-O-tetraacetyl-β-D-glucopyranosid | C30H54O10Si | 602.84 |
| Sil 27.1 | 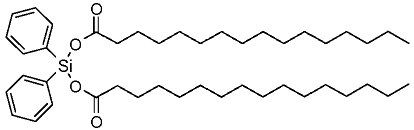 | | |
| | Di(hexadecanoyloxy)diphenylsilan | C44H72O4Si | 693.15 |
| SIL 28 | 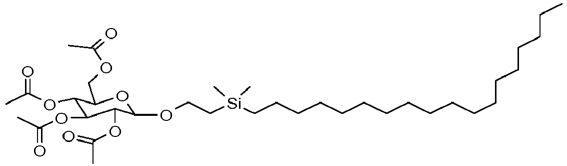 | | |
| | 2-(Dimethyloctadecylsilyl)ethyl-2,3,4,6-O-tetraacetyl-β-D-glucopyranosid | C36H66O10Si | 687.01 |
| Sil 28.1 | 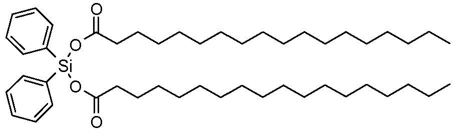 | | |
| | Di(octadecanoyloxy)diphenylsilan | C48H80O4Si | 749.26 |
| SIL 29 | 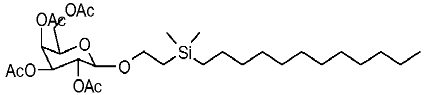 | | |
| | 2-(Dimethyldodecylsilyl)ethyl-2,3,4,6-O-tetraacetyl-β-D-galactopyranosid | C30H54O10Si | 602.84 |
| Sil 29.1 | 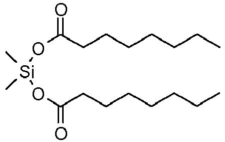 | | |
| | Di(octanoyloxy)dimethylsilan | C18H36O4Si | 344.57 |
| SIL 30 | 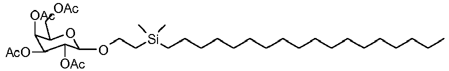 | | |
| | 2-(Dimethyloctadecylsilyl)ethyl-2,3,4,6-O-tetraacetyl-β-D-galactopyranosid | C36H66O10Si | 687.01 |
| Sil 30.1 | 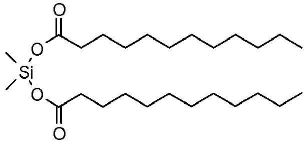 | | |
| | Di(dodecanoyloxy)dimethylsilan | C26H52O4Si | 456.79 |
| SIL 31 | 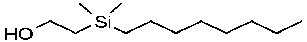 | | |
| | 2-(Dimethyl-octyl-silanyl)-ethanol | C12H28OSi | 216.44 |
| Sil 31.1 | 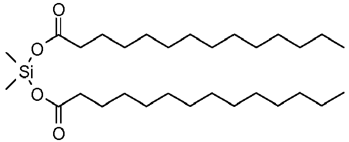 | | |
| | Di(tetradecanoyloxy)dimethylsilan | C30H60O4Si | 512.90 |
| SIL 32 | 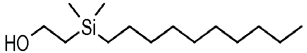 | | |
| | 2-(Dimethyl-decyl-silanyl)-ethanol | C14H32OSi | 244.5 |
| Sil 32.1 | 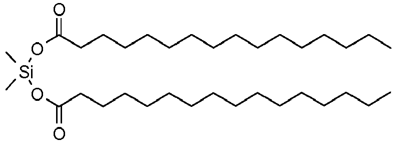 | | |
| | Di(hexadecanoyloxy)dimethylsilan | C34H68O4Si | 569.00 |
| SIL 33 | 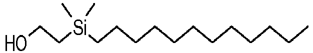 | | |
| | 2-(Dimethyl-dodecyl-silanyl)-ethanol | C16H36OSi | 272.55 |
| Sil 33.1 | 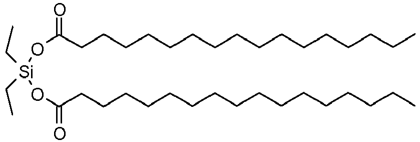 | | |
| | Di(heptadecanoyloxy)diethylylsilan | C38H76O4Si | 625.11 |
| SIL 34 | 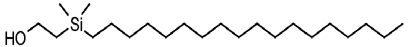 | | |
| | 2-(Dimethyl-octadecyl-silanyl)-ethanol | C22H48OSi | 356.71 |
| Sil 34.1 | 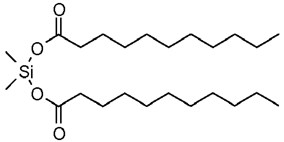 | | |
| | Di(undecanoyloxy)dimethylsilan | C24H48O4Si | 428.73 |
| SIL 35 | 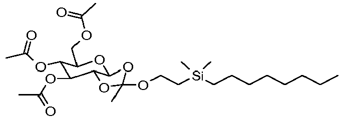 | | |
| | Acetic acid 6,7-diacetoxy-2-[2-(dimethyl-octyl-silanyl)-ethoxy]-2-methyl-tetrahydro-[1,3]dioxolo[4,5-b]pyran-5-ylmethyl ester | C26H46O10Si | 546.74 |
| Sil 35.1 | 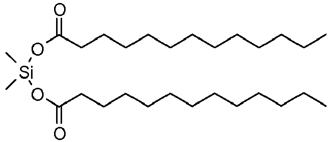 | | |
| | Di(tridecanoyloxy)dimethylsilan | C28H56O4Si | 484.84 |
| SIL 36 | 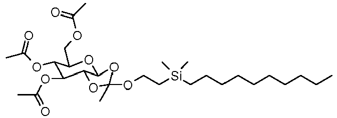 | | |
| | Acetic acid 6,7-diacetoxy-2-[2-(decyl-dimethyl-silanyl)-ethoxy]-2-methyl-tetrahydro-[1,3]dioxolo[4,5-b]pyran-5-ylmethyl ester | C28H50O10Si | 574.79 |
| Sil 36.1 | 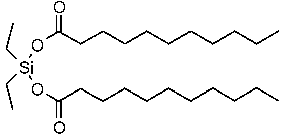 | | |
| | Di(undecanoyloxy)diethylylsilan | C26H52O4Si | 456.79 |
| SIL 37 | 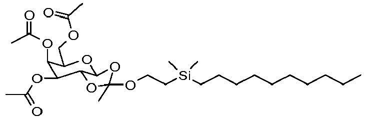 | | |
| | Acetic acid 6,7-diacetoxy-2-[2-(decyl-dimethyl-silanyl)-ethoxy]-2-methyl-tetrahydro-[1,3]dioxolo[4,5-b]pyran-5-ylmethyl ester | C28H50O10Si | 574.79 |
| Sil 37.1 | 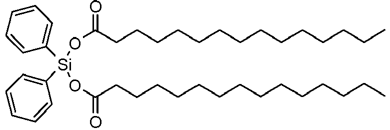 | | |
| | Di(pentadecanoyloxy)diphenylsilan | C42H68O4Si | 665.09 |
| SIL 38 | 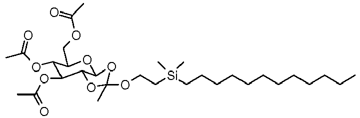 | | |
| | Acetic acid 6,7-diacetoxy-2-[2-(dodecyl-dimethyl-silanyl)-ethoxy]-2-methyl-tetrahydro-[1,3]dioxolo[4,5-b]pyran-5-ylmethyl ester | C30H54O10Si | 602.84 |
| Sil 38.1 | 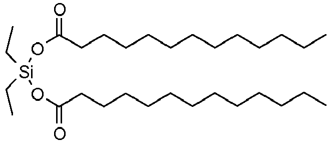 | | |
| | Di(tridecanoyloxy)diethylylsilan | C30H60O4Si | 512.90 |
| SIL 39 | 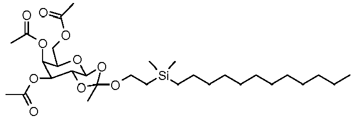 | | |
| | Acetic acid 6,7-diacetoxy-2-[2-(dodecyl-dimethyl-silanyl)-ethoxy]-2-methyl-tetrahydro-[1,3]dioxolo[4,5-b]pyran-5-ylmethyl ester | C30H54O10Si | 602.84 |
| Sil 39.1 | 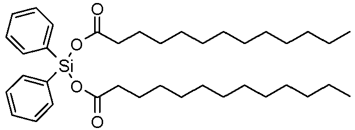 | | |
| | Di(tridecanoyloxy)diphenylsilan | C38H60O4Si | 608.99 |
| SIL 40 | 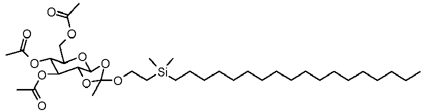 | | |
| | Acetic acid 6,7-diacetoxy-2-[2-(dimethyloctadecyl-silanyl)-ethoxy]-2-methyl-tetrahydro-[1,3]dioxolo[4,5-b]pyran-5-ylmethyl ester | C36H66O10Si | 687.01 |
| Sil 40.1 | 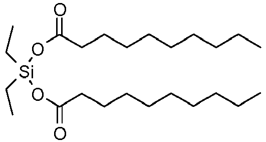 | | |
| | Di(decanoyloxy)diethylylsilan | C24H48O4Si | 428.73 |
| Sil 41.1 | 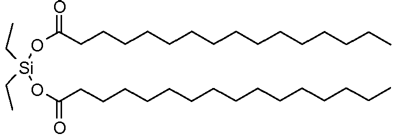 | | |
| | Di(hexadecanoyloxy)diethylylsilan | C36H72O4Si | 597.06 |
| SIL 42 | 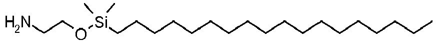 | | |
| | 2-(Dimethyl-octadecyl-silanyloxy)-ethylamine | C22H49NOSi | 371.73 |
| Sil 42.1 | 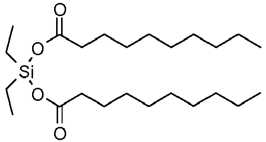 | | |
| | Di(decanoyloxy)diethylylsilan | C24H48O4Si | 428.73 |
| SIL 43 | 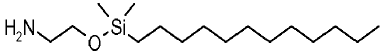 | | |
| | 2-(Dimethyl-dodecyl-silanyloxy)-ethylamine | C16H37NOSi | 287.57 |
| Sil 43.1 | 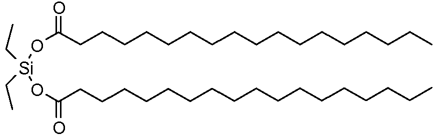 | | |
| | Di(octadecanoyloxy)diethylylsilan | C40H80O4Si | 653.17 |
| SIL 44 | 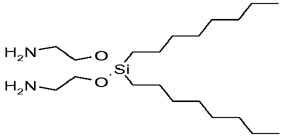 | | |
| | 2-[(2-Amino-ethoxy)-dioctyl-silanyloxy]-ethylamine | C20H46N2O2Si | 374.69 |
| Sil 44.1 | 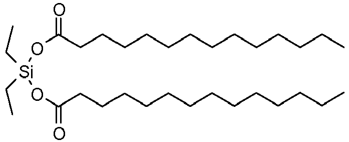 | | |
| | Di(tetradecanoyloxy)diethylylsilan | C32H64O4Si | 540.95 |
| SIL 45 | 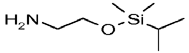 | | |
| | 2-(Dimethyl-octadecyl-silanyloxy)-ethylamine | C7H19NOSi | 161.32 |
| Sil 45.1 | 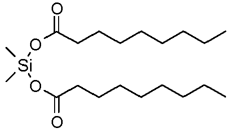 | | |
| | Di(nonanoyloxy)dimethylsilan | C20H40O4Si | 372.63 |
| SIL 46 | 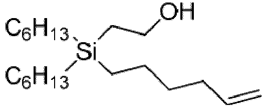 | | |
| | 2-(Hex-5-en-1-yldihexylsilyl)ethanol | C20H42OSi | 326.63 |
| SIL 47 | 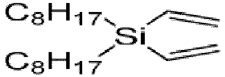 | | |
| | Dioctyldivinylsilane | C20H40Si | 308.63 |
| SIL 48 | 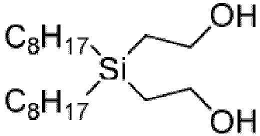 | | |
| | 2,2'-(Dioctylsilanediyl)diethanol | C20H44O2Si | 344.65 |
| SIL 49 | 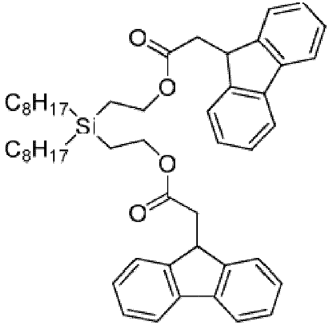 | | |
| | Bis-2-((Dioctylsilyl)ehtyl(9H-fluoren-9-yl)acetate) | C50H64O4Si | 757.13 |
| SIL 50 | 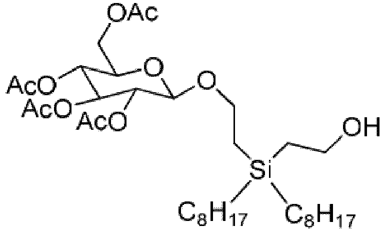 | | |
| | 2-((2-Hydroxyethyl)dioctylsilyl)ethyl 2(2,3,4,6-tetra-O-acetyl)-beta-D-glycopyranose | C34H62O7Si | 610.93 |
| SIL 51 | 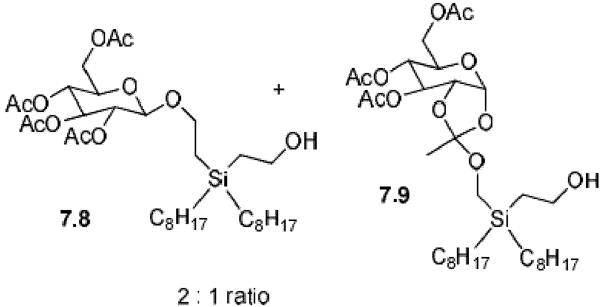 | | |
| | Combination of 2-((2-Hydroxyethyl)dioctylsily)ethyl 2-beta-D-glycopyranose and its 1,3 dioxolo(4,5-b)pyran derivative | | |
| SIL 52 | 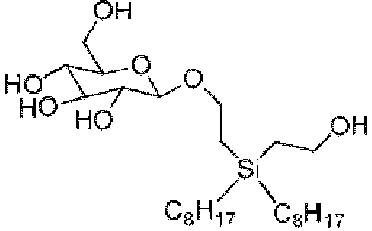 | | |
| | 2-((2-Hydroxyethyl)dioctylsily)ethyl 2-beta-D-glycopyranose | C26H54O7Si | 506.79 |
| SIL 53 | 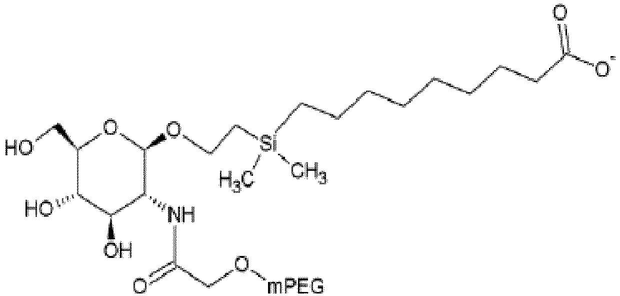 | | |
| | 2-(Dimethyl nonaoic acid silyl)ethyl-beta-D-PEG-acetylglucosaminether | C21H39NO9Si | 477.62 |
| | Remark: PEGylated Anionic Sugar Silanes | | |
| SIL 54 | 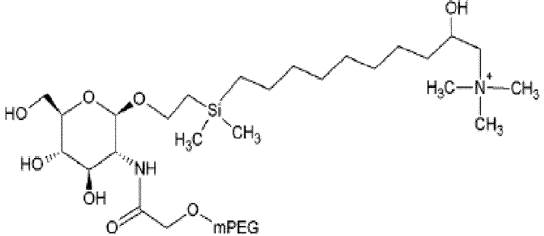 | | |
| | 2-(Dimethyloctylsilylcholin)ethyl-beta-D-PEG-acetylglucosaminether | C25H52N2O8Si | 536.77 |
| | Remark: PEGylated Cationic Sugar Silanes | | |
| SIL 55 | 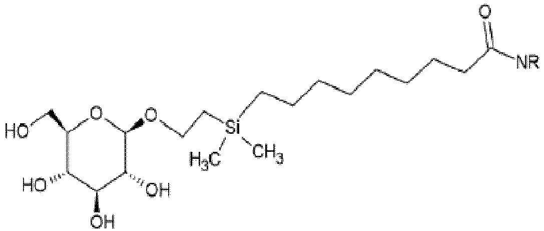 | | |
| | Example: R = ATP | C29H52O20N6 P3Si | 925.76 |
| | Remark: The residue R represents mono-di and/or polynucleotides and/or their derivatves, such as but not limited to: C,G,CG,GC,GTGCTT, Adenosine Triphosphate (ATP), Adenosine Diphosphate (ADP), Adenosine Monophosphate (AMP) | | |
| SIL 56 | 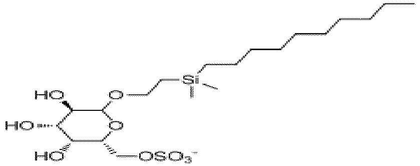 | | |
| | 2-(Dimethyldecylsilyl)ethyl-beta-D-glucopyranosidsulfate | C20H41O9SSi | 485.68 |
| | 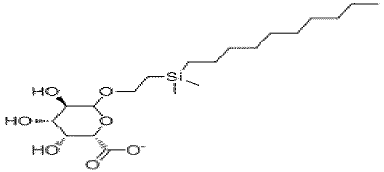 | | |
| | 2-(Dimethyldecylsilyl)ethyl-beta-D-glucopyranosidcarboxylate | C20H39O7Si | 419.6 |
| SIL58 | 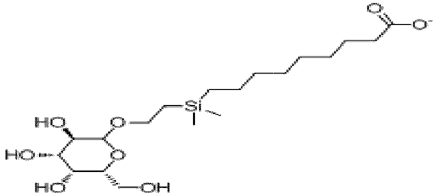 | | |
| | 2-(Dimethylnonanoic acid silyl)ethyl-beta-D-glucopyranosid | C19H37O8Si | 421.58 |
| SIL59 | 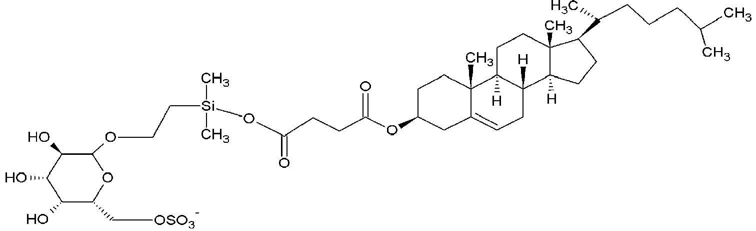 | | |
| | 2-(Dimethylcholesterylhemisuccinatesilyl)ethyl-beta-D-glucopyranosid sulfate | C40H68O13SSi | 817.11 |
| SIL60 | 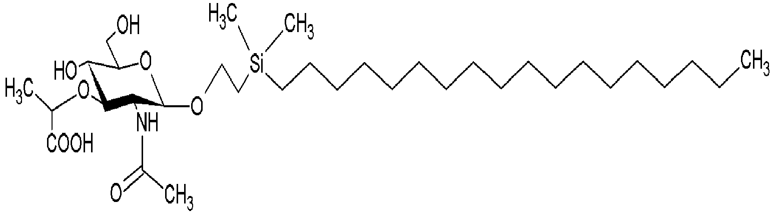 | | |
| | 2-(Dimethyloctadecylsilyl)ethyl-beta-N-acetylmuramic acid | C33H64O8Si | 616.94 |
| Cationic sugar organosilicon and amino sugar organosilicon compounds | | | |
| SIL61 | 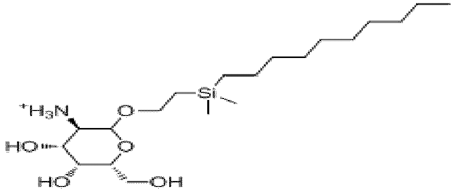 | | |
| | 1. 2-(Dimethyldecylsilyl) ethyl-beta-D-glucosamin | C20H44O5Si | 392.64 |
| SIL62 | 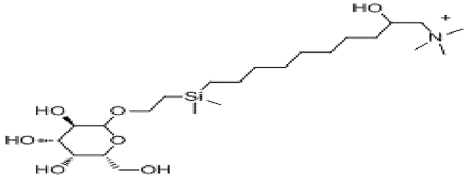 | | |
| | 2. 2-(Dimethyloctylsilylcholin) ethyl-beta-D-glucopyranosid | C23H49O7NSi | 479.72 |
| SIL63 | 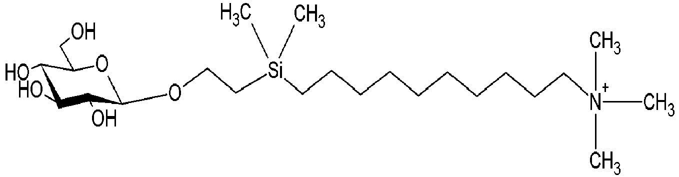 | | |
| | 1. 2-(Di-N,N,N, trimethylammoniadecylsilyl) ethyl-beta-D-glucopyranosid | C23H50O6NSi | 464.73 |
| SIL64 | 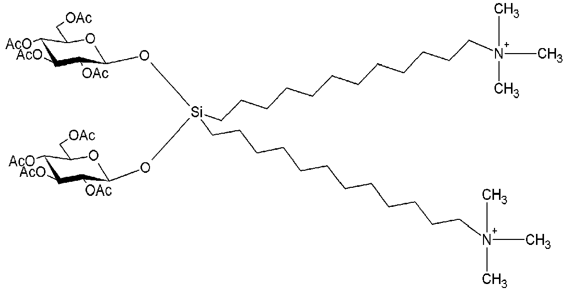 | | |
| | Di-N,N,N, trimethylammoniadidodecylsilyl-di(2,3,4,6-O-tetraacetyl-beta-D-glucopyranosid) | C38H128O20N 2Si | 961.5 |
| SIL65 | 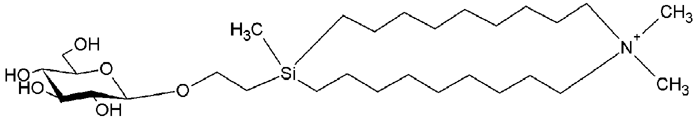 | | |
| | 2-(N,N,N,Dimethylammonia-methyl-decylsilyl)ethyl-beta-D-glucopyranosid | C29H60O6NSi | 546.87 |
| Zwitterionic and amino sugar organosilicon | | | |
| SIL66 | 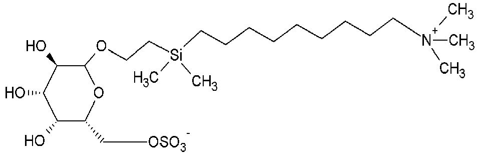 | | |
| | 2-(N,N,N,trimethylammonia nonanyllsilyl)ethyl-beta-D-glucopyranosid sulfate | C22H47O9NSS i | 463.68 |
| SIL67 | 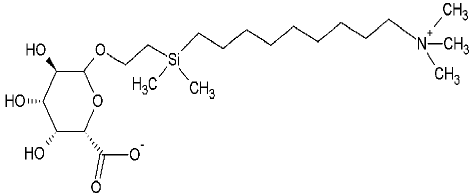 | | |
| | 2-(N,N,N,trimethylammonia nonyllsilyl)ethyl-beta-D-glucopyranosid | C22H45O7NSi | 463.68 |
| SIL68 | 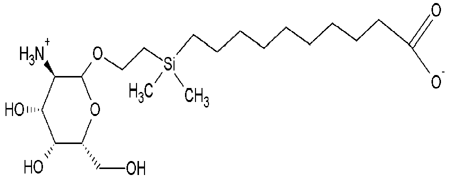 | | |
| | 2-(Dimethyldecyllsilyl)ethyl-beta-D-glucosamine | C20H41O7NSi | 435.63 |

### DEFINITIONS:

The following terms are defined as follows.

"Silane" is a chemical compound containing silicone, particularly with the chemical formula SiH₄, although other silicon-containing compounds may also be referred to herewith.

"Organosilicons" are organic compounds containing carbon-silicon bonds (C-Si).

"Sugar organosilicons" are organic compounds containing carbon-silicon bonds (C-Si) and at least one sugar group.

"Amino sugar organosilicons" are organic compounds containing carbon-silicon bonds (C-Si), at least one sugar group and one amino and/or acid group. Examples of amino sugars and derivatives are well known in the art as glucosamine, galactosamine, mannosamine, neuramine acid, muramine acid, N-acetylglucosamine, further examples include acylated sugars and aminosugars. Amino sugar organosilicons comprise residues such as glucosamine, N-acetyl glucosamine, sialic acid or galactosamine, where the amino sugar residues can be the same or different, and represent the removal of a hydrogen from monoaminosaccharide and/or diaminosaccharide and/or polysaccharide.

"Siloxanes" are a class of organic or inorganic chemical compounds of silicon, oxygen, and usually carbon and hydrogen, based on the structural unit R₂SiO, where R is an alkyl group, usually methyl.

"Siosomes" are the vesicles created from organosilicon compounds. Siosomes consist of at least one concentric, self-contained layer of organosilicon compounds with the organosilicon general structure. An aqueous compartment is enclosed by the bimolecular organosilicon membrane.

"Sugar-siosomes" refer to siosomes consisting of sugar organosilicon compounds.

As referred to in this patent "blank siosomes" refers to any and all vesicles prepared from organosilicon compounds without encapsulated and/or entrapped pharmacologically and/or immunologically active agents. Specific examples of "blank siosomes" are siosomes filled with water, salts or buffer.

"Liposomes" are the vesicles created from phospholipids. Liposomes consist of at least one concentric, self-contained layer of phospholipids and an aqueous compartment enclosed by the bimolecular phospholipid membrane.

"Encapsulations" are to be understood as the capture by a siosome or liposome of a dissolved hydrophilic solute within the region of aqueous solution inside a hydrophobic membrane, whereby the dissolved hydrophilic solutes cannot readily pass through the lipid bi-layer.

"Entrapments" are to be understood as the capture by a liposome or siosome of hydrophobic solutes dissolved in the hydrophobic region of the bi-layer membrane.

"Combinations" or "complex combinations" of the present invention are intended to be understood as complexes formed between the organosilicon, sugar organosilicon, amino sugar organosilicon compounds, their derivatives, salts and/or the vesicles formed from them, with the biological agents, selected from antigens, pre-antigens, antigen conjugates, antibodies, pre-antibodies, antibody conjugates, allergens, allergen extracts, nucleic acids, plasmids, proteins, peptides, pharmaceutical agents, immunologically active substances and/or cosmetics, whereby the complexes are held together via the sum force of non-covalent bonds including hydrophobic interactions between nonpolar regions, such as the hydrophobic fatty acid chains of the organosilicon, sugar organosilicon, amino sugar organosilicon compounds and siosomes, non-specific protein-protein interactions between protein segments of an allergen and/or the peptide residues of the organosilicon, sugar organosilicon, amino sugar organosilicon compounds, protein-saccharide interactions, Van der Waals interactions including, dipole-dipole interactions, dipole-induced dipole interactions, dispersion forces (London forces), electrostatic interactions between charged resides, for example peptide

residues and organosilicon, sugar organosilicon, amino sugar organosilicon compounds, and salts, ion-dipole forces; for example those between the salts of the buffer used in the preparation of the complex and H₂O, and ion-ion forces; for example those involving the salts of the buffer solution.

An "antigen" is to be understood as a substance that prompts the generation of antibodies and can cause an immune response.

A "pre-antigen" is to be understood as an antigen in an inactive state prior to processing to the active form.

An "antigen conjugate" is to be understood as an antigen conjugated covalently to another biological agent, such as an enzyme or other protein, or organosilicon molecule.

A "pre-antibody" is to be understood as an antibody in an inactive state prior to processing to the active form.

An "antibody conjugate" is to be understood as an antibody conjugated covalently to another biological agent, such as an enzyme or other protein, or organosilicon molecule.

The term "allergen" refers to a substance, protein or non-protein, capable of inducing allergy or specific hypersensitivity or an extract of any substance known to cause allergy. Almost any substance in the environment can be an allergen. The list of known allergens includes plant pollens, spores of mold, food preservatives, dyes, drugs, inorganic chemicals and vaccines. Allergens can enter the body by being inhaled, swallowed, touched or injected. Following primary exposure to an allergen, subsequent exposures result in hypersensitivity (allergic) reactions which may be immediate or delayed, local or systemic and include anaphylaxis and contact dermatitis.

The term "allergen extract" refers to a natural extract of multiple allergens, protein or non-protein, capable of inducing allergy or specific hypersensitivity or an extract of any substance known to cause allergy.

A "pharmaceutical agent" is to be understood as any medicament, intended for use in the diagnosis, cure, mitigation, treatment, or prevention of disease in humans or animals.

An "immunologically active substance" is to be understood as any substance that leads to an immune response in a human or animal patient.

As used herein "sugar" includes any and all monosaccharide, disaccharide, polysaccharide, amino-sugar or hydroxyl carbon acid, it's derivatives, salts and/or residues remaining after the removal of a hydrogen atom from it. The following are suitable examples of suitable monosaccharides; pentoses such as arabinose, ribose and xylose as well as hexoses such as glucose, mannose, galactose and fructose. Suitable amino sugars include e.g. glucosamin and galactosamin. A suitable carbon acid for example is glucronic acid. The hydroxyl carbon acids' hydroxyl groups can be free, partially derivatized or fully derivatized (protective groups) specific examples of amino sugar silicon compounds are listed herein.

The term "solubilization" involves the breaking of inter-ionic or intermolecular bonds in the solute, interactions between the solvent and the solute molecule or ion. According to this invention, all techniques of solubility enhancement will be used such as physical modifications such as particle size reduction, micronization, nanosuspension, modification of the crystal habit, polymorphs, pseudopolymorphism, drug dispersion in carriers, eutectic mixtures, solid dispersions, solid solutions, complexation, use of complexing agents, use of solubilizers, solubilization by surfactants, microemulsions, self microemulsifying drug delivery systems and any chemical modifications.

A "subject" for the purposes of the present invention includes both humans and other animals, particularly mammals, and other organisms. Thus the methods are applicable to both human therapy, vaccinations, and veterinary applications. In the preferred embodiment the patient is a mammal, the most preferred being a human.

The term "animal" refers to an organism with a closed circulatory system of blood vessels and includes birds, mammals and crocodiles. The term "animal" used here also includes human subjects.

An "immunologically effective amount" is the quantity of a compound, composition or carrier system of the present invention which is effective in yielding the desired immunologic response.

The terms "treating cancer," "therapy," and the like refer generally to any improvement in the mammal having the cancer wherein the improvement can be ascribed to treatment with the compounds of the present invention. The improvement can be either subjective or objective. For example, if the mammal is human, the patient may note improved vigour or vitality or decreased pain as subjective symptoms of improvement or response to therapy. Alternatively, the clinician may notice a decrease in tumour size or tumour burden based on physical exam, laboratory parameters, tumour markers or radiographic findings. Some laboratory obtained results which the clinician may observe to check for any response to therapy include normalization of tests such as white blood cell count, red blood cell count, platelet count, erythrocyte sedimentation rate, and various enzyme levels. Additionally, the clinician may observe a decrease in a detectable tumour marker(s). Alternatively, other tests can be used to evaluate objective improvement such as sonograms, nuclear magnetic resonance testing and positron emissions testing.

"Inhibiting the growth of tumour cells" can be evaluated by any accepted method of measuring whether growth of the tumour cells has been slowed or diminished. This includes direct observation and indirect evaluation such as subjective symptoms or objective signs as discussed above.

Accordingly, the carrier systems of the invention are administered to cells, tissues, healthy volunteers and subjects and/or patients. Herein what is meant by "administered" is the administration of a therapeutically effective dose of the candidate agents of the invention to a cell either in cell culture or in a patient. Herein what is meant by "therapeutically effective dose" is a dose that produces the effects for which it is administered. The exact dose will depend on the purpose of the treatment, and will be ascertainable by one skilled in the art using known techniques. As is known in the art, adjustments for systemic versus localized delivery, age, body weight, general health, sex, diet, time of administration, drug interaction and the severity of the condition may be necessary, and will be ascertainable with routine experimentation by those skilled in the art. Herein what is meant by "cells" is almost any cell in which mitosis or miosis can be altered.

Additional "carriers" may be used in the "carrier system" of the present invention, and include any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the carrier systems.

The phrase "pharmaceutically-acceptable" refers to molecular entities and compositions that do not produce an allergic or similar untoward reaction when administered to a human. The preparation of an aqueous composition that contains a protein as an active ingredient is well understood in the art. Typically, such compositions are prepared as injectables, either as liquid solutions or suspensions; solid forms suitable for solution in, or suspension in, liquid prior to injection can also be prepared. The preparation can also be emulsified.

The term "pharmaceutically acceptable salt" refers to those salts of compounds which retain the biological effectiveness and properties of the free bases and which are obtained by reaction with inorganic acids such as hydrochloric acid, hydrobromic acid, sulphuric acid, nitric acid, phosphoric acid, methanesulphonic acid, ethanesulphonic acid, toluenesulphonic acid, salicylic acid and the like. Pharmaceutically acceptable salts include alkali metal salts, such as sodium and potassium, alkaline earth salts and ammonium salts.

Therefore, as used herein, "cancer" refers to all types of cancer or neoplasm or malignant tumours found in mammals, including carcinomas and sarcomas. Examples of cancers are cancer of the brain, breast, cervix, colon, head & neck, kidney, lung, non-small cell lung, melanoma, mesothelioma, ovary, sarcoma, stomach, uterus and Medulloblastoma.

The present invention relates to the use of organosilicon, sugar organosilicon, amino sugar organosilicon compounds, their derivatives, salts and/or the vesicles formed from them in a carrier system with antigens, pre-antigens, antigen conjugates, antibodies, antibody conjugates, allergens, allergen extracts, nucleic acids, plasmids, proteins, peptides, pharmaceutical agents, immunologically active substances and/or cosmetics, for the manufacturing of a pharmaceutical, immunological and/or cosmetic composition for the different indications.

The pharmaceutical composition of the invention optionally comprises of one or more pharmaceutically acceptable adjuvants, excipients, carriers, buffers, diluents and/or customary pharmaceutical auxiliary substances. The composition of the invention is administered in a pharmaceutically acceptable formulation. The present invention pertains to any pharmaceutically acceptable formulations, such as synthetic or natural polymers in the form of macromolecular complexes, nanocapsules, microspheres, or beads, and lipid-based formulations including oil-in-water emulsions, micelles, mixed micelles, synthetic membrane vesicles, and resealed erythrocytes. In addition to the said composition and the pharmaceutically acceptable polymer, the pharmaceutically acceptable formulation of the invention can comprise additional pharmaceutically acceptable carriers and/or excipients. As used herein, a pharmaceutically acceptable carrier includes any and all solvents, dispersion media, coatings, antibacterial and anti fungal agents, isotonic and absorption delaying agents, and the like that are physiologically compatible. For example, the carrier can be suitable for injection into the cell, tissues, organs and/or blood. Excipients include pharmaceutically acceptable stabilizers and disintegrants. In another embodiment, the pharmaceutically acceptable formulations comprise lipid-based formulations. Any of the known lipid-based drug delivery systems can be used in the practice of the invention. For instance, multivesicular liposomes (MVL), multilamellar liposomes (also known as multilamellar vesicles or MLV), unilamellar liposomes, including small unilamellar liposomes (also known as unilamellar vesicles or SUV), large unilamellar liposomes (also known as large unilamellar vesicles or LUV), multivesicular siosomes (MVS), multilamellar siosomes (MLS), unilamellar siosomes including small unilamellar siosomes can all be used so long as a sustained release rate of the carrier system composition of the invention can be established.

### pH-sensitive silane lipid:

pH-sensitive silane lipids are additionally encompassed by the present invention, for example for the uptake and subsequent triggered release of enclosed active substance, whereby the siosome releases the active substance at certain pH conditions. The siosomes may include pH-sensitive lipids, which is a silane-lipid that forms bilayer vesicles in the absence of stabilising components only at specific pH ranges. These lipids are typically amphipathic lipids having hydrophobic and polar head group molecules, and when arranged into a bilayer are oriented such that the hydrophobic moiety is in contact with the interior, hydrophobic region of the bilayer membrane, and the polar head group moiety is oriented toward the exterior, polar surface of the membrane.

The pH-sensitive amphipathic lipids preferably have two hydrocarbon chains, typically acyl chains between about 8-22 carbon atoms in length, and have none or varying degrees of unsaturation.

### Polymer-derivatised silane-lipid component:

Polymer-derivatised silane-lipid component are also encompassed by the present invention. The siosomes also include a lipid derivatised with a hydrophilic polymer. The polymer derivatised lipids in the siosomes serve to stabilise the pH-sensitive silane-lipid to facilitate bilayer and siosome formation and to form a coating of polymer chains over the siosome surface to extend the blood circulation lifetime of the siosomes.

The hydrophilic polymer coating provides colloidal stability and serves to protect the siosomes from uptake by the mononuclear phagocyte system, providing a longer blood circulation lifetime for the siosomes to distribute in the organism. The polymer chains are attached to the lipid of the silane by a releasable bond for cleavage and release of the polymer chains in order to restore the pH-sensitivity of the siosomes.

Preferably, the derivatisable silane-lipid is a non-pH-sensitive siosome-forming amphipathic silane which can spontaneously form into a bilayer vesicle in water.

Hydrophilic polymers suitable for derivatising the amphipathic lipids, cationic and/or anionic organosilicon compounds include polyethyleneglycol, polyvinylpyrrolidone, polyvinylmethylether, polymethyloxazoline, polyethyloxazoline, polyhydroxypropyloxazoline, polyhydroxypropylene, methacrylamide, polymethacrylamide, polydimethylacrylamide, polyhydroxypropylmethacrylate, polyhydroxyethylacrylate, hydroxymethylcellulose, hydroxyethylcellulose, and polyaspartamide and/or their derivatives .

In a preferred embodiment, the hydrophilic polymer is polyethyleneglycol (PEG), preferably as a PEG chain having a molecular weight between 200 and 15,000 Dalton, more preferably between 500 and 5,000 Dalton.

### Siosome-entrapped active substance:

Entrapped in the siosomes is an active substance for intracellular delivery to the target cells. A variety of active substances can be entrapped in siosome vesicles, including water soluble agents, for example small water soluble organic compounds, peptides, proteins, DNA plasmids, oligonucleotides, and gene fragments, which can be stably encapsulated in the aqueous compartment of the siosomes. Furthermore, lipophilic compounds that stably partition in the lipid phase of the siosomes, or agents that can be stably attached, for example by electrostatic attachment to the outer siosome surfaces, are also intended.

The siosome-entrapped compound can also be an imaging agent for tracking progression of a disease. Imaging agents include chelates of radionuclides, such as technetium-99, indium-III, and iodine-125. The entrapped agent may also be a reporter molecule, such as an enzyme or a fluorophore, for use in in-vitro diagnostic assays.

The term "lipid" refers to any suitable material resulting in a bilayer such that a hydrophobic portion of the lipid material orients toward the bilayer, while a hydrophilic portion orients towards the aqueous phase.

The term "neutral lipid" refers to any of a number of lipid species which exist either in an uncharged or neutral zwitterionic form at physiological pH. Such lipids include, for example, diacylphophatidylcholine, diacylphosphatidylethanolamine, ceramide, sphingomyelin, cephalin and cerebrosides. The term "non-cationic lipid" refers to any neutral lipid as described above, as well as anionic lipids.

The term "charged lipid" refers to any of a number of lipid species which exist in a charged form at physiological pH. Examples of charged lipids are cationic lipids or anionic lipids.

The term "anionic lipid" refers to any of a number of lipid species which carry a net negative charge at physiological pH. Examples of anionic lipids include cardiolipin, diacylphosphatidylserine and diacylphosphatidic acid, phosphatidylglycerol and cholesterylhemisuccinate.

The term "cationic lipid" refers to any of a number of lipid species which carry a net positive charge at physiological pH. Such lipids include, but are not limited to, DODAC, DOTMA, DDAB, DOTAP, DC-Chol and DMRIE.

Additionally, a number of commercial preparations of cationic lipids are available, which can be used in the present invention. These include, for example, LIPOFECTIN®, LIPOFECTAMINE® and TRANSFECTAM®.

### Active substances:

Many highly active and useful pharmaceutical agents suffer from suboptimal pharmacokinetics and/or biodistribution. Consequently, the therapeutic use of these pharmaceutical agents can be limited. Siosomes formulations of the invention can be used to improve the efficacy and toxicity profiles, and to improve the dosing schedule of the drug by modification of the pharmacokinetic and biodistribution properties. As used herein, the term active substance includes diagnostic agents.

The term active substance includes, but is not limited to, an analgesic, an anaesthetic, an anti-acne, an antibiotic, an antibacterial, an anticancer, an anticholinergic, an anticoagulant, an antidyskinetic, an antiemetic, an antifibrotic, an antifungal, an antiglaucoma agent, an anti-inflammatory, an antineoplastic, an antiosteoporotic, an anti-Parkinson's agent, an anti-sporatic, an antipyretic, an antithrombic, an anti-viral, a calcium regulator, a keratolytic, or a sclerosing agent.

### Anti-cancer active substances:

Doxorubicin hydrochloride is a drug used in cancer chemotherapy. It is an anthracycline antibiotic, closely related to the natural product daunomycin, and like all anthracyclines it works by DNA intercalation.

It is commonly used in the treatment of a wide range of cancers, including haematological malignancies, many types of carcinoma, and soft tissue sarcomas.

Doxorubicin hydrochloride has been used alone or in combination chemotherapy for the treatment of AIDS-related Kaposi's sarcoma and combination chemotherapy that includes the drug (doxorubicin, bleomycin, and vincristine) has been a preferred regimen, although many clinicians currently consider a liposomal anthracycline (doxorubicin or daunorubicin) the first-line therapy of choice for advanced AIDS-related Kaposi's sarcoma.

The term anti-cancer substance and/or agent includes, but is not limited to, potent chemoactive substances such as cisplatin, carboplatin, oxaliplatin, bleomycin, epirubicin, mitomycin, and/or monoclonal antibodies, such as alemtuzumab, beracizumab, cetuximab and/or topoisomerase-l-inhibitors such as irinotecan, topotecan , antimetabolites such as gemicitabin and cytarabin, proteasom-inhibitors and vinca alkaoids.

### DESCRIPTION OF THE FIGURES

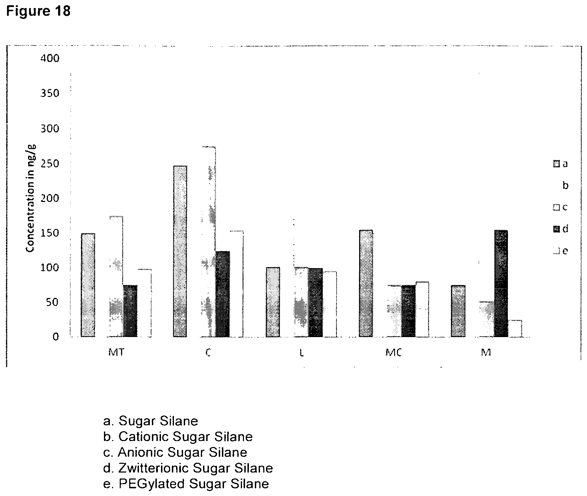

Figure 1: General formula for the silanes according to the invention:
Figure 2: Cationic sugar silanes

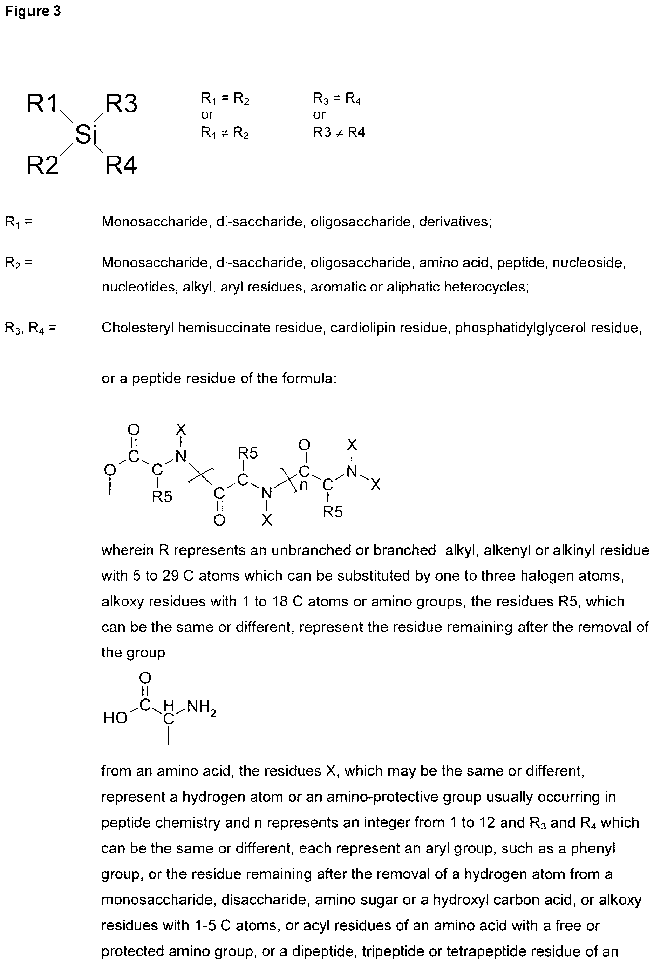

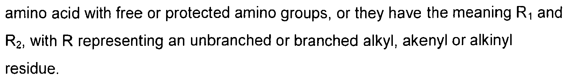

Figure 3: Anionic sugar silanes
Figure 4: PEGylated sugar silanes
Figure 5: Use of multi-lamella siosome vesicles which reach the site of action with at least one intact vesicle layer and still contain the free drug polymer complex
Figure 6: Delivery of siRNA-siosome-active substance to cell using cationic siosomes
Figure 7: Delivery of siRNA-siosome-active substance to the using anionic siosomes

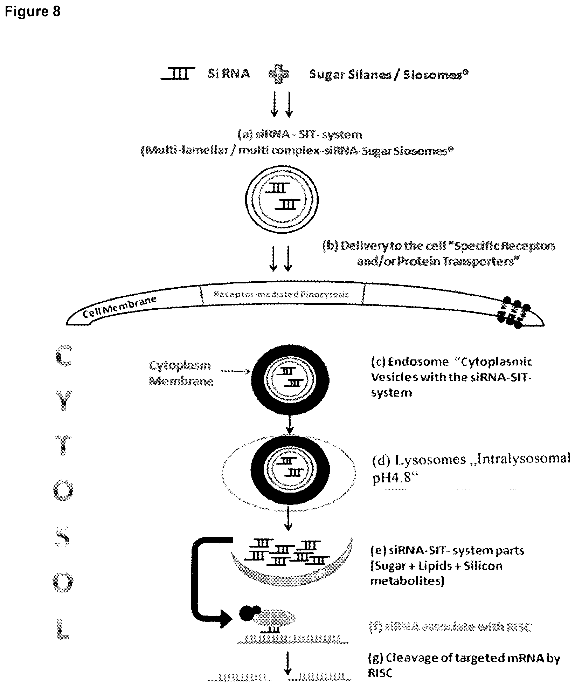

Figure 8: The following figure describes the intracellular delivery of active substances such as siRNA using the Siosomes Intracellular Targeting System (SIT-System). a) Once administered, the siRNA-SIT-System will have sugar interactions with the cell surface to enter the cell via receptor mediated endocytosis (b). The cytoplasm membrane folds inward to form with the siRNA-SIT-System coated pits (Endosome). This will then be carried into the cell cytosol to be fused with the lysosome (c). The lysosome then digests the siRNA-SIT-System at a pH of approximately pH 4.8 (d) to release the siRNA into the cell cytosol (e). The free target siRNA in the cytosol will form the RNAi-induced silencing complex (RISC) (f) to silence the target gene by cleavage of mRNA.
Figure 9: Development of diameter in nm of SIL 17
Figure 10: Development of counts of SIL 17
Figure 11: Development of diameter in nm of SIL 2
Figure 12: Total platinum concentration in blood with molar adjustment for Cis-Oxoplatin Siosome Complex - intravenous
Figure 13: Total platinum concentration in the spleen with molar adjustment for Cis-Oxoplatin Siosome Complex - intravenous
Figure 14: Total platinum concentration in the stomach with molar adjustment for Cis-Oxoplatin Siosome Complex - intravenous
Figure 15: Total platinum concentration in the lung with molar adjustment for Cis-Oxoplatin Siosome Complex - intravenous
Figure 16: Total platinum concentration in the kidneys with molar adjustment for Cis-Oxoplatin Siosome Complex - intravenous
Figure 17:
   a) Total platinum concentration in the liver - sugar silane;
   b) Total platinum concentration in the liver - cationic sugar silane;
   c) Total platinum concentration in the liver- anionic sugar silane;
   d) Total platinum concentration in the liver - zwitterionic sugar silane;
   e) Total platinum concentration in the liver - PEGylated sugar silane
Figure 18: Total platinum concentration in the liver with sugar silane, cationic sugar silane, anionic sugar silane, zwitterionic sugar silane, PEGylated sugar silane

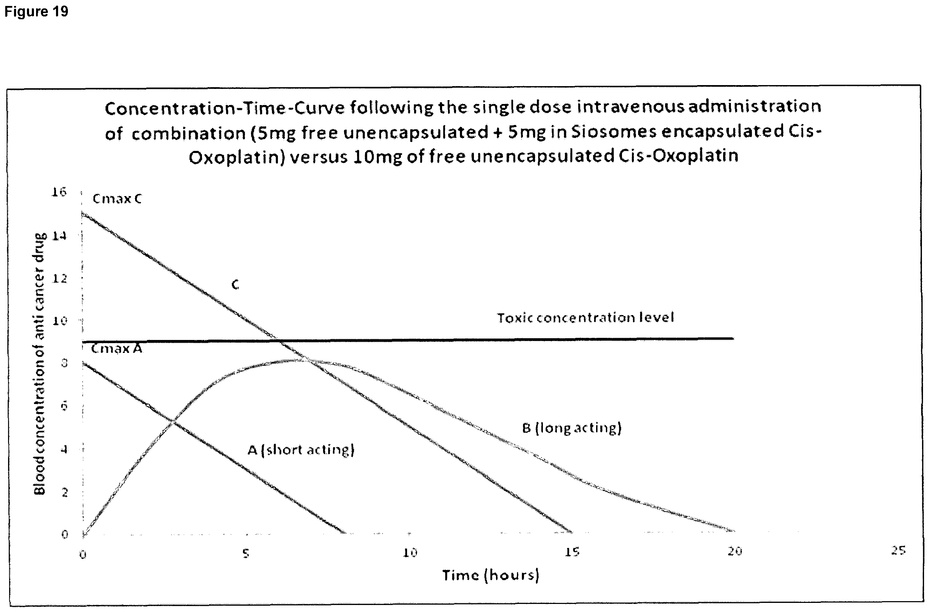

Figure 19: Concentration-Time-Curve following the single dose intravenous administration of combination (5 mg free unencapsulated + 5 mg in Siosomes encapsulated Cis-Oxoplatin) versus 10 mg of free unencapsulated Cis-Oxoplatin.
   A = Free anti cancer drug in the Siosomes® combination (short acting)
   B = Encapsulated anti cancer drug in the Siosomes® combination (long acting)
      A+B = Combination of short and long acting formulation - the total is equivalent to the "therapeutic dose" of the Siosomes®-Chemostatic-Complex (SCC), which may be/will most probably be different that the "therapeutic dose" of standard parenteral chemostatic formulation with free unencapsulated chemostatic
   C = Standard parenteral anti cancer agent (short acting infusion)

### EXAMPLES

The siosome containing preparations to be used in the method of the invention are applied in therapeutically acceptable amounts to produce therapeutically effective levels in the target. Such preparations for use may routinely contain known compatible carriers and other additives, such as buffering agents and preservatives, and optionally other active substances. Aqueous siosomal preparations may be formulated according to known methods, using suitable additives, such as dispersing or wetting agents and suspending agents. A sterile injectable preparation may be a sterile injectable solution or suspension in a non-toxic acceptable diluent or solvent, such as water, isotonic sodium chloride solution and buffer. In addition, sterile oils are often employed as a solvent or suspending medium.

The particular mode of administration selected will depend upon the particular selected active substance, the severity of the condition being treated and the dosage required for therapeutic efficacy. Such modes of administration include oral, rectal, intravaginal, topical, nasal, interdermal or parenteral routes, the term perenteral including subcutaneous, intravenous, intramuscular, intratumoural and infusion.

The carrier system according to the invention would preferably selectively release anti-cancer chemotherapeutics and any other active substances at intracellular targets at low pH-regions.

The examples are in no way intended to limit the scope of the invention.

Unless otherwise defined, all technical and scientific terms used herein have the same meaning as commonly understood by a person of ordinary skill in the art to which this invention belongs. Although methods and materials similar or equivalent to those described herein can be used in the practice or testing of the present invention, suitable methods and materials are described below.

### Determining efficiency of carrier system protein complex formation with the organosilicon, sugar organosilicon, amino sugar organosilicon compounds and/or blank (unloaded) vesicles thereof

Carrier system formation efficiency, or protein complex formation between the organosilicon, sugar organosilicon, and/or amino sugar organosilicon compounds with biological protein agents, was calculated after centrifugation by quantification of the amount of the protein in the protein-complex fraction and the free non-complexed proteins presents in the aqueous supernatant phase. The protein was quantified using a modified Lowry method, Peterson G.L. 1983 "Determination of total protein", Methods Enzymology 91: 95-119.

### In-vivo protocol for the immunogenicity tests

Adult, pathogen-free New Zealand white rabbits were used in the immunogenicity tests. The rabbits were grouped (n=4) and immunized with the antigen and/or antigen mixture. Two sets of injections per rabbit on day 0 and 14. Intramuscular (i.m) or subcutaneous injections (s.c) were given in the hind legs following a standard protocol, with the first injection set given in the right leg, the second injection set given in the left leg. The injection sites were labelled for later identification. To assess immunogenicity, sera were prepared from blood samples obtained from each rabbit on day 0 (control) and day 19 (5days after the second injection). Blood (15ml per bleed) was collected from marginal ears veins using an 18 gauge needle, and then stored at 2-8 °C overnight to allow for clot shrinkage. The samples were then centrifuged (400x g) and the sera were removed by pipette and frozen as individual samples at -10°C to -25°C until assayed. The suspensions were injected with 1.0ml dose volumes.

### Homogenisation of the carrier systems

High pressure homogenisation using micro fluidiser was used for the preparation of the complex combinations, pressure range: 170-2700 bar.

### Lyophilisation/Dehydration of the carrier systems

The appropriate volume of suspension was added into each vial in 2 ml increments with vigorous vortexing between additions. Lyophilisation took place using freeze drying technology controlling all aspects of the lyophilisation cycle. The 1 shelf temperature ranges from -70°C to 60°C, process condenser temperature as low as -85°C and vacuum indication of 760 torr to 1 militorr. The dehydration of the carrier system without the use of a protective sugar depends on the organosilicon, sugar organosilicon and/or amino sugar organosilicon compound concentrations.

Dehydrated carrier system complexes are prepared by drying the preparations under reduced pressure in the presence of one or more protective sugars, e.g. disaccharides such as trehalose and sucrose. Sugar organosilicon or amino sugar organosilicon compounds could be used as protective compounds for dehydration and/or lyophilisation of the carrier systems.

The dehydration was performed to an end point which results in sufficient water being left in the preparation (e.g. at least 4-10 moles water/ silicon-lipid) so that the integrity of a substantial portion of the silicon-lipid combinations is retained upon rehydration.

### Hydration of the lyophilised carrier systems

Hydration is the final step in the preparation of the formulation for injection and/or other pharmaceutical formulations (for oral, nasal and/or topical administration). Typically, hydration solution is added in a series of aliquots to lyophilised carrier systems with vortex mixing after each addition of hydration media. Generally, water for injection (WFI) is used. Here, we also tested WFI that was supplemented with 5% ethanol (EtOH), which has the added advantage of enhancing hydration of the carrier systems.

### Extrusion of the carrier system suspensions

Virtually all organosilicon, sugar organosilicon and/or amino sugar organosilicon compound complexes with proteins, alleregens, and biological mixtures can be rapidly extruded resulting in a homogenous formulation of the preparations. Avestin LiposoFast and Northern Lipids Extruders (Thermobarrel Extruder) equipment attached to a nitrogen gas line was used.

### Preparation of dispersions

A dispersion is a homogenous mixture of substances that are not soluble in each other. Dispersion can be achieved by sonication, extrusion, high pressure homogenisation, shaking, freezing and thawing.

### Preparation of Organosilicons

Examples 1-12 describe the synthesis of a number of the organosilicons. This procedure was employed to prepare a number of the organosilicons of interest.

### Preparation of amino sugar organosilicon compound

Example 12 describes the procedures used for the preparation of an example of the sugar organosilicons. Modified and/or different procedures have also been used for the synthesis of the other sugar organosilicon compounds.

### Preparation of siosomes and sugar siosomes

Example 13 describes the procedure used for the preparation of both blank (unloaded) siosomes and/or sugar-siosomes. The blank (unloaded) siosomes have water encapsulated inside the Siosomes and between the lipid layers.

All of the carrier systems and methods disclosed and claimed herein can be made and executed without undue experimentation in light of the present disclosure. While the carrier systems and methods of this invention have been described in terms of preferred embodiments, it will be apparent to those of skill in the art that variations may be applied to the carrier systems and methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

### Example 1: Preparation of Di(decanoyloxy)dimethylsilane (Sil 21.1)

0.012 mol dimethyldichlorosilane is added to 50ml anhydrous ether, to which is further added 0.02 mol sodium decanoate under agitation at 40°C. To increase the yield, an excess of dimethyl dichlorosilane is added. This is followed by approximately another 3 hours of agitation at 40°C. For hydrolysis of the excess dimethyldichlorosilane, water is added and approximately 10 ml ether is applied for extraction 3 to 5 times. The combined ether extracts are dried over anhydrous sodium sulphate and, after filtering off, evaporated in a vacuum. The remaining di(decanoyloxy)dimethylsilane is recrystalised from heptane.

| | |
|---|---|
| Molecular formula: | C₂₂H₄₄O₄Si |
| Molecular mass: | 400.68 gmol⁻¹ |
| Melting point: | 32-33°C |
| Yield | 92% |

### Example 2: Preparation of Di(octadecanoloxy)dimethylsilane (Sil 22.1)

0.012 mol dimethyldichlorosilane is added to 50ml anhydrous ether, to which 0.02mol sodium octanoate is further added, under agitation at 40°C. This is followed by approximately 3 hours of further agitation at the same temperature. Disintegration of the excess dimethyldichlorosilane in water is carried out as per example 1. Recrystallisation from heptane is then performed.

| | |
|---|---|
| Molecular formula: | C₃₈H₇₆O₄Si |
| Molecular mass: | 625.09 gmol⁻¹ |
| Melting point: | 62-64°C |
| Yield | 68% |

### Example 3: Preparation of Di(octanoloxy)diphenylsilane (Sil 23.01)

0.01 mol dichlorophenyl saline is added to 50ml anhydrous ether, to which 0.02mol sodium octanoate is further added under agitation at 40°C. This is followed by approximately another 5 hours of agitation, after which water is added and approximately 10ml of ether is applied for extraction three times. The combined extracts are dried over sodium sulphate and evaporated in a vacuum.

| | |
|---|---|
| Molecular formula: | C₂₈H₄₀O₄Si |
| Molecular mass: | 468.7 gmol⁻¹ |
| Melting point | 88-90°C |
| Yield | 88% |

### Example 4: Synthesis of Sil 2 (2-(Dimethyldecylsilyl)ethyl-beta-D-gluopyranosid

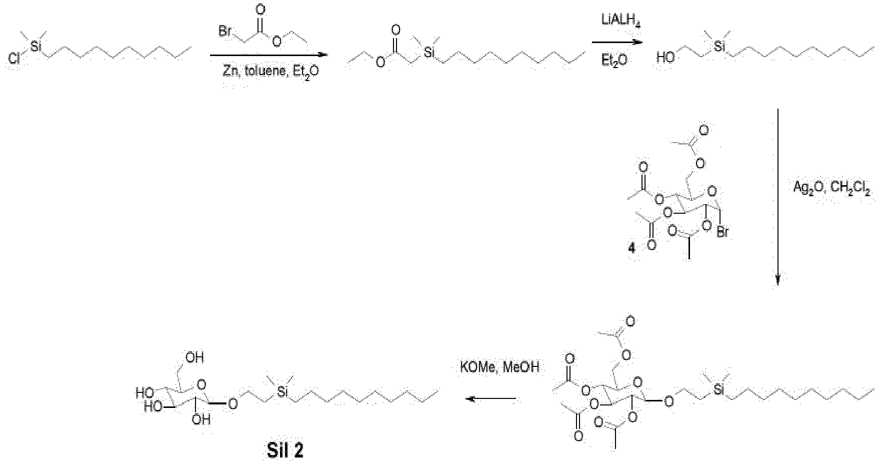

### Example 5: Synthesis of SIL 8 (1-O-(Decyldimethylsilyl)-2,3,4,6-tetra-O-acetyl-beta-galactose)

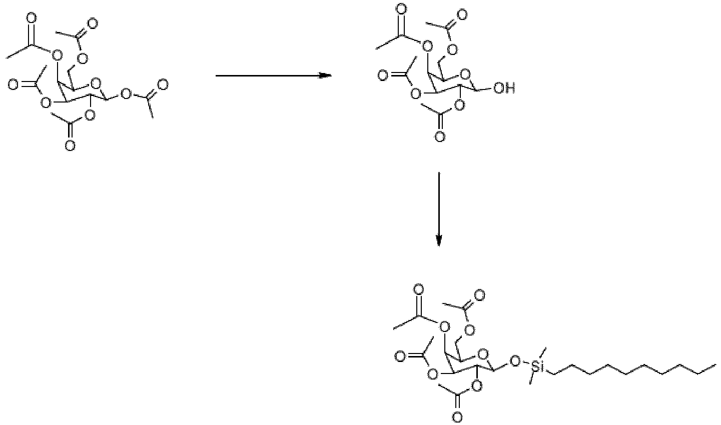

### Example 6: Synthesis of SIL 12 (1-O-Diocytlsilyl-di(2,3,4,6-O-tetraacetyl-beta-D-glucopyranoside)

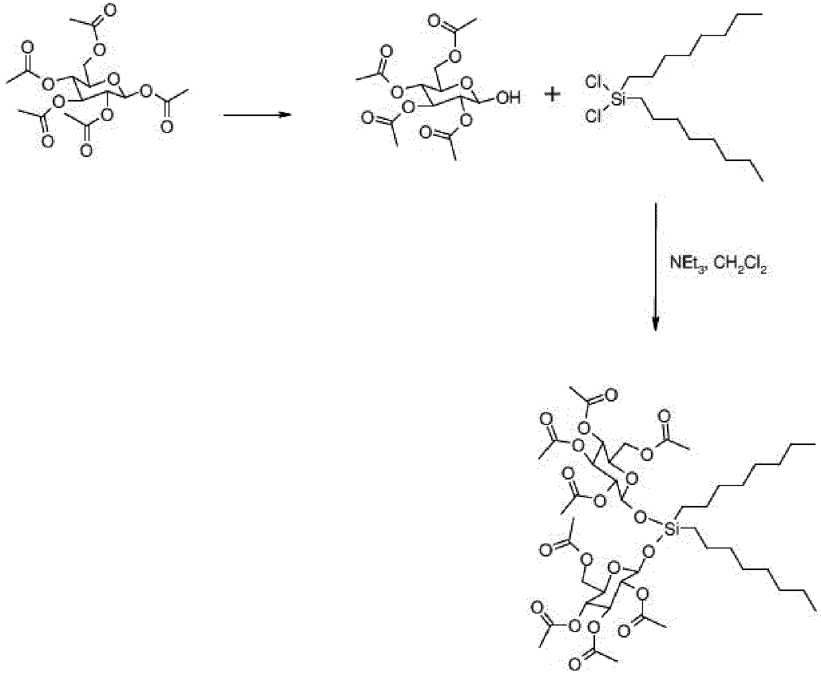

### Example 7: Synthesis of SIL 14 (1-O-Dioctadecylsilyl-di(2,3,4,6-O-tetraacetyl-beta-D-glucopyranoside)

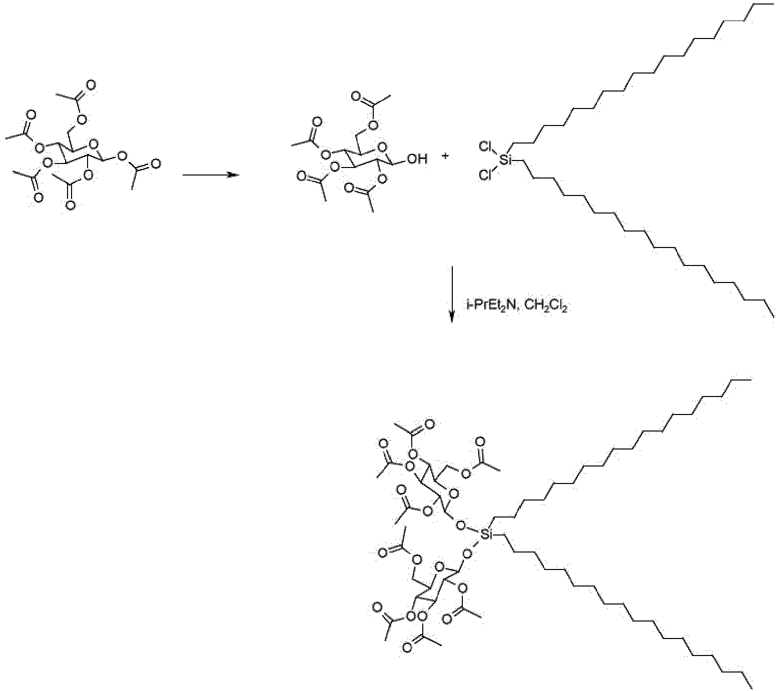

### Example 8: Synthesis of SIL 17 (1-O-Didodecylsilyl-di(2,3,4,6-O-tetraacetyl-beta-D-glucopyranoside)

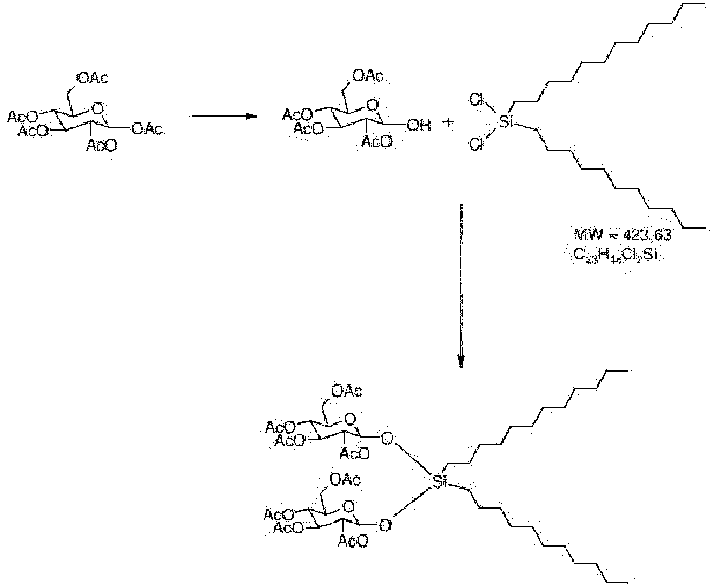

### Example 9: Synthesis of SIL 28 (2-(Dimethyloctadecylsilyl)ethyl-2,3,4,6-O-tetraacetyl-beta-D-glucopyranosid

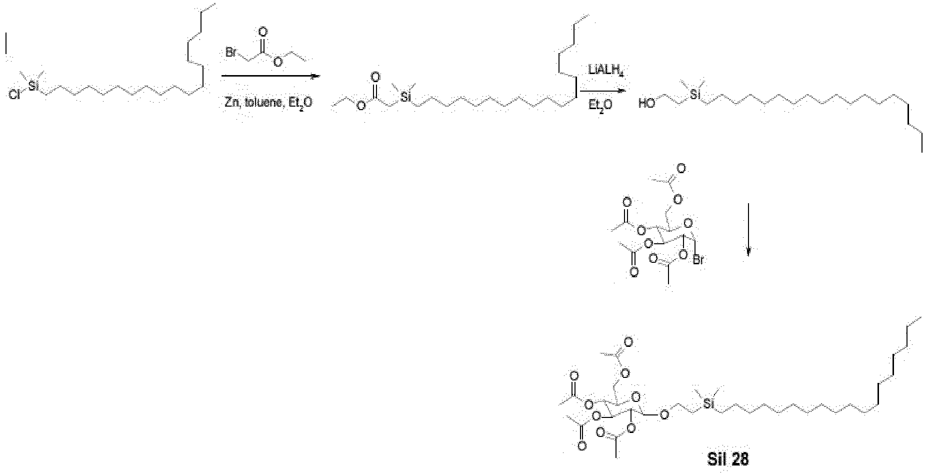

### Example 10: Synthesis of Dioctyldivinylsilane (SIL 47)

A dry flask was charged with magnesium (4.63 g, 191 mmol, 3.3 eq), dry THF (10 mL) and a crystal of iodine. A small portion of a solution of vinylbromide (1.0 M in THF) was injected into the magnesium and this was heated to reflux to start the Grignard reaction. When the reaction started, the rest of the vinylbromide solution (total: 173 mL, 173 mmol, 3.0 eq) was added over 20 minutes while the reaction mixture boils by itself. Upon complete addition the reaction mixture was heated to reflux for 100 minutes and subsequently dichloro-di-n-octyl silane (20 mL, 57.8 mmol, 1.0 eq) was added over 5 minutes to the boiling mixture upon which the boiling became more vigorous. The reaction mixture was boiled for another 4 hours and then allowed to cool to room temperature. The mixture was carefully quenched with 1 M HCl (25 mL) and the aqueous layer was subsequently extracted with TMBE (2 x 25 mL). The combined organic layers were washed with brine (20 mL), dried (Na₂SO₄) and concentrated to dryness. The resulting oil was purified by column chromatography (SiO₂, 100% heptanes, Rf product: 0.91). This gave Dioctyldivinylsilane as a colorless oil (16.3 g, 95% yield) in 97.3% purity (GC-MS).

### Example 11: Synthesis of 2,2'-(Dioctylsilanediyl)diethanol (SIL 48)

To a solution of 9-BBN in THF (0.5 M, 305 mL) was added dropwise dioctyldivinylsilane (15.43 g, 50 mmol). The reaction mixture was stirred for 1.75 h. The reaction mixture was cooled to room temperature. Sodium hydroxide (aq., 3 M, 51 mL) was added. Hydrogen peroxide (aq., 30 w/w%, 51 mL) was added dropwise (CAUTION! Exotherm and gas formation). The reaction mixture was heated under reflux for 1.5 h. The reaction mixture was cooled to room temperature and brine (66 mL) was added. The organic phase was dried over K₂CO₃ (s) over the weekend. The solution was filtered and treated with saturated Na₂S₂O₅ to quench the remaining H₂O₂. The water layer was separated, the organic layer was dried over Na₂SO₄ and concentrated to dryness. The mixture was purified over silica (1 kg) applying ethyl acetate/heptanes (1/1, v/v) as eluent. This yielded 12.3 g of 2,2'-(Dioctylsilanediyl)diethanol (72%).

### Example 12: Preparation of amino sugar organosilicon compound

Synthesis of tetradecyl-(2,3,4,5-tetra-O-acetyl-β-D-gluco-pyranosyloxy)silanes. There exist various possibilities for the formation of glucosidic bonds to the anomeric C-atom of the sugar, as described in the literature. To the most important variants belong to the Königs-Knorr reaction, the trichlorine-acetamidate-method and the TMSOTf method. All known methods are essentially S_{N}2-reactions, and through the right selection of the protective groups at the carbohydrate part and observance of suitable conditions of reaction, we obtained 1, 2-trans-tied-glucosides. A further requirement is that the anomeric C atom must be activated through a leaving group which determines the centre of the reaction. The aglycon must have one free hydroxyl group if necessary selectively non-blocked.

### Example 13: Preparation of siosomes

10µmol di(decanoyloxy)dimethylsilane is dissolved in 2ml ethanol, and small doses are gradually added over a period of 4 hours to an aqueous phase (volume 2ml) which can contain the compounds to be encapsulated and has been brought to a temperature of 80°C. Depending on the solubility of the compound to be encapsulated, it can also be a component of the organic phase. The material is the injected into pure water. In this case the agitation rate is 2000±200rpm. The encapsulated compounds are separated from the non-encapsulated compounds by dialysis, gel filtration or centrifuging, for example and the contents of the siosomes determined. The siosomes prepared in this manner had a uniform size of 300 to 500nm and are evidently all uni-lamellar. The siosomes stability was good.

The higher the number of side chain (from C=8 to C=18), the larger the siosomes become (200nm to 2µm). While in the case of di(octanoyloxy)dimethylsilane and di(decanoyloxy)dimethylsilane, nearly all the siosomes produced are unilamellar and of uniform size, other siosomes prepared according to the invention (with the number of carbon atoms in side chain C=12 to C= 18) have predominantly multilamellar structures. These siosomes have a size of up to 2µm.

### Example 14: Preparation of a carrier system complex of amino sugar organosilicon compound with insulin.

A dispersion of 10µmol of Didodecylsilyl-di(2,3,4,6-0-tetraacetyl-β-D-glucopyranosid) as the representative of amino sugar organosilicon compound, 0.01 M Tris/HCl, pH 7.4 and an aqueous solution of insulin (10µmol, volume 3ml) as antigen was prepared by mixing with a high pressure homogeniser. The homogenisation time may vary. The mixture was then incubated at 37°C for 30 minutes and sterile filtrated and lyophilised at the temperature of -70°C. The lyophilised insulin-amino sugar organosilicon complex was reconstituted by hydration in sterile (deionized) water and the carrier system solution was used for in-vitro investigations.

After reconstitution by rehydration, the said carrier system surprisingly retains in solution more than 95% of the biological activity of insulin. The yield of the preparation process resulted from 3 independent experiments, and was 80-90% of the starting concentrations. Stability tests using radioimmunoassay performed at 0, 4, 8, 12 and 48 hours at 4°C, 25 °C and 37°C have shown that the carrier system complex of organosilicon-insulin is surprisingly stable and the insulin retains its biological activity.

### Example 15: Preparation of a carrier system comprising blank (unloaded) siosomes with antigens

A dispersion of lyophilised blank sugar-siosomes prepared from 2-(dimethyldodecosilyl)-2,3,4,6-tetra-o-acetyl-β-D-glucopyranosid and 0.01 M Tris/HCl, pH7.4 and an aqueous solution of the antigen mixture comprising of Cathepsin B (EC 3.4.22.1), MW 30kDa as Thiolproteinease and Cathepsin C (EC 3.4.14.1), MW 200kDa as multimeric, dipeptidyl peptidase 1 enzyme was prepared at 37°C by vortexing and/or high pressure homogenisation.

Directly afterward, the said dispersion was heated for 20 minutes at 25°C (until complete fluidisation of the blank siosome). The mixture was then lyophilised and stored at 4-8°C. The biological activities of Cathepsin B and C in the carrier system complex were determined after reconstitution of the lyophilised samples by hydration with sterile (deionized) water using Cathepsin Activity Assay Kit. Surprisingly, the carrier system retains more than 95% of the biologically active principles in solution. The carrier system showed long-termed stability as a lyophilisate at 4-8°C. The use of simple procedures according to this invention showed unexpected results in creating carrier systems, with the antigens Cathepsin B and C as examples for proteins, with lyophilised blank (unloaded) sugar-siosomes, demonstrates the potential for scale-up preparation of larger amounts.

### Example 16: Preparation of a carrier system comprising doxorubicin-sugar-siosomes-complex

A doxorubicin hydrochloride solution at 10 mg/ml was prepared using physiological saline. The predetermined amount of the doxorubicin hydrochloride solution was added to the sugar siosome dispersion using the cationic organosilicon compounds such as 1.2-(Dimethyldecylsilyl)ethyl-beta-D-glucosamin, 2.2-(Dimethyloctylsilylcholin)ethyl-beta-D-glucopyranosid and/or 1.2-(DiN,N,N,trimethylammoniadecylsilyl)ethyl-beta-D-glucopyranosid and the mixture was adjusted to pH 7.4, using 1 N NaOH or saturated sodium hydrogencarbonate and the mixture was stirred at 65°C for 30 minutes the thereby introduce the doxorubicin hydrochloride in the siosomes.

After doxorubicin hydrochloride introduction, the sample was cooled in an ice bath. Gel filtration was conducted using a column (Sepharose 4, fast flow, having a diameter of 2.8 cm and a length of 20 cm, fully substituted with 10% sucrose (pH 6.5)) to remove the doxorubicin hydrochloride that had not been introduced in the siosomes.

The amount of the sugar silanes in the siosomes, the amounts of the doxorubicin hydrochloride encapsulated in the siosomes and the siosomes themselves were measured by the same methods as in the above examples.

Concentration of the doxorubicin hydrochloride encapsulated in the siosomes was determined by measuring absorbance at 480 nm, using a spectrophotometer for the solution prepared by adding 2 ml of methanol to 40 µl of the doxorubicin siosomes. An alternative HPLC-based assay has also been used for the quantification of the doxorubicin in the formulations.

The siosome size was measured using Zetasizer (Malvern Instruments).

**Table 5:**

| **Cationic organosilicon compound** | **Encapsulation efficiency (%)** | **Particle size range (nm)** |
|---|---|---|
| 1.2-(Dimethyldecylsilyl)ethyl-beta-D-glucosamin (SIL 61) | 75-82 | 120-240 |
| 2.2-(Dimethyloctylsilylcholin)ethyl-beta-D-glucopyranosid (SIL 62) | 70-80 | 150-220 |
| 1.2-(Di-N,N,N,trimethylammoniadecylsilyl)ethyl-beta-D-glucopyranosid (SIL 64) | 70-85 | 120-200 |

Unexpectedly, the encapsulation efficiency was high and the particle sizes were in the lower range of 120-240 nm.

### Example 17: Preparation of PEGylated doxorubicin sugar Siosomes carrier

Doxorubicin hydrochloride was dissolved in PEG 600 at a concentration of 10 mg/ml. Tris-buffer was added at pH 7.4 and the temperature was kept at 65°C for 30 minutes under stirring.

The sample was cooled in an ice bath. A dispersion of 10 µmol of Didodecylsilyl-di(2,3,4,6-O-tetraacetyl-β-D-glucopyranosid) as the representatives of amino sugar organo-silicon compound and the doxorubicin hydrochloride, and PEG 600 was prepared by mixing with a high speed homogeniser.

The homogenisation time may vary. The mixture was then incubated at 37°C for 30 minutes. The doxorubicin hydrochloride, and PEG 600 that had not been introduced in siosomes has been removed, and the siosomes mixtures has been lyophilised at the temperature of -70°C.

The above procedures described in example 17 were employed to prepare carrier systems of doxorubicin hydrochloride using PEG 2000 in ethanol, PEG 4000 in ethanol and HEPES as buffer.

In addition, different ratios of doxorubicin hydrochloride : PEG : silanes have been used, for example at a ratio of 1 : 3 : 4.

Surprisingly and unexpectedly, the pre-treatment and/or dissolution of doxorubicin in PEG with the different molecular weights 600, 2000 and 4000 showed strong influence on the encapsulation efficiency of the doxorubicin-PEG and its release from the siosomes. In addition and unexpectedly, it has been shown that the ratio of doxorubicin hydrochloride : PEG : Organosilicon compound influences the targeting and biodistribution of doxorubicin.

### Example 18: Preparation of Doxorubicin-Siosomes carrier using silanes, sugar silanes, cationic, anionic, zwitterionic and PEGylated organosilicon compounds

The above procedures described in example 16 were employed to prepare the carrier siosomes systems in this example. According to these methods of preparation, a dispersion of each of the following organo-silicon compounds were prepared, using doxorubicin hydrochloride solutions at 10 µg/ml.
Sil 8.1: Decyldimethylsilyl-α-D-galactopyranosid
Sil 9.1: Dodecyldimethylsilyl-α-D-glucopyranosid
SIL 15: 1-O-Dioctadecylsilyl-di(2,3,4,6-O-tetracetyl-β-D-galactopyranosid)
SIL 14: 1-o-Dioctadecylsilyl-di(2,3,4,6-O-tetracetyl-β-D-glucopyranosid)1-o-
SIL17: Didodecylsilyl-di(2,3,4,6-O-tetracetyl-β-D-glucopyranosid)
SIL18: Tridodecylsilyl-(2,3,4,6-O-tetracetyl-β-D-glucopyranosid)
Sil22.1 Di(octadecanoyloxy)dimethylsilan

### Cationic organosilicon compounds:

| | |
|---|---|
| SIL 61 | 1.2-(Dimethyldecylsilyl)ethyl-beta-D-glucosamin |
| SIL 62 | 2.2-(Dimethyloctylsilylcholin)ethyl-beta-glucopyranosid |
| SIL 63 | 1.2-(Di-N,N,N,trimethylammoniadecylsilyl)ethyl-beta-D-glucopyranosid |
| SIL 64 | Di-N,N,N,trimethylammoniadidodecylsilyl-di(2,3,4,6-O-tetraacetyl-beta-D-glucopyranosid) |
| SIL 65 | 2-(N,N,N,Dimethylammonia-methyl-decylsilyl)ethyl-beta-D-glucopyranosid |

### Anionic sugar organo-silicon compounds

| | |
|---|---|
| SIL 56 | 2-(Dimethyldecylsilyl)ethyl-beta-D-glucopyranosid-sulfate |
| SIL 57 | 2-(Dimethyldecylsilyl)ethyl-beta-D-glucopyranosid-carboxylate |
| SIL 58 | 2-(Dimethylnonanoic acid silyl)ethyl-beta-D-glucopyranosid |
| SIL 59 | 2-(Dimethylcholesterylhemisuccinatesilyl)ethyl-beta-D-glucopyranosid sulphate |
| SIL 60 | 2-(Dimethyloctadecylsilyl)ethyl-beta-N-acetylmuramic acid |

### Zwitterionic and amino sugar organosilicon compounds

| | |
|---|---|
| SIL 66 | 2-(N,N,N,trimethylammonianonanylsilyl)ethyl-beta-D-glucopyranosid sulphate |
| SIL 67 | 2-(N,N,N,trimethylammonianonylsilyl)ethyl-beta-D-glucopyranosid |
| SIL 68 | 2-(Dimethyldecylsilyl)ethyl-beta-D-glucosamine |

### PEGylated organosilicon compounds

| | |
|---|---|
| SIL 53 | 2-(Dimethylnonaoic acid silyl)ethyl-beta-D-PEG-acetylglucosaminether |
| SIL 54 | 2-(Dimethyloctylsilylcholin)ethyl-beta-D-PEG-acetylglucosaminether |

Each of the above mentioned silanes have been used for the encapsulation of the doxorubicin hydrochloride and the preparation of the lyophilisate according to the procedures described in this invention.

The ratio of doxorubicin HCl to silanes were as follows:

| Doxorubicin HCl | : | Sugar silane / silane |
|---|---|---|
| 1 | : | 1 |
| 1 | : | 3 |
| 1 | : | 5 |
| 1 | : | 10 |

The following parameters have been determined:
- 1: Physico chemical properties
- 2: Encapsulated efficiency of doxorubicin
- 3: Stability
- 4: Particle size distribution prior to lyophilisation and after dehydration using sterile water for injection
- 5: Release profile

| Organosilicon compound | Encapsulation efficiency (%) | Particle size range of the Doxorubicin-siosomes (nm) |
|---|---|---|
| Sil 8.1 | 65-70 | 100-220 |
| Sil 9.1 | 60-65 | 150-200 |
| SIL 15 | 70-78 | 180-250 |
| SIL 14 | 75-80 | 210-260 |
| SIL 17 | 80-85 | 180-240 |
| SIL 18 | 55-60 | 160-250 |
| SIL 21.1 | 60-75 | 210-240 |
| SIL 61 | 80-85 | 200-220 |
| SIL 62 | 70-75 | 220-250 |
| SIL 63 | 65-70 | 120-200 |
| SIL 64 | 70-75 | 140-220 |
| SIL 65 | 60-70 | 200-250 |
| SIL 56 | 60-70 | 200-220 |
| SIL 57 | 75-80 | 180-270 |
| SIL 58 | 70-80 | 150-270 |
| SIL 59 | 60-70 | 180-220 |
| SIL 60 | 60-70 | 200-250 |
| SIL 66 | 55-70 | 150-220 |
| SIL 67 | 60-70 | 200-280 |
| SIL 68 | 70-80 | 180-220 |
| SIL 53 | 80-85 | 180-250 |
| SIL 54 | 80-85 | 200-250 |

Unexpectedly, the above-mentioned results using the different organosilicon compounds for the preparation of the doxorubicin-siosomes carriers according to the procedures of this invention all showed high encapsulation efficiency and narrow particle size ranges and distribution, despite the enormous difference in the chemical structures of these compounds. This distinguishes the carrier systems according to the organosilicon compounds of this invention used for the preparation of the carrier systems from the carrier systems of the prior art and exhibits in addition adjustable targeting properties for drugs and agents.

In addition, it was unexpected that, despite their different chemical structures and charge, the carrier systems formed stable particles with different but adjustable release and efficacy profiles.

### Example 19: Preparation of Doxorubicin-siosomes carriers using combinations of organosilicon compounds

The above procedures described in example 18 have been employed to prepare the following combination siosomes. Formulations with doxorubicin hydrochloride:
1. SIL8 + SIL17
2. SIL9 + SIL17
3. SIL14 + SIL17
4. SIL8 + SIL18
5. SIL8 + SIL17 + SIL22
6. SIL8 + SIL15 + SIL17
7. SIL18
8. SIL22
9. SIL8 + SIL22

The paramters described in example 18 have been determined using the standard procedures according to this invention. The molar ratio of doxorubicin hydrochloride to sugar silane / silane was as follows:

| Doxorubicin HCl | : | Sugar silane / silane "Total amount" |
|---|---|---|
| 1 | : | 1 |
| 1 | : | 3 |
| 1 | : | 5 |
| 1 | : | 10 |

| Organosilicon compounds and combinations | Encapsulation efficiency (%) | Particle size (nm) |
|---|---|---|
| SIL 8 + SIL 17 | 75-80 | 120-250 |
| SIL 9 + SIL 17 | 70-80 | 150-220 |
| SIL 14 + SIL 17 | 65-70 | 200-320 |
| SIL 8 + SIL 18 | 60-80 | 180-270 |
| SIL 8 + SIL 17 + SIL 22 | 70-80 | 250-300 |
| SIL 8 + SIL 15 + SIL 17 | 60-70 | 150-240 |
| SIL 18 | 60-80 | 220-350 |
| SIL 22 | 55-70 | 250-300 |
| SIL 8 + SIL 22 | 60-85 | 220-300 |

Unexpectedly, the above-mentioned results using the different organosilicon compounds and/or the combinations thereof with the different chemical structure and charge have shown very high encapsulation efficiencies in the range of 55-80% and stable particles in the range of 120-300 nm. In addition, the carriers composed of the mixture and/or combinations have shown different release profiles and tissue distributions. It was also unexpected that the encapsulation efficiency of doxorubicin in the siosomes will not be influenced by the molar ratio higher than Doxorubicin : sugar silane 1 : 5.

### Example 20: Preparation of nucleic acids-siosomes complex using cationic silanes, sugar silanes and/or siosomes

The preparation of the siosomes to be used according to the invention will be as mentioned in the examples. Briefly, the silanes and any further lipids to be used are mixed in a suitable solvent, for example in acetone, ethanol, and evaporated to dryness under a stream of nitrogen. To prepare the siosomes, the dried lipid mixture is hydrated in a buffer at selected pH, including short periodical vortexing, and sonicated at the end of the hydration period in order to form multilamellar siosomes.

Nucleic acid includes in this context DNA, RNA, siRNA, and oligonucleotides of DNA and RNA.

To prepare the nucleic acid-siosome complex, the nucleic acid and the lipids are each diluted in an appropriate solvent at selected pH, for example in serum-free DMEM (Dulbecco's Modified Eagle's Medium), mixed and preincubated for a suitable period of time. The amount of nucleic acid to lipid can varied, but a weight ratio between nucleic acid and lipid in the range of 0.10 : 10 to 3 : 4 has been found to be satisfactory.

Due to charge interactions, the nucleic acid forms a strong complex with the cationic siosomes surface after simple mixing of the components.

Good transfection results have been obtained in tests using 2 or 4 µg respectively of RNA to 20 to 40 µg of total lipid.

### Example 21: Preparation of nucleic acid-silane/siosome active substance

The procedures for the preparation of the nucleic acid-siosomes complex using cationic silanes, sugar silanes and/or cationic siosome, sugar siosomes according to the invention are briefly described.

### (i) Using cationic silanes and/or cationic siosomes:

The cationic organosilicon compounds are, but not limited to:
- 1.2-(Dimethyldeclysilyl)ethyl-beta-D-glucosamin, [cationic silane (SIL 61)]
- 2.2-(Dimethyloctylsilylcholin)ethyl-beta-D-glucopyranosid, [cationic silane SIL 62)]
- 1.2-(Di-N,N,N,trimethylammoniadecylsilyl)ethyl-beta-D-glucopyranosid, [cationic silane (63)]
- Di-N,N,N,trimethylammoniadidodecylsilyl-di(2,3,4,6-O-tetraacetyl-beta-D-glucopyranosid), [cationic silane (64)]
- 2-(N,N,N,Dimethylammonia-methyl-decylsilyl)ethyl-beta-D-glucopyranosid, [cationic silane (65)]
- and/or their derivatives and/or siosomes.

The cationic silanes-lipid mixture and/or the empty cationic siosomes (the same or different siosomes prepared from the same or different silanes) loaded with buffer and/or cations (2⁺) such as Ca²⁺, Zn²⁺, Mg²⁺ at selected pH-values, is hydrated in an appropriate medium containing the nucleic acid.

The mixture will be vortexed and/or sonicated for a period of time at suitable temperature.

Procedures for characterisation, particle stirring, stability, release, lyophilisation, sterilisation, rehydration, storage, and administration were employed according to this invention.

The amount of nucleic acid to silanes/siosomes lipid can vary.

| Cationic silane | Encapsulation efficiency (%) | Particle size of the siosomes (nm) |
|---|---|---|
| Cationic silane (SIL 61) | 80-85 | 80-120 |
| Cationic silane (SIL62) | 75-80 | 110-180 |
| Cationic silane (SIL 63) | 70-80 | 150-250 |
| Cationic silane (SIL 64) | 65-75 | 120-220 |
| Cationic silane 5 (SIL 65) | 80-85 | 160-240 |

All cationic silanes have shown unexpected high encapsulation efficiency despite their different chemical structure and charge. In addition, the formed siosomes particle sizes have a narrow range at the lower particle size of 80-250 nm.

### (ii) Using PEGylated cationic silanes and/or PEGylated cationic siosomes:

According to the present invention, PEGylated organosilicon compounds and/or derivatives such as 2-(Dimethyl nonaoic acid silyl)ethyl-beta-D-PEG-actylglucosaminether, 2-(Dimethyloctylsilylcholin)ethyl-beta-D-PEG-acetylglucosaminether have been used for the preparation of siosomes carrier and targeting systems for nucleic acids such as DNA, RNA, siRNA, antisense molecules and/or nucleotides.

The above procedures as described in example 21 (i) were employed to prepare carrier systems, comprising of PEGylated cationic organosilicon, sugar organosilicon, amino-sugar organosilicon compounds and/or PEGylated cationic siosomes with nucleic acids, such as DNA, RNA, siRNA and nucleotides.

### (iii) Using cationic silanes and/or cationic siosomes and PEG:

The above procedures as described in example 13 were employed to prepare carrier systems, comprising of cationic organosilicon, sugar organosilicon, amino-sugar organosilicon compounds and/or cationic siosomes with nucleic acids, such as DNA, RNA, siRNA and PEG. The molecular weight of PEG can vary from 200 to 10,000 Daltons.

Unexpectedly, the siosomes carriers with these agents have shown stable particles and/or complexes with relatively long half-life time in vivo (rat plasma). In addition and unexpectedly, the siosomes carriers with the nucleic acids and/or antisense molecules have shown different specificity for tissue targeting, bioaccumulation and intracellular targeting and distribution.

### Example 22: Preparation of nucleic acids-silane/siosomes active substances, using multiple anionic and/or cationic organosilicon compounds and/or anionic and/or cationic salts

The following compounds are anionic organosilicon compounds such as the anionic sugar organosilicon according to the present invention:
- 2-(Dimethyldecylsilyl)ethyl-beta-D-glucopyranosid sulphate (SIL 56)
- 2-(Dimethyldecylsilyl)ethyl-beta-D-glucopyranosid carboxylate (SIL 57)
- 2-(Dimethylnonanoic acid silyl)ethyl-beta-D-glucopyranosid (SIL 58)
- 2-(Dimethylcholesylhemisuccinatesilyl)ethyl-beta-D-glucopyranosid sulphate (SIL59)
- 2-(Dimethyloctadecylsilyl)ethyl-beta-N-acetylmuramic acid (SIL 60)
- and/or their derivatives.

The procedures for the preparation of the nucleic acid-siosome complex as active substance using anionic silanes, sugar silanes and/or anionic siosomes, sugar siosomes are briefly described herein:
Nucleic acids (DNA, RNA, siRNA, nucleotides) are treated with silanes, sugar silanes and/or siosomes, sugar siosomes and/or with cationic silanes, sugar silanes and/or siosomes, sugar siosomes and/or cationic salts such as Zn2+, Ca2+, Mg2+, or Na+ and/or hydrophilic polymers such as PEG (MW 200 - 10000 daltons). The resulting nucleic acid-silane/siosome complex are then mixed with anionic silanes/sugar silanes, anionic siosomes/sugar siosomes and/or cationic salts such as Zn2+, Ca2+, Mg2+, or Na+. The resulting compound is a carrier system for administration of nucleic acid-silane/siosomes active substances.

Otherwise, the procedures described in the invention were employed for the preparation of the silanes, siosomes, complex characterisation, stability, release, particle sizing, sterilisation and rehydration according to the invention.

It was unexpected that the coating of the carrier composed of the siosomes and the nucleic acids (DNA, RNA, siRNA, nucleotides) with cationic salts such as Zn²⁺, Mg²⁺, Fe²⁺ and/or hydrophilic polymers have formed stable particles and/or complexes, which could be used for the preparation of further specific carriers.

Surprisingly and unexpectedly, the above-mentioned positive charged coated siosomes carriers have formed further stable complexes with the anionic silanes/sugar silanes and/or anionic siosomes. These anionic coated carriers have formed unexpected stable cationic carriers with the cationic salts such as Zn²⁺, Ca²⁺, Mg²⁺ and/or Na⁺.

It was unexpected that the preparation of the multiple coated carrier systems with the siosomes, organosilicon compounds and DNA, RNA, siRNA, nucleotide and/or genetic materials according to the procedures of this invention have shown in vivo long half-time, specific and adjustable tissue and intracellular targeting.

In addition, it was unexpected to retain the structure of the nucleic acid following the multi-coating procedures according to this invention.

The ratio between the used nucleic acids, silanes, siosomes-lipids and/or other lipids, PEG, cations, can vary.

### Example 23: Preparation of active substance-liposomes-siosomes-complex (Lipo-Sio-Complex)

Lipid refers to any suitable material resulting in a bilayer. Lipids used for the preparation of the siosomes, such as phospholipids (phosphatiylethanolamines, phosphatidylcholine, phosphatidylinositol, phosphatidylserine), cationic lipids, such as DOTMA (Lipofectin™), DOSPA (LipofectAMINE™), DOTAP, DC-Chol, DOPE, GL-67™, and anionic lipids, such as cardiolipin (diphosphatidylglycerol) and/or cholesterylhemisuccinate will be used according to the invention in combination with the silanes, sugar silanes, amino-sugar silanes, anionic sugar silanes, cationic sugar silanes, zwitterionic silanes, and/or PEGylated silanes. Preparations of the siosomes-liposomes-active substance according to the invention are briefly described:
Lipids for the preparation of liposomes (A) are mixed with the silanes, sugar silanes, cationic, anionic silanes and/or PEGylated silanes (B), which results in a mixture of the lipids (A) and (B) in appropriate solvents, such as ethanol, acetone or chloroform. The mixture of the lipids occurs by vortex mixing under nitrogen, followed by the removal of residual solvent under high vacuum for a period of time. Lipids are hydrated with appropriate buffers containing the agent and/or active substances at selected pH, temperature and salt concentrations. Thereafter, separation of the unencapsulated agent, lyophilisation, dehydration, characterisation, particle sizing, release, stability, storage and administration can be performed according to the procedures of the invention and to any procedures common to those skilled in the art.

### Example 24: Preparation of siosomes with encapsulated liposomes

The procedures disclosed in this invention can also be applied for the encapsulation of liposomes within siosomes. Liposomes with encapsulated agents and/or active substances are prepared according to the known procedures for the liposomes or lipid bilayer preparations. Particle sizing, separation of the free (unencapsulated) agent, in-vitro stability, lyophilisation will be performed according to the standard methods and procedures of the invention.

Liposomes will be encapsulated in the siosomes prepared according to the procedures described in the invention. The liposomes-siosomes-active substance complex will be lyophilised according to the procedures of the present invention with a cryoprotectant.

Reconstitution is carried out to form a liposome-siosome-complex concentrate, using an appropriate buffer at a selected pH.

### Example 25: Preparation for Siosomes-DNA-Complex formulation using Heating Method (HM-Siosomes)

The preparation procedures according to this invention has unexpected advantage to prepare the Siosomes®-DNA-Complex without the use of any hazardous chemical or process.

This method involves the hydration procedures of the organosilicon compounds and/or derivatives claimed in this invention e.g. the Siosomes® components of 2-(Dimethyldecylsilyl)ethyl-β-D-glucopyranosid or 1-O-Dioctadecylsilyl-di(2,3,4,6-O-tetraacetyl-β-D-glucopyranosid), or any of the cationic sugar organosilicon compounds such as 1,2-(Dimethyldecylsilyl)ethyl-β-D-glucosamone or any of the anionic sugar organosilicon compounds such as 2-(Dimethyldecylsilyl)ethyl-β-D-glucopyranosidsulfate or any Zwitterionic Sugar Silane such as 2-(N,N,N,trimethylammonia nonanylsilyl)ethyl-β-D-glucopyranosid sulfate or any the PEGylated Sugar Silane such as 2-(Dimethylnonaoic acid silyl)ethyl-β-D-PEG acetylglucosaminether or any combination of them, in the presence or without glycerol 1-10% v/v in an aqueous medium that the hydration will take place under heating 40°C-120°C.

Suprisingly and unexpected that the formed Siosomes® according to the procedures described in this invention have shown high physical stability concerning the Siosomal size, homogenous size distribution and drug retention and chemical stability concerns the drug degradation as well as lipid oxidation and hydrolysis.

In addition, the formed HM-Siosomes® were unexpected stable against freezing and thawing, thus ideal for the freeze-drying and the preparation of Siosomes® lyophilised powders.

According to the procedures of this invention, the incorporation of plasmid DNA molecules which are sensitive to high temperature, to the HM-Siosomes® was carried out at room temperature by incubation of DNA with the empty, pre-formed HM-Siosomes®.

According to the procedures of this invention, the incorporation of genetic materials, pharmaceutical agents, antigens and drugs can be achieved by several routes including but not limited to:
- Adding with the drug to the reaction medium along with the Siosomal ingredients with or without glycerol and/or other Silanes and/or Sugar Silanes and/or lipid
- Adding the drug to the reaction medium when temperature has dropped to a point not lower than the transition temperature (T_{C}) of the Silanes and/or organosilicon compounds
- Adding the drug and/or antigen and/or DNA and/or RNA to the HM-Siosomes® after they are prepared e.g. at room temperature

It is known that formation of Siosomes® requires heating the Siosomal components at temperatures not lower than the T_{C} of the Silanes and/or Sugar Silanes. This is because below T_{C} lipids are in the gel state and cannot usually form closed continous bilayer structures.

According to the invention procedures, when cholesterol (or any other sterol) is used as a Siosomal components, HM-Siosomes® are prepared successfully at 120°C.

Since the majority of the Silanes and Sugar organosilicon compounds molecules employed as Siosomal constituents have transition temperatures below 60°C, in the absence of cholesterol (or other sterols which require high temperatures to be dissolved) HM-Siosomes® can be prepared for example at 60°C-70°C.
- Studies indicated no irreversible change in DNA secondary structure even after heating for 2 hours at 60°C.
- Siosomes® mean diameters 0.2 to 0.5 µm with encapsulation efficiencies of 8 to 14 L/mol of Silanes
- Both uni- and oligolamellar vesicles formed.
- Vesicle size, encapsulation efficiency, capture volume µl/mg Silanes/Sugar Silanes, and permeability varied as a function of the Siosomes® composition, ionic strength of the buffer used, and the relative rations of the Drug:Silanes/Sugar Silanes.

### Example 26: Preparation and stability of the unloaded Siosomes® using the Sugar Silanes - 2-(Dimethyldeculsilyl)ethyl-β-D-glucopyranosid (SIL 2) and Didodecylsilyl-di(2,3,4,6-0-tetra-acetyl-β-D-glucopyranosid) (SIL 17)

The following are the general procedures according to this invention for the preparation of the Siosomes® using ethanol injection method:
An ethanol solution of the Sugar Silane was injected rapidly into an excess of de-ionized water through a syringe equipped with a fine needle. The force of injection is in many cases sufficient to achieve a good Siosomes® suspension. In some cases the Siosomes® suspension was sonicated during the injection and afterwards for additional 15-45 minutes. The ratio of injected ethanol to water is maintained in a way that the resulting suspension contains not more than 4-7.5% ethanol. The Siosomes® suspension was prepared with a Sugar Silane concentration of 0.5-2.5 mg/ml depending on the used Silane. With this concentration, the final Siosomes® suspension is ready for use for many applications and for the preparation of the lyophilised Siosomes® powder.

The obtained test samples were analyzed using a Zetasizer from Malvern to measure the particle size of the Siosomes® and its concentration (via determination of the count rate which related to the sample quality and concentration).

The Zetasizer from Malvern performs particle size measurements using a process called Dynamic Light Scattering (DLS). Dynamic Light Scattering (also known as PCS - Photon Correlation Spectroscopy) measures Brownian motion and relates this to the size of the particles. It does this by illuminating the particles with a laser and analyzing the intensity fluctuations in the scattered light. If the particles or molecules are illuminated with a laser, the intensity of the scattered light fluctuates at a rate that is dependent upon the size of the particles as smaller particles are "kicked" further by the solvent molecules and move more rapidly. Analysis of these intensity fluctuations yields the velocity of the Brownian motion and hence the particle size using the Stokes-Einstein relationship.

### Stability Testing

The unloaded Siosomes^{®} prepared according to the procedures of this invention using ethanolic solution injection has been investigated for stability.

The following parameters have been considered:
- The optimal particle size of the Siosomes^{®} for the further application of the Siosomes^{®} for the encapsulation of active, for screening and for the performance of in-vitro and in-vivo animal studies
- The optimal manufacturing conditions to achieve the best particle size distribution for the required particle size (approximately 90 %)
- The performance of the Stability testing for the unloaded Siosomes^{®} using the particle size measurements and their distribution
- The performance of the Stability testing of the prepared unloaded Siosomes^{®} at 4°C, 25°C and 37°C and 42°C
- Stability testing of the prepared encapsulated Siosomes^{®} at: Acidic conditions, alkaline conditions, oxidative conditions and light

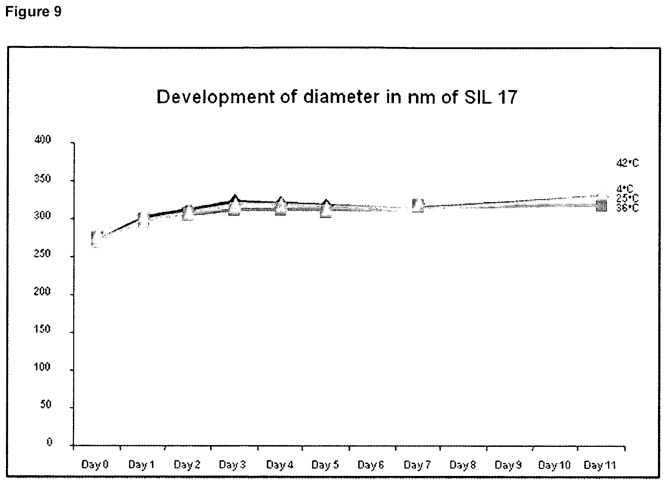

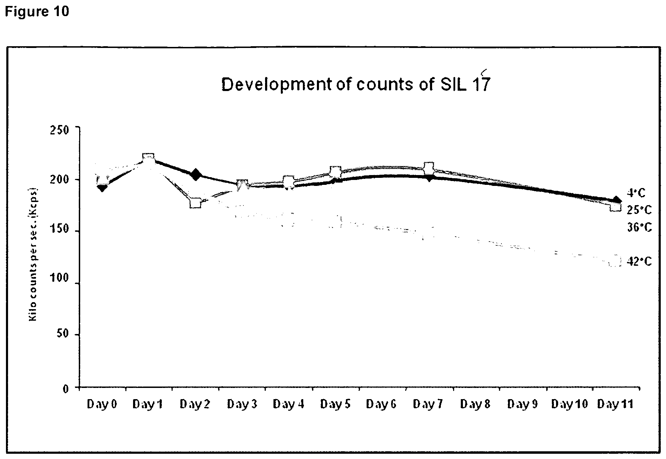

Figures 9 and 10 illustrate the stability of the Siosomes^{®} derived from SIL 17 for each temperature. The curves below show the minor differences between the changes of the Siosomes^{®} particle size diameter versus the temperature and stability period of the Siosomes^{®} prepared from SIL 17. It is important to notice, that the diameter range is more depending on the used Silane than on the method used for the Siosomes^{®} preparation. The best generated results based on the quality and quantity of the prepared Siosomes^{®} have been found in the diameter range of 250-350 nm. The aqueous solutions of these suspensions are stable for several days.

All measurements are performed with a Malvern Zetaziser. The Zetasizer was programmed to perform 3 x 15 measurements for each sample. For each temperature and reading point (for each temperature test series 12 sample measurements were performed) two samples has been tested.

The count rate is measured in Kcps (Kilo counts per second), and enables the samples quality and concentration to be checked.

It is obvious that the count rate is stable for Siosomes^{®} derived from SIL 17 over several days. At 42°C the count drops a little bit after several days but not drastically.

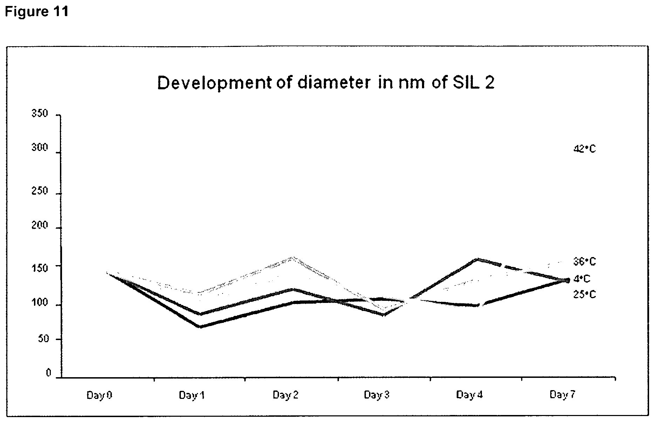

Figure 11 illustrates the stability of the Siosomes^{®} derived from SIL 2 for each temperature. The curves show the minor differences between the changes of the Siosomes^{®} particle size diameter versus the temperature and stability period of the Siosomes^{®} prepared from SIL 2. At the temperature of 42°C the diameter is growing after 4 days.

The sugar Silane Didodecylsilyl-di(2,3,4,6-O-tetra-acetyl-beta-D-glucopyranosid) (SIL17) has been used for the encapsulation of the chemostatic anticancer drug Cis-Oxoplatin (platinum IV) in Siosomes®. The Cis-Oxoplatin-Siosomes®-Complex has been prepared according to the procedures of this invention and used for the performance of different *in vitro* and *in vivo* animal studies.

The Siosomes® lypholised powders following their reconstitution e.g. with sterile water for injections have shown very long stability at different temperatures. This will have advantageous for the clinical use of the Siosomes® formulations, and the reduction of the treatment costs due to stability of the Drug-Siosomes® emulsions (multiple use during several days).

### Example 27: Assessment of the Pharmacokinetic and Bioaccumulation of the Platinum-Siosomes®-Complexes Following the Parenteral Application in Rats

IPSS E011: A Clinical Study in Rats on the Pharmacokinetics and Bioaccumulation of Platinum Complexes following the Repeated Dose over 7 Consecutive Days of Formulations (PT-11, PT-12 and PT-13), with 7 Days Non-Treatment Period

### Study medication:

| Drug Code | Formulation | Route of Administration | Dose |
|---|---|---|---|
| PT-11 | Standard Cis-oxoplatin | Oral | 1.0 mg |
| PT-12 | Standard Cis-oxoplatin | Intravenous | 1.0 mg |
| PT-13 | Cis-oxoplatin-Siosomes®-Complex | Intravenous | 1.0 mg |

### Study outlines:

| | |
|---|---|
| Number of animals | 30 rats in total: |
| | • 18 rats in 3 treatment groups (6 rats per group) |
| | • 12 rats in a 2 untreated control groups (6 rats per group) |
| Inclusion Criteria | • Male |
| | • 4-6 weeks old |
| | • Body weight: 180 - 200 grams |
| Exclusion Criteria | • Female |
| | • Any concomitant disease |
| | • Any concomitant medication |
| Route of Administration | • Treatment group PT-11 will receive drugs by the oral route |
| | • Treatment groups PT-12 and PT-13 will receive drugs by the intravenous route |
| Dosage Timings | • All animals from the control groups will receive no medication |
| | • All animals from the treatment groups will receive repeated doses administrated at day 1,2,3,4,5,6 and 7. |
| Target Parameters | To investigate the effects on distribution and elimination of different routes of administration and of different formulations in comparison to the Cis-oxoplatin-Siosomes®-Complex. |
| Determination of Total Platinum in Blood, and the following organs: Spleen, Stomach, Lungs and Kidney | A validated Atom Absorption Spectroscopy (AAS) method has been used for the determination of Total Platinum in blood and tissues. |

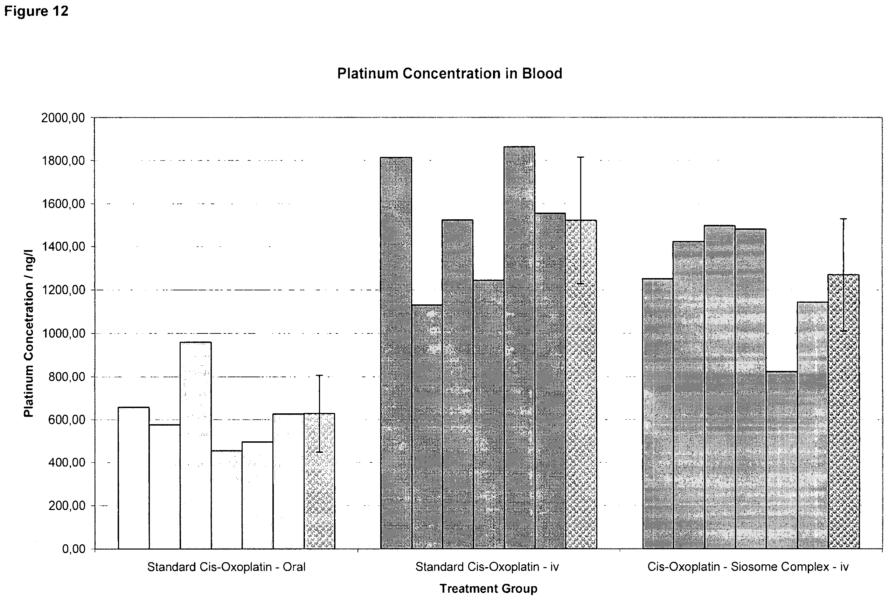

The following tables and figure 12 illustrate a summary of the generated results. It demonstrates the differences in the blood and tissue distributions following the intravenous administration of Cis-oxoplatin-Siosomes®-Complex in comparison to the free unencapsulated Cis-oxoplatin.

### Distribution of the Total Platinum Concentration in Blood

| **Rat Number** | **Concentration of Total Platinum in Blood (ng/ml)** |
|---|---|
| 13 | 1814,30 |
| 14 | 1131,40 |
| 15 | 1522,90 |
| 16 | 1244,30 |
| 17 | 1864,30 |
| 18 | 1554,30 |
| **Average** | **1521,92** |
| **Standard Deviation ±** | **294,39** |
| **Coefficient of Variation %** | **19** |

| | |
|---|---|
| Total Platinum Concentration in Blood - Standard Cis-Oxoplatin: Intravenous | |

| **Rat Number** | **Concentration of Total Platinum in Blood (ng/ml)** |
|---|---|
| 19 | 625,70 |
| 20 | 711,40 |
| 21 | 748,60 |
| 22 | 740,00 |
| 23 | 411,40 |
| 24 | 572,90 |
| **Average** | **635,00** |
| **Standard Deviation ±** | **129,39** |
| **Coefficient of Variation %** | **20** |

| | |
|---|---|
| Total Platinum Concentration in Blood - Cis-Oxoplatin-Siosomes-Complex: Intravenous | |

### Distribution of the Total Platinum Concentration in Spleen

| **Rat Number** | **Concentration of Total Platinum in Spleen (µg/g)** |
|---|---|
| 13 | 1,99 |
| 14 | 2,28 |
| 15 | 2,35 |
| 16 | 2,10 |
| 17 | 3,81 |
| 18 | 1,80 |
| **Average** | **2,39** |
| **Standard Deviation ±** | **0,72** |
| **Coefficient of Variation %** | **30** |

| | |
|---|---|
| Total Platinum Concentration in the Spleen - Standard Cis-Oxoplatinum Intravenous | |

| **Rat Number** | **Concentration of Total Platinum in Spleen (µg/g)** |
|---|---|
| 19 | 0,272 |
| 20 | 0,84 |
| 21 | 0,88 |
| 22 | 1,06 |
| 23 | 0,44 |
| 24 | 0,97 |
| **Average** | **0,74** |
| **Standard Deviation ±** | **0,31** |
| **Coefficient of Variation %** | **42** |

| | |
|---|---|
| Total Platinum Concentration in the Spleen - Cis-Oxoplatin-Siosomes-Complex: Intravenous | |

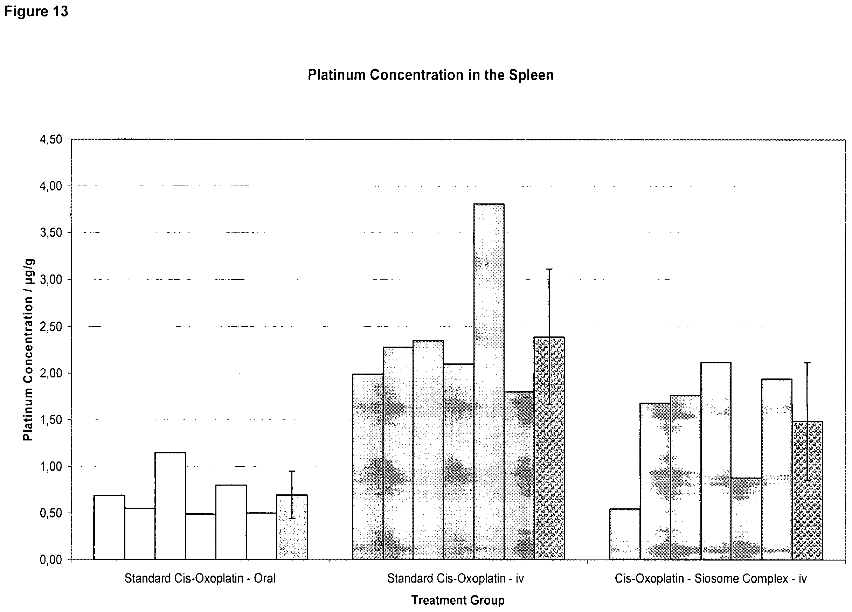

It was unexpected that the concentration of total platinum in spleen following the intravenous administration of the Cis-Oxoplatin-Siosomes®-Complex is lower than the concentration following the administration of the free unencapsulated drug. This resulted in less toxicity in the spleen (Fig. 13).

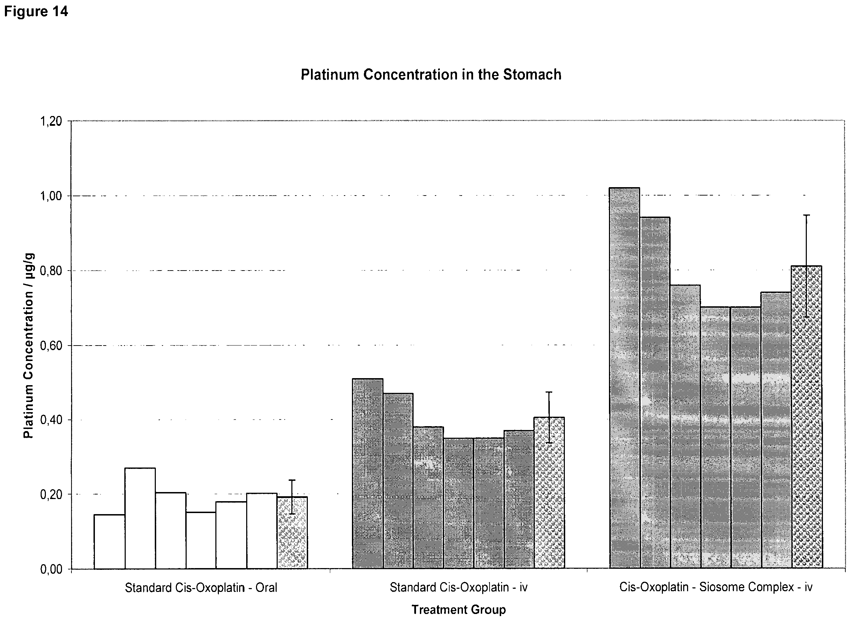

### Distribution of Total Platinum Concentration in Stomach (Fig. 14)

| **Rat Number** | **Concentration of Total Platinum in Stomach (µg/g)** |
|---|---|
| 13 | 0,51 |
| 14 | 0,47 |
| 15 | 0,38 |
| 16 | 0,35 |
| 17 | 0,35 |
| 18 | 0,37 |
| **Average** | **0,41** |
| **Standard Deviation ±** | **0,07** |
| **Coefficient of Variation %** | **17** |

| | |
|---|---|
| Total Platinum Concentration in the Stomach - Standard Cis-Oxoplatin Intravenous | |

| **Rat Number** | **Concentration of Total Platinum in Stomach (µg/g)** |
|---|---|
| 19 | 0,32 |
| 20 | 0,20 |
| 21 | 0,21 |
| 22 | 0,28 |
| 23 | 0,22 |
| 24 | 0,21 |
| **Average** | **0,24** |
| **Standard Deviation ±** | **0,05** |
| **Coefficient of Variation %** | **20** |

| | |
|---|---|
| Total Platinum Concentration in the Stomach - Cis-Oxoplatin Siosomes Complex Intravenous | |

### Distribution of Total Platinum Concentration in Lung

| **Rat Number** | **Concentration of Total Platinum in Lung (µg/g)** |
|---|---|
| 13 | 0,80 |
| 14 | 0,62 |
| 15 | 1,09 |
| 16 | 0,52 |
| 17 | 0,71 |
| 18 | 0,86 |
| **Average** | **0,77** |
| **Standard Deviation ±** | **0,20** |
| **Coefficient of Variation %** | **26** |

| | |
|---|---|
| Total Platinum Concentration in the Lung - Standard Cis-Oxoplatin Intravenous | |

| **Rat Number** | **Concentration of Total Platinum in Lung (µg/g)** |
|---|---|
| 19 | 3,14 |
| 20 | 0,35 |
| 21 | 0,31 |
| 22 | 0,43 |
| 23 | 0,29 |
| 24 | 0,26 |
| **Average** | **0,80** |
| **Standard Deviation ±** | **1,15** |
| **Coefficient of Variation %** | **145** |

| | |
|---|---|
| Total Platinum Concentration in the Lung - Cis-Oxoplatin Siosomes Complex Intravenous | |

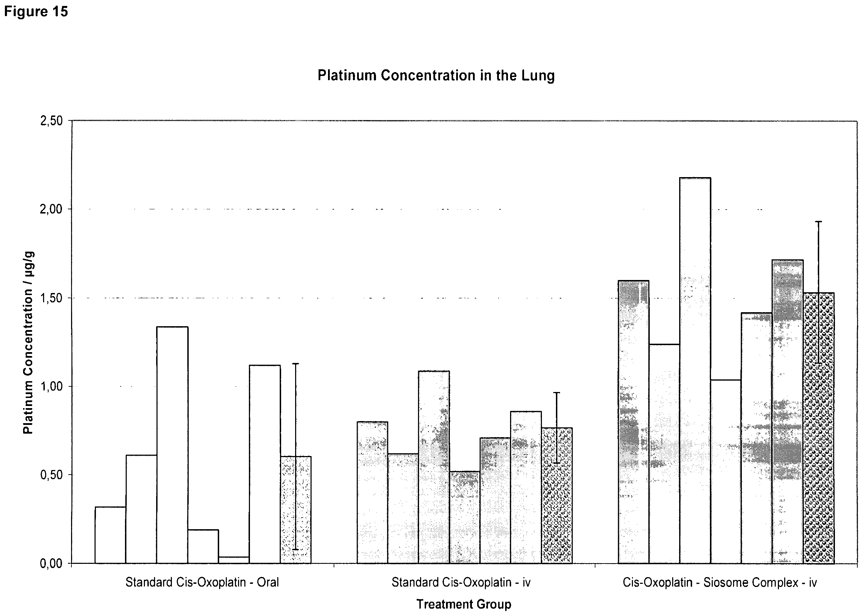

It was unexpected that the concentration of total platinum in lung following the administration of Cis-Oxoplatin-Siosomes®-Complex was higher than the platinum concentration following the administration of the same amount of the free unencapsulated drug. This will be of advantage of the the Drug-Siosomes®-formulation to target the lung e.g.for the treatment of lung cancer (Fig. 15).

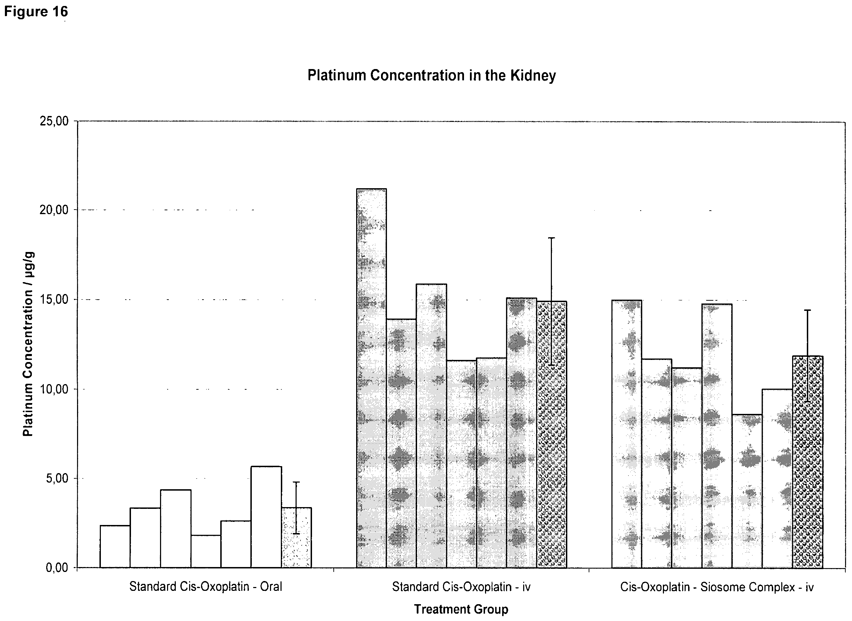

### Distribution of Total Platinum Concentration in Kidney (Fig. 16)

| **Rat Number** | **Concentration of Total Platinum in Kidney (µg/g)** |
|---|---|
| 13 | 21,22 |
| 14 | 13,92 |
| 15 | 15,88 |
| 16 | 11,62 |
| 17 | 11,76 |
| 18 | 15,12 |
| **Average** | **14,92** |
| **Standard Deviation ±** | **3,54** |
| **Coefficient of Variation %** | **24** |

| | |
|---|---|
| Total Platinum Concentration in the Kidney - Standard Cis-Oxoplatin Intravenous | |

| **Rat Number** | **Concentration of Total Platinum in Kidney (µg/g)** |
|---|---|
| 19 | 7,50 |
| 20 | 5,86 |
| 21 | 5,61 |
| 22 | 7,39 |
| 23 | 4,30 |
| 24 | 5,02 |
| **Average** | **5,95** |
| **Standard Deviation ±** | **1,28** |
| **Coefficient of Variation %** | **22** |

| | |
|---|---|
| Total Platinum Concentration in the Kidney - Cis-Oxoplatin Siosomes Complex Intravenous | |

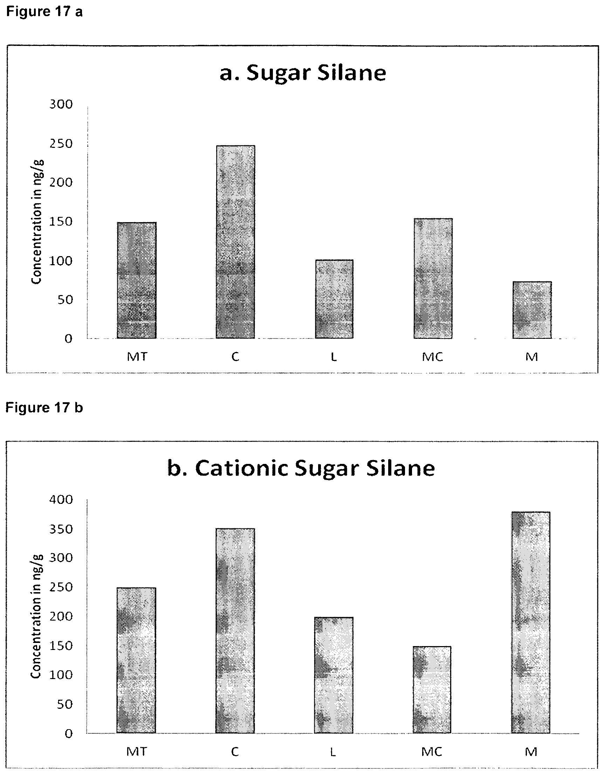

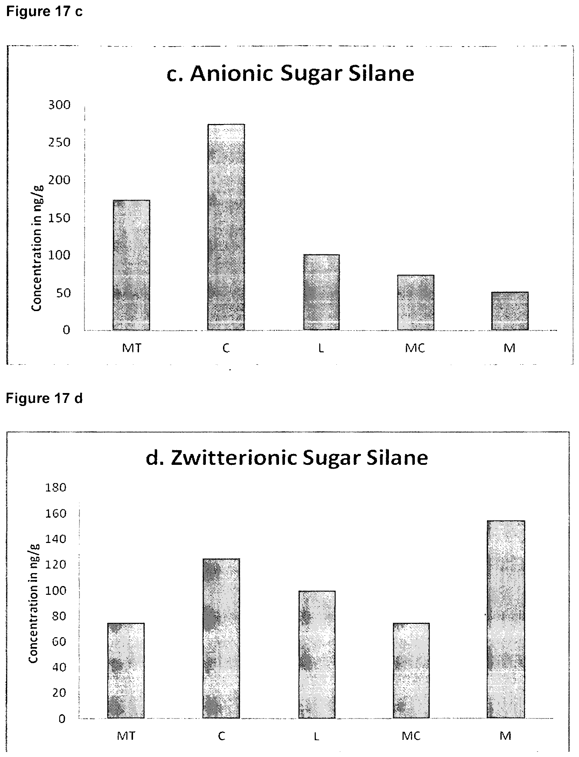

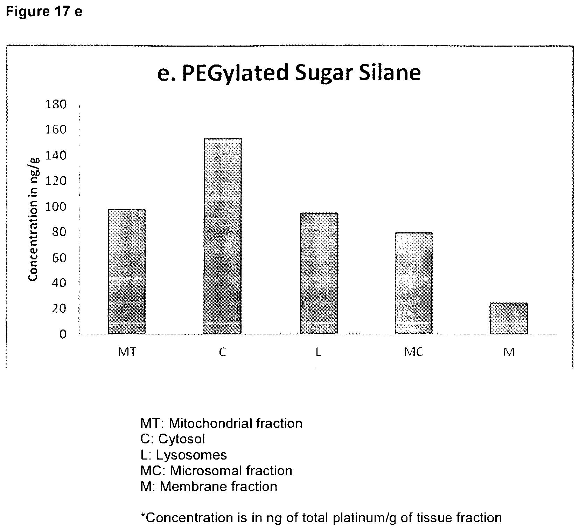

### Example 28: Intracellular Distribution of Cis-Oxoplatin within the Rat Liver Using Cis-Oxoplatin Encapsulated in Siosomes® (Figures 17, 18)

1. The objective of the study to assess the intracellular distribution of Cis-Oxoplatin (as model drug molecule) in rat liver using Cis-Oxoplatin encapsulated in Siosomes®, using the following organosilicon compounds:
   a. Sugar Organosilicon Compound: **Didodecylsilyi-di(2,3,4,6-O-tetraacetyl-β-D-glucopyranosid (SIL 17)**
   b. Cationic Sugar Organosilicon Compound: **2.2-(Dimethyloctylsilylcholin)ethyl- β-D-glucopyranosid (SIL62)**
   c. Anionic Sugar Organosilicon Compound: **2-(Dimethyidecylsilyl)ethyl-β-D-glucopyranosidsulfate (SIL56)**
   d. Zwitterionic Sugar Organosilicon Compound: **2-(N,N,N, trimethylammonianonaylsilyl)ethyl- P-D-glucopyranosidsulfate (SIL66)**
   e. PEGylated Organosilicon Compound: **2-(Dimethyloctylsilylcholin)ethyl-β-D-PEG-acetylglucosaminether (SIL54)**
2. The different Siosomes® using each of the above mentioned organosilicon compounds have been prepared according to the procedures of this invention. The particle size range of the Siosomes® was 120-300 nm.

### Study Design

| | | |
|---|---|---|
| A. | Number of animals: | 60 rats total (30 rats in 5 treatment groups, 30 rats in 5 control treatment groups) |
| B. | Inclusion criteria: | Male, 4-6 weeks old, body weight of 250-350 grams |
| C. | Exclusion criteria: | Female, any concomitant disease and medication |
| D. | Procedures: | Animal met all of the inclusion criteria and did not meet any of the exclusion criteria were enrolled into the study. 60 animals have been enrolled in total. |

### i. Formulation of Siosomes® for administration

The Platinum-Siosomes®-Complex formulations were supplied in vials containing 10 mg each. Before administration, the contents of each vial was dissolved using 2 ml of sterile distilled water.

Each rat was administered with 1.5 mg investigative formulation, therefore each rat was administered 300 µl.

### ii. Treatment

At the start of the study, all animals in all treatment groups received 1.5 mg of investigative drug. All drugs given via intravenous route were treated in an ultrasonic bath for five minutes at room temperature.

### iii. Fractionation of the liver tissues

Tissue fractionation was performed by differential centrifugation yielding 5 fractions:
a. Mitochondrial fraction
b. Cytosol
c. Lysosomes
d. Microsomal fraction
e. Membrane fraction

### iv. Measurement

Animals have been sacrificed 3 hours after the administration of the Siosomes® formulations. The livers have been removed from all animals. The liver homogenate has been prepared from the liver of each treatment group and used for the fractionation of the liver cells.

Each of the animals in the treatment control group for the determination of total platinum in the liver homogenate have been administered the Drug-Siosomes® formulations in the same way/procedures as the treatment groups.

Total platinum concentrations have been determined in each fraction for the treated and untreated animal groups.
- Average animal weight = 211.64 ± 21.23 g
- Average liver weight = 10.40 ± 1.27 g
- Average concentration of total platinum in the liver homogenate of the treatment groups (control)
   Group 1: Sugar Organosilicon Compound = 2.34 ± 0.82 µg/g
   Group 2: Cationic Sugar Organosilicon Compound = 4.40 ± 1.30 µg/g
   Group 3: Anionic Sugar Organosilicon Compound = 2.20 ± 0.90 µg/g
   Group 4: Zwitterionic Sugar Organosilicon Compound = 3.10 ± 0.70 µg/g
   Group 5: PEGylated Organosilicon Compound = 2.90 ± 1.20 µg/g

It was unexpected that the concentration of total platinum in the liver homogenate following the i.v administration of cationic Siosomes® prepared from Cationic Sugar Organosilicon compound was significantly higher than the measured concentration in the liver tissue homogenate of the other treatment groups. Therefore, it was unexpected that the liver targeting with the cationic Siosomes® is more specific than the other Siosomes® according to this invention.

Surprisingly it was unexpected that the intracellular distribution of the encapsulated drug in all fractions was significantly higher following the intravenous application of Cationic Sugar Silanes. As all expected results are the concentrations in the cytosol and membrane fractions which are higher than the measured concentrations in the same fractions following the administration of the other encapsulated Siosomes®.

### Example 29: Preparation of a combination of clearly defined free unencapsulated Cis-Oxoplatin as anti-cancer drug with clearly defined Cis-Oxoplatin encapsulated in Siosomes® (Figure 19)

According to the procedures of this invention for the preparation of the combination formulation, a batch of 50% of free Cis-Oxoplatin (5mg)and 50% of encapsulated Cis-Oxoplatin (5mg) in Siosomes® have been prepared and investigated in animal study with Rats. The combination has been administered intravenously versus 10mg of free un-encapsulated Cis-Oxoplatin. The total platinum concentration in blood has been determined using Atom Absorption Spectroscopy (AAS).

It was unexpected that the toxic effects decreased drastically and the pharmacokinetic profile has shown combined short and long acting profiles. This will positively influence the efficacy profile of the combination and the improvement of the Quality of Life of the patients.

## Claims

1. Carrier and targeting system for the intracellular delivery and release of a siosome- and/or siosome-liposome-entrapped active substance, comprising a composition, wherein said composition comprises:
- organosilicon, sugar organosilicon, amino-sugar organosilicon, alkyl, alkoxy or acyloxy organosilicon, peptide organosilicon, nucleotide organosilicon and/or amino acid(s) organosilicon,
- active substance, and
- lipids,
whereby
the organosilicon, sugar organosilicon, or amino-sugar organosilicon compounds are **characterized by** the general formula (I): 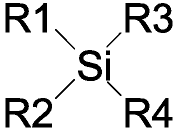 whereby R₁ and R₂ can be the same or different, and where R₃ and R₄ can be the same or different, whereby
R₁, R₂ = Monosaccharide, disaccharide, oligosaccharide, nucleotide, alkyl or aryl, aromatic or aliphatic heterocycles, and
R₃, R₄ = Polynucleotide, peptide, aliphatic chains, proteins, siRNA, RNA, DNA, ligand, vector, fatty acid, acyl group,
**characterized in that**
one or more cationic lipids are covalently attached to the silicon and/or to chemical groups and/or residues of R₁, R₂, R₃ and R₄ of compounds according to the general formula (I).

2. Carrier and targeting system according to the preceding claim comprising a cationic sugar silane, **characterized in that** one or more cationic lipids are covalently attached to the silicon and/or to chemical groups and/or residues of R₁, R₂, R₃ and R₄ of the general formula (I), whereby
R₁ = Monosaccharide, di-saccharide,amino saccharide, oligosaccharide;
R₂ = Monosaccharide, di-saccharide, oligosaccharide, amino acid, peptide, nucleoside, nucleotides, alkyl, aryl residues, aromatic or aliphatic heterocycles;
R₃, R₄ = cationic lipid, and/or cationic amino-acid lipid, or
R₃, R₄ = 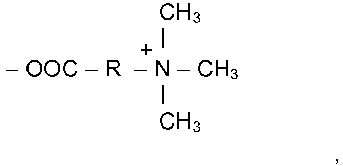 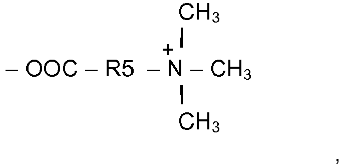 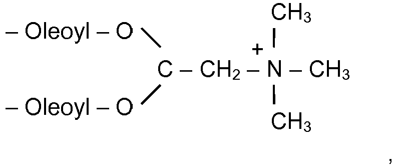
or a peptide residue of the formula: 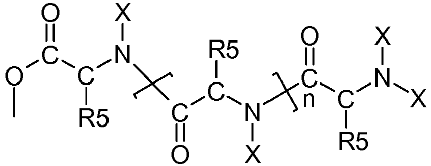 wherein
R represents an unbranched or branched alkyl, alkenyl or alkinyl residue with 5 to 29 C atoms which can be substituted by one to three halogen atoms, alkoxy residues with 1 to 18 C atoms or amino groups,
the residues R5, which can be the same or different, represent the residue remaining after the removal of the group 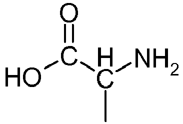 from an amino acid,
the residues X, which may be the same or different, represent a hydrogen atom or an amino-protective group usually occurring in peptide chemistry, and
n represents an integer from 1 to 12, or wherein
R₃ and R₄, which can be the same or different, each represent an aryl group, such as a phenyl group, or the residue remaining after the removal of a hydrogen atom from a monosaccharide, disaccharide, amino sugar or a hydroxyl carbon acid, or alkoxy residues with 1-5 C atoms, or acyl residues of an amino acid with a free or protected amino group, or a dipeptide, tripeptide or tetrapeptide residue of an amino acid with free or protected amino groups, or they have the meaning R₁ and R₂.

3. Carrier and targeting system according to any one of the preceding claims, **characterized in that** the encapsulated and/or entrapped active substance is an anti-cancer agent, such as doxorubicin, cis-platin, carbo-platin and/or oxali-platin, and/or genetic material, preferably a nucleic acid such as DNA, RNA, siRNA and/or an anti-viral and/or anti-bacterial agent.

4. Carrier and targeting system according to any one of the preceding claims, **characterized in that** polyethylene-glycol is covalently attached to the silicone and/or one or more of the groups R₁, R₂, R₃ and R₄ of the general formula (I), preferably by means of a cleavable spacer linker.

5. Carrier and targeting system according to any one of the preceding claims, **characterized in that** the composition comprises a cryoprotectant, preferably a disaccharide selected from the group consisting of sucrose, maltose, trehalose, and/or lactose, more preferably a disaccharide having a concentration of 1-40%.

6. Carrier and targeting system according to any one of the preceding claims, comprising additionally of liposomes, unsaturated lipids, a vesicle-forming lipid, a hydrophilic polymer, glycolipids such as cerebiosides and gangliosides and/or liposomes, whereby the vesicle-forming lipids preferably exhibit two hydrocarbon chains, preferably between 8-26 carbon atoms in length, typically acyl chains, and a polar head group, and more preferably that the phospholipids of the liposomes are selected from the group comprising phosphatidylcholine (PC), phosphatidylethanolamine (PE), phosphatidic acid (PA), Phosphatidyl-inositol (PI), and sphingomyelin (SM).

7. Method for preparing the carrier and targeting system according to any one of the preceding claims, **characterized by**
a) mixing one or more organosilicon, sugar organosilicon, amino-sugar organosilicon, alkyl, alkoxy or acyloxy organosilicon, peptide organosilicon and/or amino acid(s) organosilicon, lipids, salts and/or the vesicles formed from them, with one or more polymers, such as PEG, one or more salts, such as Zn²⁺, Ca²⁺, Mg²⁺, Na⁺, buffers, with one or more active substances, such as doxorubicin, cis-oxoplatin, oxali-platin, nucleic acids, such as DNA, RNA, siRNA, nucleosides at selected pH, salt concentration and temperature, preferably in a buffer solution that comprises TRIS, HEPES; MOPS, MES and/or other commonly used biological buffers,
**characterized in that**
one or more cationic lipids are covalently attached to the silicon and/or to chemical groups and/or residues of R₁, R₂, R₃ and R₄ of compounds according to the general formula (I);
b) homogenisation, sonication and/or extrusion of the mixture, followed by
c) separation of the free active substance,
d) sterile filtration of the mixture,
e) lyophilisation,
f) reconstitution to form a siosome and/or a combined siosome-liposome complex concentrate.

8. Method according to claim 7, **characterized in that** the active substance, preferably nucleic acids, DNA, RNA and/or siRNA, is loaded with cations prior to preparation of the siosome carrier systems.

9. Method according to claims 7 or 8, **characterized in that** the cationic silanes and/or siosomes are loaded with anionic salts.

10. Carrier and targeting system according to any one of claims 1 to 6 for use as a medicament, adjuvant or as a prophylaxis for diseases.

11. Carrier and targeting system according to any one of claims 1 to 6 for use as a medicament in the treatment of cancer.

12. Use of the carrier and targeting system according to any one of the claims 1 to 6 in cosmetics.

13. Pharmaceutical agent comprising the carrier and targeting system according to any one of claims 1 to 6 and a pharmaceutically acceptable carrier and/or pharmaceutically acceptable salt.

## Patentansprüche

1. Träger- und Targeting-System für interzelluläre Zuführung und Abgabe eines in Siosom und/oder Siosom/Liposom eingeschlossenen Wirkstoffs, das eine Zusammensetzung umfasst, wobei die Zusammensetzung Folgendes umfasst:
- Organosilikon, Zucker-Organosilikon, Aminozucker-Organosilikon, Alkyl-, Alkoxy- oder Acyloxy-Organosilikon, Peptid-Organosilikon, Nukleotid-Organosilikon und/oder Aminosäure(n)-Organosilikon,
- einen Wirkstoff und
- Lipide,
wobei
die Organosilikon-, Zucker-Organosilikon- oder Aminozucker-Organosilikon-Verbindungen durch die folgende allgemeine Formel (I) gekennzeichnet sind: 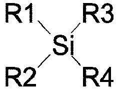 wobei R₁ und R₂ gleich oder verschieden sein können und wobei R₃ und R₄ gleich oder verschieden sein können, wobei
R₁, R₂ = Monosaccharid, Disaccharid, Oligosaccharid, Nukleotid, Alkyl oder Aryl, aromatische oder aliphatische Heterocyclen und
R₃, R₄ = Polynukleotid, Peptid, aliphatische Ketten, Proteine, siRNA, RNA, DAN, Ligand, Vektor, Fettsäure, Acylgruppe,
**dadurch gekennzeichnet, dass**
ein oder mehrere kationische Lipide kovalent an das Silikon und/oder an chemische Gruppen und/oder Reste von R₁, R₂, R₃ und R₄ von Verbindungen gemäß der allgemeinen Formel (I) gebunden sind.

2. Träger- und Targeting-System nach dem vorangehenden Anspruch, das ein kationisches Zucker-Silan umfasst, **dadurch gekennzeichnet, dass** die einen oder mehreren kationischen Lipide kovalent an das Silikon und/oder an chemische Gruppen und/oder Reste von R₁, R₂, R₃ und R₄ von Verbindungen gemäß der allgemeinen Formel (I) gebunden sind,
wobei
R₁ = Monosaccharid, Disaccharid, Aminosaccharid, Oligosaccharid;
R₂ = Monosaccharid, Disaccharid, Oligosaccharid Aminosäure, Peptid, Nukleosid, Nukleotide, Alkyl, Arylreste, aromatische oder aliphatische Heterocyclen;
R₃, R₄ = kationisches Lipid und/oder kationisches Aminosäure-Lipid oder
R₃, R₄ = 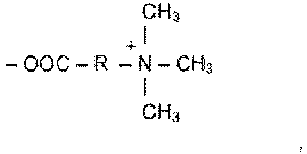 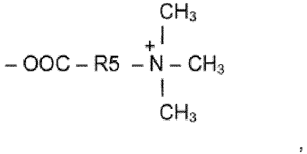 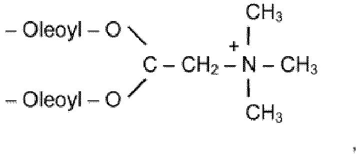
oder ein Peptidrest der Formel: 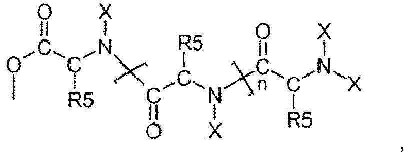 wobei
R Folgendes darstellt: einen unverzweigten oder verzweigten Alkyl-, Alkenyl- oder Alkinylrest mit 5 bis 29 C-Atomen, die durch ein bis drei Halogenatome substituiert werden können, Alkoxyreste mit 1 bis 18 C-Atomen oder Aminogruppen,
die Reste R5, die gleich oder verschieden sein können, stellen nach dem Entfernen der Gruppe 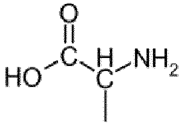 den übrigbleibenden Rest von einer Aminosäure dar,
die Reste X, die gleich oder verschieden sein können, ein Wasserstoffatom oder eine AminoSchutzgruppe darstellen, die üblicherweise in der Peptidchemie vorkommt, und
n eine ganze Zahl zwischen 1 und 12 darstellt oder wobei
R₃ und R₄, die gleich oder verschieden sein können, jeweils eine Arylgruppe darstellen, wie etwa eine Phenylgruppe oder den nach dem Entfernen eines Wasserstoffatoms von einem Monosaccharid, Disaccharid, Aminozucker oder einer Hydroxyl-Carbonsäure übrig bleibenden Rest oder Alkoxyreste mit 1-5 C-Atomen oder Acylreste einer Aminosäure mit einer freien oder geschützten Aminogruppe oder einen Dipeptid-, Tripeptid- oder Tetrapeptid-Rest einer Aminosäure mit freien oder geschützten Aminogruppen, oder die das übrige R₁ und R₂ aufweisen.

3. Träger- und Targeting-System nach einem der vorhergehenden Ansprüche, **dadurch gekennzeichnet, dass** die umhüllte und/oder eingeschlossene Wirksubstanz ein Antikrebsmittel ist, wie etwa Doxorubicin, Cis-Platin, Carboplatin und/oder Oxaliplatin, und/oder genetisches Material, vorzugsweise eine Nukleinsäure, wie etwa DNA, RNA, siRNA und/oder ein antivirales und/oder antibakterielles Mittel.

4. Träger- und Targeting-System nach einem der vorhergehenden Ansprüche, **dadurch gekennzeichnet, dass** Polyethylenglykol kovalent an das Silikon und/oder eine oder mehrere der Gruppen R₁, R₂, R₃ und R₄ der allgemeinen Formel (I) gebunden ist, vorzugsweise mithilfe eines spaltbaren Abstandshalters.

5. Träger- und Targeting-System nach einem der vorhergehenden Ansprüche, **dadurch gekennzeichnet, dass** die Zusammensetzung ein Gefrierschutzmittel umfasst, vorzugsweise ein Disaccharid, das aus der Gruppe bestehend aus Saccharose, Maltose, Trehalose und/oder Laktose ausgewählt ist, noch bevorzugter ein Disaccharid, das eine Konzentration von 1-40 % aufweist.

6. Träger- und Targeting-System nach einem der vorhergehenden Ansprüche, das zusätzlich Folgendes umfasst: Liposomen, ungesättigte Lipide, ein visikelbildendes Lipid, ein hydrophiles Polymer, Glykolipide, wie etwa Cerebroside und Ganglioside und/oder Liposomen, wobei die visikelbildenden Lipide vorzugsweise zwei Kohlenwasserstoffketten, vorzugsweise mit einer Länge von 8-26 Kohlenstoffatomen, häufig Acylketten und eine polare Kopfgruppe aufweisen und wobei noch bevorzugter die Phospholipide der Liposomen aus der Gruppe umfassend Phosphatidylcholin (PC), Phosphatidylethanolamin (PE), Phosphatidsäure (PA), Phosphatidylinositol (PI) und Sphingomyelin (SM) ausgewählt sind.

7. Verfahren zur Herstellung des Träger- und Targeting-Systems nach einem der vorhergehenden Ansprüche, **gekennzeichnet durch**:
a) Mischen von einem oder mehreren Organosilikon, Zucker-Organosilikon, Aminozucker-Organosilikon, Alkyl-, Alkoxy- oder Acyloxy-Organosilikon, Peptid-Organosilikon und/oder Aminosäuren(n)-Organosilikon, Lipiden, Salzen und/oder den daraus gebildeten Vesikeln mit einem oder mehreren Polymeren, wie etwa PEG, einem oder mehreren Salzen, wie etwa Zn²⁺, Ca²⁺, Mg²⁺, Na²⁺, Puffern mit einem oder mehreren Wirkstoffen, wie etwa Doxorubicin, Cis-Oxoplatin, Carboplatin, Nukleinsäuren, wie etwa DNA, RNA, siRNA, Nukleosiden mit ausgewähltem pH-Wert sowie ausgewählter Salzkonzentration und Temperatur, vorzugsweise in einer Pufferlösung, die TRIS, HEPES; MOPS, MES und/oder andere häufig verwendete biologische Puffer umfasst,
**dadurch gekennzeichnet, dass**
ein oder mehrere kationische Lipide kovalent an das Silikon und/oder chemische Gruppen und/oder Reste von R₁, R₂, R₃ und R₄ von Verbindungen gemäß der allgemeinen Formel (I) gebunden sind;
b) Homogenisierung, Beschallung und/oder Extrusion der Mischung, gefolgt von
c) Trennung des freien Wirkstoffs,
d) Sterilfiltration der Mischung,
e) Lyophilisierung,
f) Rekonstitution, um ein Siosom- und/oder ein kombiniertes Siosom-/Liposom-Komplex-Konzentrat zu bilden.

8. Verfahren nach Anspruch 7, **dadurch gekennzeichnet, dass** der Wirkstoff, vorzugsweise Nukleinsäuren, DNA, RNA und/oder siRNA vor der Herstellung des Siosom-Trägersystems mit Kationen beladen wird.

9. Verfahren nach Anspruch 7 oder 8, **dadurch gekennzeichnet, dass** die kationischen Silane und/oder Siosome mit anionischen Salzen beladen werden.

10. Träger- und Targeting-System nach einem der Ansprüche 1 bis 6 zur Verwendung als ein Arzneimittel, Adjuvans oder als eine Prophylaxe gegen Krankheiten.

11. Träger- und Targeting-System nach einem der Ansprüche 1 bis 6 zur Verwendung bei der Behandlung von Krebs.

12. Verwendung des Träger- und Targeting-Systems nach einem der Ansprüche 1 bis 6 in der Kosmetik.

13. Pharmazeutisches Mittel, das das Träger- und Targeting-System nach einem der Ansprüche 1 bis 6 umfasst, und pharmazeutisch annehmbarer Träger und/oder pharmazeutisch annehmbares Salz.

## Revendications

1. Système de support et de ciblage pour l'administration et la libération intracellulaire d'une substance active piégée dans un siosome et/ou un siosome-liposome, comprenant une composition, dans lequel ladite composition comprend :
- un organosilicium, un organosilicium-sucre, un organosilicium-amino-sucre, un organosilicium d'alkyle, d'alcoxy ou d'acyloxy, un organosilicium peptidique, un organosilicium nucléotidique et/ou un organosilicium d'acide(s) aminé(s),
- une substance active, et
- des lipides,
moyennant quoi
les composés d'organosilicium, d'organosilicium-sucre ou d'organosilicium-amino-sucre sont **caractérisés par** la formule générale (I) : 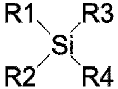 moyennant quoi R₁ et R₂ peuvent être les mêmes ou différents, et où R₃ et R₄ peuvent être les mêmes ou différents, moyennant quoi
R₁, R₂ = Monosaccharide, disaccharide, oligosaccharide, nucléotide, alkyle ou aryle, hétérocycles aromatiques ou aliphatiques, et
R₃, R₄ = Polynucléotide, peptide, chaînes aliphatiques, protéines, ARNsi, ARN, ADN, ligand, vecteur, acide gras, groupe acyle,
**caractérisé en ce qu'**un
ou plusieurs lipides cationiques sont liés de façon covalente au silicium et/ou aux groupes chimiques et/ou aux résidus de R₁, R₂, R₃ et R₄ des composés selon la formule générale (I).

2. Système de support et de ciblage selon la revendication précédente, comprenant un silane-sucre cationique, **caractérisé en ce qu'**un ou plusieurs lipides cationiques sont liés de façon covalente au silicium et/ou aux groupes chimiques et/ou aux résidus de R₁, R₂, R₃ et R₄ de la formule générale (I), moyennant quoi
R₁ = Monosaccharide, disaccharide, amino-saccharide, oligosaccharide ;
R₂ = Monosaccharide, disaccharide, oligosaccharide, acide aminé, peptide, nucléoside, nucléotides, alkyl, résidus aryle, hétérocycles aromatiques ou aliphatiques ;
R₃, R₄ = lipide cationique et/ou lipide acide aminé cationique, ou
R₃, R₄ = 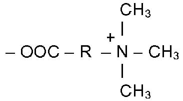 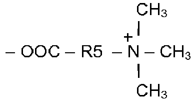 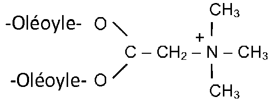
ou un résidu peptidique de formule : 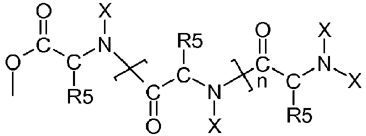 dans lequel
R représente un résidu alkyle, alcényle ou alcinyle, ramifié ou non, avec 5 à 29 atomes de carbone qui peuvent être substitués avec 1 à 3 atomes d'halogène, des résidus alcoxy avec 1 à 18 atomes de carbone ou des groupes amino,
les résidus R5, qui peuvent être les mêmes ou différents, représentent le résidu restant après élimination du groupe 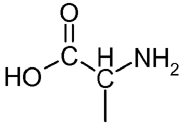 à partir d'un acide aminé,
les résidus X, qui peuvent être les mêmes ou différents, représentent un atome d'hydrogène ou un groupe protecteur d'amino qui survient généralement dans la chimie peptidique, et
n représente un entier de 1 à 12, ou dans lequel
R₃ et R₄, qui peuvent être les mêmes ou différents, représentent chacun un groupe aryle, tel qu'un groupe phényle, ou le résidu restant après élimination d'un atome d'hydrogène d'un monosaccharide, d'un disaccharide, d'un amino-sucre ou d'un acide carboné hydroxylé, ou des résidus alcoxy avec 1 à 5 atomes de carbone, ou des résidus acyle d'un acide aminé avec un groupe amino libre ou protégé, ou un résidu dipeptide, tripeptide ou tétrapeptide d'un acide aminé avec des groupes amino libres ou protégés, ou ils ont la signification de R₁ et R₂.

3. Système de support et de ciblage selon l'une quelconque des revendications précédentes, **caractérisé en ce que** la substance active encapsulée et/ou piégée est un agent anticancéreux, tel que la doxorubicine, le cis-platine, le carbo-platine et/ou l'oxali-platine, et/ou un matériel génétique, de préférence un acide nucléique tel que l'ADN, l'ARN, l'ARNsi et/ou un agent antiviral et/ou antibactérien.

4. Système de support et de ciblage selon l'une quelconque des revendications précédentes, **caractérisé en ce que** le polyéthylène glycol est lié de façon covalente au silicium et/ou à un ou plusieurs des groupes R₁, R₂, R₃ et R₄ de formule générale (I), de préférence au moyen d'un liant espaceur clivable.

5. Système de support et de ciblage selon l'une quelconque des revendications précédentes, **caractérisé en ce que** la composition comprend un cryoprotecteur, de préférence un disaccharide choisi dans le groupe constitué de saccharose, de maltose, de tréhalose et/ou de lactose, mieux encore un disaccharide ayant une concentration de 1 à 40 %.

6. Système de support et de ciblage selon l'une quelconque des revendications précédentes, comprenant en sus des liposomes, des lipides non-saturés, un lipide formant vésicule, un polymère hydrophile, des glycolipides tels que les cérébiosides et les gangliosides et/ou des liposomes, dans lequel les lipides formant vésicule démontrent préférablement deux chaînes hydrocarbonées, de préférence d'une longueur entre 8 à 26 atomes de carbone, généralement des chaînes acyle, et un groupe à tête polaire, et mieux encore les phospholipides des liposomes sont choisis dans le groupe constitué de la phosphatidylcholine (PC), de la phosphatidyléthanolamine (PE), de l'acide phosphatidique(PA), du phosphatidyl-inositol (PI) et de la sphingomyéline (SM).

7. Méthode de préparation du système de support et de ciblage selon l'une quelconque des revendications précédentes, **caractérisée par**
a) le mélange d'un ou de plusieurs organosilicium, organosilicium-sucre, organosilicium-amino-sucre, organosilicium d'alkyle, d'alcoxy ou d'acyloxy, organosilicium peptidique et/ou organosilicium d'acide(s) aminé(s), lipides, sels et/ou les vésicules formées à partir de ceux-ci, avec un ou plusieurs polymères, tels que PEG, un ou plusieurs sels, tels que Zn²⁺, Ca²⁺, Mg²⁺, Na⁺, des tampons, avec un ou plusieurs substances actives, telles que la doxorubicine, le cis-oxoplatine, l'oxali-platine, les acides nucléiques, tels que l'ADN, l'ARN, l'ARNsi, des nucléosides à un pH, une concentration de sel et une température choisis, de préférence dans une solution tampon qui contient du TRIS, de l'HEPES ; du MOPS, du MES et/ou d'autres tampons biologiques couramment utilisés,
**caractérisée en ce**
**qu'**un ou plusieurs lipides cationiques sont liés de façon covalente au silicium et/ou aux groupes chimiques et/ou aux résidus de R₁, R₂, R₃ et R₄ des composés selon la formule générale (I) ;
b) l'homogénéisation, la sonication et/ou l'extrusion du mélange, suivi par
c) la séparation de la substance active libre,
d) la filtration stérile du mélange,
e) la lyophilisation,
f) la reconstitution pour former un concentré de complexe de siosome et/ou siosome-liposome combiné.

8. Méthode selon la revendication 7, **caractérisée en ce que** la substance active, de préférence les acides nucléiques, l'ADN, l'ARN et/ou l'ARNsi, est chargée de cations avant la préparation des systèmes de support de siosome.

9. Méthode selon les revendications 7 ou 8, **caractérisée en ce que** les silanes cationiques et/ou les siosomes sont chargés de sels anioniques.

10. Système de support et de ciblage selon l'une quelconque des revendications 1 à 6, pour une utilisation comme un médicament, adjuvant ou comme une prophylaxie pour des maladies.

11. Système de support et de ciblage selon l'une quelconque des revendications 1 à 6, pour une utilisation comme un médicament dans le traitement du cancer.

12. Utilisation du système de support et de ciblage selon l'une quelconque des revendications 1 à 6 dans des cosmétiques.

13. Agent pharmaceutique comprenant le système de support et de ciblage selon l'une quelconque des revendications 1 à 6, et un support pharmaceutiquement acceptable et/ou un sel pharmaceutiquement acceptable.
